(19) Europäisches Patentamt
European Patent Office
Office européen des brevets

(11) EP 0 978 517 A2

(12) **EUROPÄISCHE PATENTANMELDUNG**

(43) Veröffentlichungstag:
**09.02.2000 Patentblatt 2000/06**

(21) Anmeldenummer: **99119410.1**

(22) Anmeldetag: **17.07.1997**

(51) Int. Cl.$^7$: **C07D 487/14**, A61K 31/505
// (C07D487/14, 249:00, 239:00, 235:00)

(84) Benannte Vertragsstaaten:
**AT BE CH DE DK ES FI FR GB GR IE IT LI LU MC NL PT SE**
Benannte Erstreckungsstaaten:
**AL LT LV RO SI**

(30) Priorität: **20.07.1996 DE 19629378**

(62) Dokumentnummer(n) der früheren Anmeldung(en) nach Art. 76 EPÜ:
**97933683.1 / 0 880 524**

(71) Anmelder:
- **Boehringer Ingelheim Pharma KG**
**55216 Ingelheim am Rhein (DE)**
Benannte Vertragsstaaten:
**BE CH DE DK ES FI FR GR IT LI LU MC NL PT SE AT**
- **Boehringer Ingelheim International GmbH**
**55216 Ingelheim (DE)**
Benannte Vertragsstaaten:
**GB IE**

(72) Erfinder:
- **Kuefner-Muehl, Ulrike**
**55218 Ingelheim am Rhein (DE)**
- **Kummer, Werner**
**55218 Ingelheim am Rhein (DE)**
- **Pohl, Gerald**
**55435 Gau-Algesheim (DE)**
- **Gaida, Wolfram**
**55218 Ingelheim am Rhein (DE)**
- **Lehr, Erich**
**55425 Waldalgesheim (DE)**
- **Mierau, Joachim**
**55127 Mainz am Rhein 33 (DE)**
- **Weiser, Thomas**
**55268 Nieder-Olm (DE)**

(74) Vertreter:
**Laudien, Dieter, Dr. et al**
**Boehringer Ingelheim GmbH**
**Abteilung Patente**
**55216 Ingelheim (DE)**

Bemerkungen:
Diese Anmeldung ist am 30 - 09 - 1999 als Teilanmeldung zu der unter INID-Kode 62 erwähnten Anmeldung eingereicht worden.

# (54) Triazolpurine, Verfahren zu ihrer Herstellung und ihre Verwendung als Arzneimittel

(57) Die Erfindung betrifft neue Triazolopurin-Derivate sowie Triazolopurin-Grundkörper, Verfahren zu ihrer Herstellung und ihre Verwendung als Arzneimittel.

EP 0 978 517 A2

**Beschreibung**

Die Erfindung betrifft neue Triazolopurin-Derivate sowie Triazolopurin-Grundkörper, Verfahren zu ihrer Herstellung und ihre Verwendung als Arzneimittel.

Die neuen Triazolopurinderivate besitzen die Struktur der allgemeinen Formel (I):

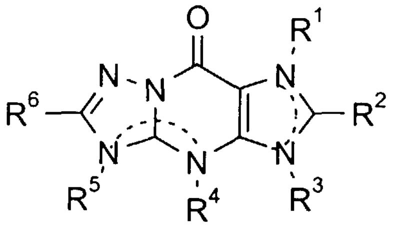

(I)

worin

$R^1$ oder $R^3$ Wasserstoff, ein gegebenenfalls substituiertes $C_1$-$C_{10}$-Alkyl, $C_2$-$C_{10}$-Alkenyl- oder $C_2$-$C_{10}$-Alkinyl, ein gegebenenfalls substituiertes $C_3$-$C_8$-Cycloalkyl, ein gegebenenfalls substituiertes $C_4$-$C_8$-Cycloalkenyl oder ein gegebenenfalls substituiertes $C_6$-$C_8$-Cycloalkinyl;

$R^1$ oder $R^3$ ein gegebenenfalls substituiertes $C_6$-$C_{10}$-Aryl-$C_1$-$C_6$-alkyl, $C_6$-$C_{10}$-Aryl-$C_2$-$C_6$-alkenyl, $C_6$-$C_{10}$-Aryl-$C_2$-$C_6$-alkinyl, ein gegebenenfalls substituiertes $C_6$-$C_{10}$-Aryl- oder ein gegebenenfalls substituiertes Heteroaryl, ein gegebenenfalls substituierter 5, 6 oder 7-gliedriger Heterocyclus, der als Heteroatome ein oder mehrere Atome aus der Gruppe Stickstoff, Sauerstoff oder Schwefel enthält, wobei der Heterocylus über ein Kohlenstoffatom des Ringes verknüpft ist;

$R^1$ oder $R^3$ ein gegebenenfalls substituiertes $C_1$-$C_{10}$-Alkyloxycarbonyl, ein gegebenenfalls substituiertes $C_2$-$C_{10}$-Alkenyloxycarbonyl, ein gegebenenfalls substituiertes $C_2$-$C_{10}$-Alkinyloxycarbonyl, ein gegebenenfalls substituiertes $C_3$-$C_8$-Cycloalkyloxycarbonyl, ein gegebenenfalls substituiertes $C_6$-$C_{10}$-Aryloxycarbonyl, ein gegebenenfalls substituiertes $C_6$-$C_{10}$-Aryl-$C_1$-$C_6$-alkyloxycarbonyl, A-O-CO- worin A die Bedeutung eines gegebenenfalls substituierten 5, 6 oder 7-gliedrigen Heterocyclus hat, der als Heteroatome ein oder mehrere Atome aus der Gruppe Stickstoff, Sauerstoff oder Schwefel enthält, wobei A über ein Kohlenstoffatom des Ringes verknüpft ist,

$R^1$ oder $R^3$ ein gegebenenfalls substituiertes $C_1$-$C_{10}$-Alkylsulfonyl, $C_2$-$C_{10}$-Alkenylsulfony, $C_2$-$C_{10}$-Alkinylsulfonyl, ein gegebenenfalls substituiertes $C_3$-$C_8$-Cycloalkylsulfonyl, ein gegebenenfalls substituiertes $C_6$-$C_{10}$-Arylsulfonyl, ein gegebenenfalls substituiertes $C_6$-$C_{10}$-Aryl-$C_1$-$C_6$-alkylsulfonyl, ein Rest A-$SO_2$- worin A die oben genannte Bedeutung aufweist und über ein Kohlenstoffatom des Rings verknüpft ist;

$R^1$ oder $R^3$ ein gegebenenfalls substituiertes $C_1$-$C_{10}$-Alkylcarbonyl, ein gegebenenfalls substituiertes $C_2$-$C_{10}$-Alkenylcarbonyl, ein gegebenenfalls substituiertes $C_2$-$C_{10}$-Alkinylcarbonyl, ein gegebenenfalls substituiertes $C_3$-$C_8$-Cycloalkylcarbonyl, ein gegebenenfalls substituiertes $C_6$-$C_{10}$-Arylcarbonyl, ein gegebenenfalls substituiertes $C_6$-$C_{10}$-Aryl-$C_1$-$C_6$-alkylcarbonyl, ein Rest A-CO- worin A die zuvor genannte Bedeutung hat;

$R^1$ oder $R^3$ ein Rest der allgemeinen Formel

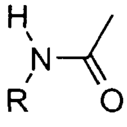

worin

R Wasserstoff, Phenyl, substituiertes Phenyl, ein gegebenenfalls substituiertes Benzyl, eine gegebenenfalls substituierte Cycloalkylgruppe mit 3 bis 6 Kohlenstoffatomen, eine verzweigte oder unverzweigte $C_1$-$C_{10}$-Alkyl-, bevorzugt
$C_1$-$C_4$-Alkyl-, $C_2$-$C_{10}$-Alkenyl- oder $C_2$-$C_{10}$-Alkinylgruppe, die gegebenenfalls durch Hydroxy, Phenyl, substituiertes Phenyl, Amino, substituiertes Amino substituiert sein kann, bedeutet;

$R^2$ Wasserstoff, Hydroxy, Amino, Halogen, Nitro, $CF_3$, COOH, Mercapto, $C_1$-$C_6$-Alkylmercapto, ein gegebenenfalls substituiertes $C_1$-$C_{10}$-Alkyl, ein gegebenenfalls substituiertes $C_2$-$C_{10}$-Alkenyl, ein gegebenenfalls substituiertes $C_2$-$C_{10}$-Alkinyl, ein gegebenenfalls substituiertes $C_3$-$C_8$-Cycloalkyl, ein gegebenenfalls substituiertes $C_5$-$C_8$-Cycloalkanon, ein gegebenenfalls substituiertes $C_4$-$C_8$-Cycloalkenyl, ein gegebenenfalls substituiertes $C_6$-$C_8$-Cycloalkinyl, ein gegebenenfalls substituiertes $C_6$-$C_{10}$-Aryl, ein gegebenenfalls substituiertes $C_6$-$C_{10}$-Aryl-$C_1$-$C_6$-alkyl, $C_6$-$C_{10}$-Aryl-$C_2$-$C_6$-alkenyl, $C_6$-$C_{10}$-Aryl-$C_2$-$C_6$-alkinyl, $C_3$-$C_8$-Cycloalkyl-$C_1$-$C_6$-alkyl, $C_3$-$C_8$-Cylcloalkyl-$C_2$-$C_6$-akenyl, oder $C_3$-$C_8$-Cycloalkyl-$C_2$-$C_6$-alkinyl;

$R^2$ ein gegebenenfalls substituierter 5, 6 oder 7-gliedriger Heterocyclus, der als Heteroatom ein oder mehrere Atome aus der Gruppe Stickstoff, Sauerstoff oder Schwefel enthält, wobei der Stickstoff gegebenenfalls substituiert sein kann und der Heterocyclus über ein C-Atom oder N-Atom direkt oder über eine $C_1$-$C_6$-Alkyl, $C_2$-$C_6$-Alkenyl, oder $C_2$-$C_6$-Alkinylbrücke angeknüpft sein kann;

$R^2$ ein gegebenenfalls substituiertes $C_1$-$C_{10}$-Alkylsulfonyl, $C_2$-$C_{10}$-Alkenylsulfonyl- oder $C_2$-$C_{10}$-Alkinylsulfonyl, ein gegebenenfalls substituiertes $C_3$-$C_8$-Cycloalkylsulfonyl, ein gegebenenfalls substituiertes $C_6$-$C_{10}$-Arylsulfonyl, ein gegebenenfalls substituiertes Heteroarylsulfonyl oder ein gegebenenfalls substituiertes $C_6$-$C_{10}$-Aryl-$C_1$-$C_6$-alkylsulfonyl;

$R^2$ ein gegebenenfalls substituiertes $C_1$-$C_{10}$-Alkylsulfonyloxy, $C_2$-$C_{10}$-Alkenylsulfonyloxy- oder $C_2$-$C_{10}$-Alkinylsulfonyloxy, ein gegebenenfalls substituiertes $C_3$-$C_8$-Cycloalkylsulfonyloxy, ein gegebenenfalls substituiertes $C_6$-$C_{10}$-Arylsulfonyloxy, ein gegebenenfalls substituiertes $C_6$-$C_{10}$-Aryl-$C_1$-$C_6$-alkylsulfonyloxy oder ein gegebenenfalls substituiertes Heteroarylsulfonyloxy;

$R^2$ ein gegebenenfalls substituiertes $C_1$-$C_{10}$-Alkylsulfonylamino, $C_2$-$C_{10}$-Alkenylsulfonylamino- oder $C_2$-$C_{10}$-Alkinylsulfonylamino, ein gegebenenfalls substituiertes $C_3$-$C_8$-Cycloalkylsulfonylamino, ein gegebenenfalls substituiertes $C_6$-$C_{10}$-Arylsulfonylamino, ein gegebenenfalls substituiertes $C_6$-$C_{10}$-Aryl-$C_1$-$C_6$-alkylsulfonylamino, oder ein gegebenenfalls substituiertes Heteroarylsulfonylamino;

$R^2$ ein gegebenenfalls substituiertes $C_1$-$C_{10}$-Alkylaminocarbonyl, $C_1$-$C_{10}$-Dialkylaminocarbonyl, $C_1$-$C_{10}$-Alkyl-$C_2$-$C_{10}$-alkenylaminocarbonyl-, $C_2$-$C_{10}$-Alkenylaminocarbonyl, $C_2$-$C_{10}$-Dialkenylaminocarbonyl, $C_2$-$C_{10}$-Alkinylaminocarbonyl oder $C_1$-$C_{10}$-Alkyl-$C_2$-$C_{10}$-alkinylaminocarbonyl, ein gegebenenfalls substituiertes $C_3$-$C_8$-Cycloalkylaminocarbonyl, ein gegebenenfalls substituiertes $C_6$-$C_{10}$-Arylaminocarbonyl, ein gegebenenfalls substituiertes $C_6$-$C_{10}$-Aryl-$C_1$-$C_6$-alkylaminocarbonyl oder ein gegebenenfalls substituiertes Heteroarylaminocarbonyl;

$R^2$ ein gegebenenfalls substituiertes $C_1$-$C_{10}$-Alkyloxycarbonylamino, $C_2$-$C_{10}$-Alkenyloxycarbonylamino oder $C_2$-$C_{10}$-Alkinyloxycarbonylamino, ein gegebenenfalls substituiertes $C_3$-$C_8$-Cycloalkyloxycarbonylamino, ein gegebenenfalls substituiertes $C_6$-$C_{10}$-Aryloxycarbonylamino, ein gegebenenfalls substituiertes $C_6$-$C_{10}$-Aryl-$C_1$-$C_6$-alkyloxycarbonylamino oder ein gegebenenfalls substituiertes Heteroaryloxycarbonylamino;

$R^2$ ein gegebenenfalls substituiertes $C_1$-$C_{10}$-Alkylaminocarbonyloxy, $C_2$-$C_{10}$-Alkenylaminocarbonyloxy oder $C_2$-$C_{10}$-Alkinylaminocarbonyloxy, ein gegebenenfalls substituiertes $C_3$-$C_8$-Cycloalkylaminocarbonyloxy, ein gegebenenfalls substituiertes $C_6$-$C_{10}$-Arylaminocarbonyloxy, ein gegebenenfalls substituiertes $C_6$-$C_{10}$-Aryl-$C_1$-$C_6$-alkylaminocarbonyloxy oder ein gegebenenfalls substituiertes Heteroarylaminocarbonyloxy;

$R^2$ ein gegebenenfalls substituiertes $C_1$-$C_{10}$-Alkyl-N-amidino, $C_2$-$C_{10}$-Alkenyl-N-amidino oder $C_2$-$C_{10}$-Alkinyl-N-amidino, ein gegebenenfalls substituiertes $C_3$-$C_8$-Cycloalkyl-N-amidino, ein gegebenenfalls substituiertes $C_6$-$C_{10}$-Aryl-N-amidino, ein gegebenenfalls substituiertes $C_6$-$C_{10}$-Aryl-$C_1$-$C_6$-alkyl-N-amidino oder ein gegebenenfalls substituiertes Heteroaryl-N-amidino;

$R^2$ ein gegebenenfalls substituiertes $C_1$-$C_{10}$-Alkyloxy, $C_2$-$C_{10}$-Alkenyloxy oder $C_2$-$C_{10}$-Alkinyloxy, ein gegebenenfalls substituiertes $C_3$-$C_8$-Cycloalkyloxy, ein gegebenenfalls substituiertes $C_6$-$C_{10}$-Aryloxy, ein gegebenenfalls substituiertes $C_6$-$C_{10}$-Aryl-$C_1$-$C_6$-alkyloxy oder ein gegebenenfalls substituiertes Heteroaryloxy;

$R^2$ ein gegebenenfalls substituiertes $C_1$-$C_{10}$-Alkyloxycarbonyl, $C_2$-$C_{10}$-Alkenyloxycarbonyl oder $C_2$-$C_{10}$-Alkinyloxycarbonyl, ein gegebenenfalls substituiertes $C_3$-$C_8$-Cycloalkyloxycarbonyl, ein gegebenenfalls substituiertes $C_6$-$C_{10}$-Aryloxycarbonyl, ein gegebenenfalls substituiertes $C_6$-$C_{10}$-Aryl-$C_1$-$C_6$-alkyloxycarbonyl oder ein gegebenenfalls substituiertes Heteroaryloxycarbonyl;

$R^2$ ein gegebenenfalls substituiertes $C_1$-$C_{10}$-Alkylcarbonyloxy, $C_2$-$C_{10}$-Alkenylcarbonyloxy- oder $C_2$-$C_{10}$-Alkinylcarbonyloxy, ein gegebenenfalls substituiertes $C_3$-$C_8$-Cycloalkylcarbonyloxy, ein gegebenenfalls substituiertes $C_6$-$C_{10}$-Arylcarbonyloxy, ein gegebenenfalls substituiertes $C_6$-$C_{10}$-Aryl-$C_1$-$C_6$-alkylcarbonyloxy oder ein gegebenenfalls substituiertes Heteroarylcarbonyloxy;

$R^2$ ein gegebenenfalls substituiertes $C_1$-$C_{10}$-Alkylthio, $C_2$-$C_{10}$-Alkenylthio oder $C_2$-$C_{10}$-Alkinylthio, ein gegebenenfalls substituiertes $C_3$-$C_8$-Cycloalkylthio, ein gegebenenfalls substituiertes $C_6$-$C_{10}$-Arylthio, ein gegebenenfalls substituiertes $C_6$-$C_{10}$-Aryl-$C_1$-$C_6$-alkylthio oder ein gegebenenfalls substituiertes Heteroarylthio;

$R^2$ ein gegebenenfalls substituiertes Amin, bevorzugt $NR^8R^9$;

$R^2$ ein gegebenenfalls substituierte Rest der Formel

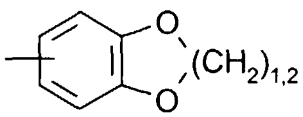
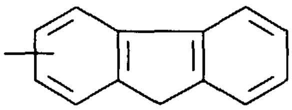
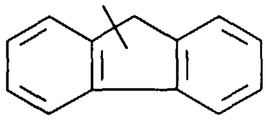

$R^2$ ein gegebenenfalls substituierter Rest der Formel

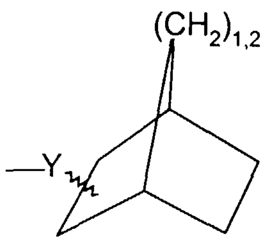
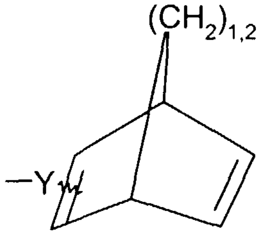
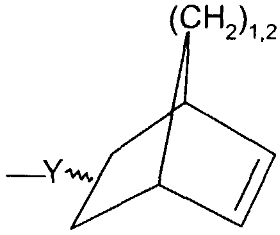

wobei Y eine Einfachbindung oder ein Alkylen, ein Alkenylen oder ein Alkinylen mit bis zu 6, bevorzugt mit bis zu 4 Kohlenstoffatomen in der Kette ist;

$R^2$ ein gegebenenfalls substituierter Rest der Formel

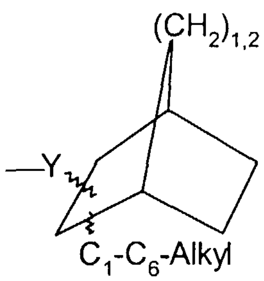

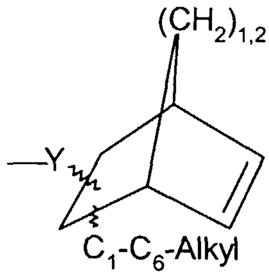

wobei Y eine Einfachbindung oder ein Alkylen, ein Alkenylen oder ein Alkinylen mit bis zu 6, bevorzugt mit bis zu 4 Kohlenstoffatomen in der Kette ist;

$R^2$ ein gegebenenfalls substituierter Rest der Formel

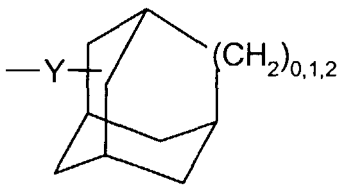

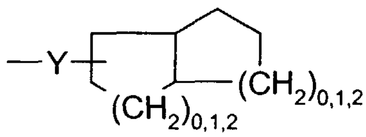

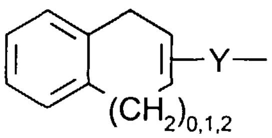

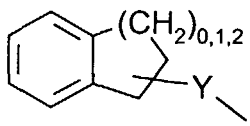

wobei Y eine Einfachbindung oder ein Alkylen, ein Alkenylen oder ein Alkinylen mit bis zu 6, bevorzugt mit bis zu 4 Kohlenstoffatomen in der Kette ist;

$R^4$ oder $R^5$ ein Wasserstoffatom, ein gegebenenfalls substituiertes $C_1$-$C_{10}$-Alkyl, $C_2$-$C_{10}$-Alkenyl- oder $C_2$-$C_{10}$-Alkinyl, ein gegebenenfalls substituiertes $C_3$-$C_8$-Cycloalkyl, ein gegebenenfalls substituiertes $C_4$-$C_8$-Cycloalkenyl oder ein gegebenenfalls substituiertes $C_6$-$C_8$-Cycloalkinyl, ein gegebenenfalls substituiertes $C_6$-$C_{10}$-Aryl-$C_1$-$C_6$-alkyl, $C_6$-$C_{10}$-Aryl-$C_2$-$C_6$-alkenyl, $C_6$-$C_{10}$-Aryl-$C_2$-$C_6$-alkinyl, ein gegebenenfalls substituiertes $C_6$-$C_{10}$-Aryl oder ein gegebenenfalls substituiertes Heteroaryl;

$R^4$ oder $R^5$ ein gegebenenfalls substituierter 5,6 oder 7-gliedriger Heterocyclus, der als Heteroatome ein oder mehrere Atome aus der Gruppe Stickstoff, Sauerstoff oder Schwefel enthält, wobei der Heterocylus über ein Kohlenstoffatom des Rings gebunden ist;

$R^4$ oder $R^5$ ein gegebenenfalls substituiertes $C_1$-$C_{10}$-Alkyloxycarbonyl, ein gegebenenfalls substituiertes $C_2$-$C_{10}$-Alkenyloxycarbonyl, ein gegebenenfalls substituiertes $C_2$-$C_{10}$-Alkinyloxycarbonyl, ein gegebenenfalls substituiertes $C_3$-$C_8$-Cycloalkyloxycarbonyl, ein gegebenenfalls substituiertes $C_6$-$C_{10}$-Aryl-$C_1$-$C_6$-alkyloxycarbonyl, ein gegebenenfalls substituiertes $C_6$-$C_{10}$-Aryloxycarbonyl;

$R^4$ oder $R^5$ A-O-CO- worin A die Bedeutung eines gegebenenfalls substituierten 5, 6 oder 7-gliedrigen Heterocyclus hat, der als Heteroatome ein oder mehrere Atome aus der Gruppe Stickstoff, Sauerstoff oder Schwefel enthält;

$R^4$ oder $R^5$ ein gegebenenfalls substituiertes $C_1$-$C_{10}$-Alkylsulfonyl, $C_2$-$C_{10}$-Alkenylsulfony, $C_2$-$C_{10}$-Alkinylsulfonyl, ein gegebenenfalls substituiertes $C_3$-$C_8$-Cycloalkylsulfonyl, ein gegebenenfalls substituiertes $C_6$-$C_{10}$-

Arylsulfonyl, ein Rest A-$SO_2$- worin A die oben genannte Bedeutung aufweist und über ein Kohlenstoffatom des Rings verknüpft ist;

$R^4$ oder $R^5$ ein gegebenenfalls substituiertes $C_1$-$C_{10}$-Alkylcarbonyl, ein gegebenenfalls substituiertes $C_2$-$C_{10}$-Alkenylcarbonyl, ein gegebenenfalls substituiertes $C_2$-$C_{10}$-Alkinylcarbonyl, ein gegebenenfalls substituiertes $C_3$-$C_8$-Cycloalkylcarbonyl, ein gegebenenfalls substituiertes $C_6$-$C_{10}$-Arylcarbonyl, ein gegebenenfalls substituiertes $C_6$-$C_{10}$-Aryl-$C_1$-$C_6$-alkylcarbonyl, ein Rest A-CO- worin A die zuvor genannte Bedeutung hat;

$R^4$ oder $R^5$ ein Rest der allgemeinen Formel

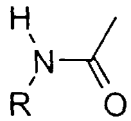

worin

R Wasserstoff, Phenyl, substituiertes Phenyl, ein gegebenenfalls substituiertes Benzyl, eine gegebenenfalls substituierte Cycloalkylgruppe mit 3 bis 6 Kohlenstoffatomen, eine verzweigte oder unverzweigte $C_1$-$C_{10}$-Alkyl-, bevorzugt $C_1$-$C_4$-Alkyl-, $C_2$-$C_{10}$-Alkenyl- oder $C_2$-$C_{10}$-Alkinylgruppe, die gegebenenfalls durch Hydroxy, Phenyl, substituiertes Phenyl, Amino, substituiertes Amino substituiert sein kann, bedeutet;

$R^6$ Wasserstoff, Hydroxy, -CHO, Amino, Halogen, Nitro, $CF_3$, COOH, Mercapto, $C_1$-$C_6$-Alkylmercapto, ein gegebenenfalls substituiertes $C_1$-$C_{10}$-Alkyl, ein gegebenenfalls substituiertes $C_2$-$C_{10}$-Alkenyl, ein gegebenenfalls substituiertes $C_2$-$C_{10}$-Alkinyl, ein gegebenenfalls substituiertes $C_3$-$C_8$-Cycloalkyl, ein gegebenenfalls substituiertes $C_5$-$C_8$-Cycloalkanon, ein gegebenenfalls substituiertes $C_4$-$C_8$-Cycloalkenyl, ein gegebenenfalls substituiertes $C_6$-$C_8$-Cycloalkinyl, ein gegebenenfalls substituiertes $C_6$-$C_{10}$-Aryl, ein gegebenenfalls substituiertes $C_6$-$C_{10}$-Aryl-$C_1$-$C_6$-alkyl, $C_6$-$C_{10}$-Aryl-$C_2$-$C_6$-alkenyl, $C_6$-$C_{10}$-Aryl-$C_2$-$C_6$-alkinyl, ein gegebenenfalls substituiertes $C_3$-$C_8$-Cycloalkyl-$C_1$-$C_6$-alkyl, $C_3$-$C_8$-Cylcloalkyl-$C_2$-$C_6$-alkenyl oder $C_3$-$C_8$-Cycloalkyl-$C_2$-$C_6$-alkinyl;

$R^6$ ein gegebenenfalls substituierter 5, 6 oder 7-gliedriger Heterocyclus, der als Heteroatom ein oder mehrere Atome aus der Gruppe Stickstoff, Sauerstoff oder Schwefel enthält, wobei der Stickstoff gegebenenfalls substituiert sein kann und der Heterocyclus über ein C-Atom oder N-Atom direkt oder über eine $C_1$-$C_6$-Alkyl, $C_2$-$C_6$-Alkenyl oder $C_2$-$C_6$-Alkinylbrücke angeknüpft sein kann;

$R^6$ ein gegebenenfalls substituiertes $C_1$-$C_{10}$-Alkylsulfonyl, $C_2$-$C_{10}$-Alkenylsulfonyl oder $C_2$-$C_{10}$-Alkinylsulfonyl, ein gegebenenfalls substituiertes $C_3$-$C_8$-Cycloalkylsulfonyl, ein gegebenenfalls substituiertes $C_6$-$C_{10}$-Arylsulfonyl, ein gegebenenfalls substituiertes $C_6$-$C_{10}$-Aryl-$C_1$-$C_6$-alkylsulfonyl oder ein gegebenenfalls substituiertes Heteroarylsulfonyl;

$R^6$ ein gegebenenfalls substituiertes $C_1$-$C_{10}$-Alkylsulfonyloxy, $C_2$-$C_{10}$-Alkenylsulfonyloxy- oder $C_2$-$C_{10}$-Alkinylsulfonyloxy, ein gegebenenfalls substituiertes $C_3$-$C_8$-Cycloalkylsulfonyloxy, ein gegebenenfalls substituiertes $C_6$-$C_{10}$-Arylsulfonyloxy, ein gegebenenfalls substituiertes $C_6$-$C_{10}$-Aryl-$C_1$-$C_6$-alkylsulfonyloxy oder ein gegebenenfalls substituiertes Heteroarylsulfonyloxy;

$R^6$ ein gegebenenfalls substituiertes $C_1$-$C_{10}$-Alkylsulfonylamino, $C_2$-$C_{10}$-Alkenylsulfonylamino oder $C_2$-$C_{10}$-Alkinylsulfonylamino, ein gegebenenfalls substituiertes $C_3$-$C_8$-Cycloalkylsulfonylamino, ein gegebenenfalls substituiertes $C_6$-$C_{10}$-Arylsulfonylamino, ein gegebenenfalls substituiertes $C_6$-$C_{10}$-Aryl-$C_1$-$C_6$-alkylsulfonylamino oder ein gegebenenfalls substituiertes Heteroarylsulfonylamino;

$R^6$ ein gegebenenfalls substituiertes $C_1$-$C_{10}$-Alkylaminocarbonyl, $C_1$-$C_{10}$-Dialkylaminocarbonyl, $C_1$-$C_{10}$-Alkyl-$C_2$-$C_{10}$-alkenylaminocarbonyl, $C_2$-$C_{10}$-Alkenylaminocarbonyl, $C_2$-$C_{10}$-Dialkenylaminocarbonyl, $C_2$-$C_{10}$-Alkinylaminocarbonyl oder $C_1$-$C_{10}$-Alkyl-$C_2$-$C_{10}$-alkinylaminocarbonyl, ein gegebenenfalls substituiertes $C_3$-$C_8$-Cycloalkylaminocarbonyl, ein gegebenenfalls substituiertes $C_6$-$C_{10}$-Arylaminocarbonyl, ein

gegebenenfalls substituiertes $C_6$-$C_{10}$-Aryl-$C_1$-$C_6$-alkylaminocarbonyl oder ein gegebenenfalls substituiertes Heteroarylaminocarbonyl;

$R^6$ ein gegebenenfalls substituiertes $C_1$-$C_{10}$-Alkylaminocarbonylamino, $C_2$-$C_{10}$-Alkenylaminocarbonylamino oder $C_2$-$C_{10}$-Alkinylaminocarbonylamino, ein gegebenenfalls substituiertes $C_3$-$C_8$-Cycloalkylaminocarbonylamino, ein gegebenenfalls substituiertes $C_6$-$C_{10}$-Arylaminocarbonylamino, ein gegebenenfalls substituiertes $C_6$-$C_{10}$-Aryl-$C_1$-$C_6$-alkylaminocarbonylamino oder ein gegebenenfalls substituiertes Heteroarylaminocarbonylamino;

$R^6$ ein gegebenenfalls substituiertes $C_1$-$C_{10}$-Alkyloxycarbonylamino, $C_2$-$C_{10}$-Alkenyloxycarbonylamino oder $C_2$-$C_{10}$-Alkinyloxycarbonylamino, ein gegebenenfalls substituiertes $C_3$-$C_8$-Cycloalkyloxycarbonylamino, ein gegebenenfalls substituiertes $C_6$-$C_{10}$-Alyloxycarbonylamino, ein gegebenenfalls substituiertes $C_6$-$C_{10}$-Aryl-$C_1$-$C_6$-alkyloxycarbonylamino oder ein gegebenenfalls substituiertes Heteroaryloxycarbonylamino;

$R^6$ ein gegebenenfalls substituiertes $C_1$-$C_{10}$-Alkylaminocarbonyloxy, $C_2$-$C_{10}$-Alkenylaminocarbonyloxy oder $C_2$-$C_{10}$-Alkinylaminocarbonyloxy, ein gegebenenfalls substituiertes $C_3$-$C_8$-Cycloalkylaminocarbonyloxy, ein gegebenenfalls substituiertes $C_6$-$C_{10}$-Arylaminocarbonyloxy, ein gegebenenfalls substituiertes $C_6$-$C_{10}$-Aryl-$C_1$-$C_6$-alkylaminocarbonyloxy oder ein gegebenenfalls substituiertes Heteroarylaminocarbonyloxy;

$R^6$ ein gegebenenfalls substituiertes $C_1$-$C_{10}$-Alkyl-N-amidino, $C_2$-$C_{10}$-Alkenyl-N-amidino- oder $C_2$-$C_{10}$-Alkinyl-N-amidino, ein gegebenenfalls substituiertes $C_3$-$C_8$-Cycloalkyl-N-amidino, ein gegebenenfalls substituiertes $C_6$-$C_{10}$-Aryl-N-amidino, ein gegebenenfalls substituiertes $C_6$-$C_{10}$-Aryl-$C_1$-$C_6$-alkyl-N-amidino oder ein gegebenenfalls substituiertes Heteroaryl-N-amidino;

$R^6$ ein gegebenenfalls substituiertes $C_1$-$C_{10}$-Alkyloxy, $C_2$-$C_{10}$-Alkenyloxy oder $C_2$-$C_{10}$-Alkinyloxy, ein gegebenenfalls substituiertes $C_3$-$C_8$-Cycloalkyloxy, ein gegebenenfalls substituiertes $C_6$-$C_{10}$-Aryloxy, ein gegebenenfalls substituiertes $C_6$-$C_{10}$-Aryl-$C_1$-$C_6$-alkyloxy oder ein gegebenenfalls substituiertes Heteroaryloxy;

$R^6$ ein gegebenenfalls substituiertes $C_1$ -$C_{10}$-Alkylcarbonyl, ein gegebenenfalls substituiertes $C_2$-$C_{10}$-Alkenylcarbonyl, ein gegebenenfalls substituiertes $C_2$-$C_{10}$-Alkinylcarbonyl, ein gegebenenfalls substituiertes $C_3$-$C_8$-Cycloalkylcarbonyl, ein gegebenenfalls substituiertes $C_6$-$C_{10}$-Arylcarbonyl, ein gegebenenfalls substituiertes $C_6$-$C_{10}$-Aryl-$C_1$-$C_6$-alkylcarbonyl;

$R^6$ ein Rest A-CO-, worin A die zuvor genannte Bedeutung hat;

$R^6$ ein gegebenenfalls substituiertes $C_1$-$C_{10}$-Alkyloxycarbonyl, $C_2$-$C_{10}$-Alkenyloxycarbonyl oder $C_2$-$C_{10}$-Alkinyloxycarbonyl, ein gegebenenfalls substituiertes $C_3$-$C_8$-Cycloalkyloxycarbonyl, ein gegebenenfalls substituiertes $C_6$-$C_{10}$-Aryloxycarbonyl, ein gegebenenfalls substituiertes $C_6$-$C_{10}$-Aryl-$C_1$-$C_6$-alkyloxycarbonyl oder ein gegebenenfalls substituiertes Heteroaryloxycarbonyl;

$R^6$ ein gegebenenfalls substituiertes $C_1$-$C_{10}$-Alkylcarbonyloxy, $C_2$-$C_{10}$-Alkenylcarbonyloxy- oder $C_2$-$C_{10}$-Alkinylcarbonyloxy, ein gegebenenfalls substituiertes $C_3$-$C_8$-Cycloalkylcarbonyloxy, ein gegebenenfalls substituiertes $C_6$-$C_{10}$-Arylcarbonyloxy, ein gegebenenfalls substituiertes $C_6$-$C_{10}$-Aryl-$C_1$-$C_6$-alkylcarbonyloxy oder ein gegebenenfalls substituiertes Heteroarylcarbonyloxy;

$R^6$ ein gegebenenfalls substituiertes $C_1$-$C_{10}$-Alkylthio, $C_2$-$C_{10}$ Alkenylthio- oder $C_2$-$C_{10}$-Alkinylthio, ein gegebenenfalls substituiertes $C_3$-$C_8$-Cycloalkylthio, ein gegebenenfalls substituiertes $C_6$-$C_{10}$-Arylthio, ein gegebenenfalls substituiertes $C_6$-$C_{10}$-Aryl-$C_1$-$C_6$-alkylthio oder ein gegebenenfalls substituiertes Heteroarylthio;

$R^6$ ein gegebenenfalls substituiertes $C_1$-$C_{10}$-Alkylcarbonyloxy-$C_1$-$C_6$-alkyl, $C_2$-$C_{10}$-Alkenylcarbonyloxy-$C_1$-$C_6$-alkyl oder $C_2$-$C_{10}$-Alkinylcarbonyloxy-$C_1$-$C_6$-alkyl, ein gegebenenfalls substituiertes $C_3$-$C_8$-Cycloalkycarbonyloxy-$C_1$-$C_6$-alkyl, ein gegebenenfalls substituiertes $C_6$-$C_{10}$-Arylcarbonyloxy-$C_1$-$C_6$-alkyl, $C_6$-$C_{10}$-Aryl-$C_1$-$C_6$-alkylcarbonyloxy-$C_1$-$C_6$-alkyl oder Heteroarylcarbonyloxy-$C_1$-$C_6$-alkyl;

$R^6$ ein gegebenfalls substituierter Rest der Formel

$$R^{10}-O-B-(CH_2)_n-$$

mit n=1, 2, 3 oder 4, wobei B ein $C_6$-$C_{10}$-Aryl oder eine Einfachbindung bedeutet;

$R^6$ ein gegebenenfalls substituiertes Amin, bevorzugt $NR^8R^9$;

$R^6$ ein gegebenenfalls substituierte Rest der Formel

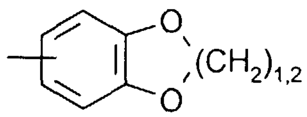

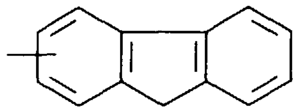

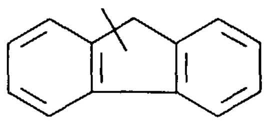

$R^6$ ein gegebenenfalls substituierter Rest der Formel

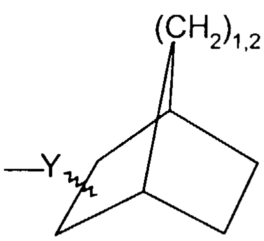

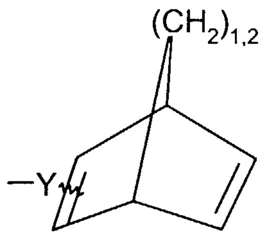

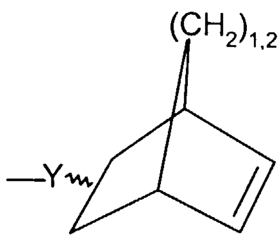

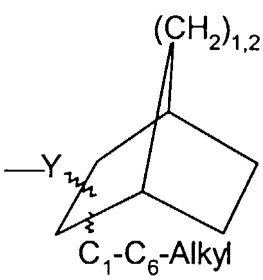

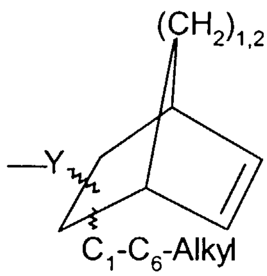

wobei Y eine Einfachbindung oder ein Alkylen, ein Alkenylen oder ein Alkinylen mit bis zu 6, bevorzugt 4 Kohlenstoffatomen in der Kette ist;

$R^6$ ein gegebenenfalls substituierter Rest der Formel

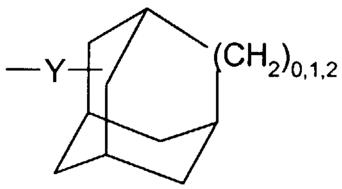

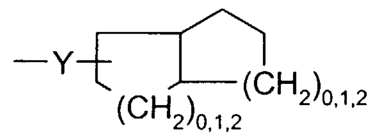

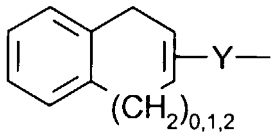

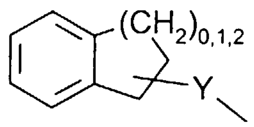

wobei Y eine Einfachbindung oder ein Alkylen, ein Alkenylen oder ein Alkinylen mit bis zu 6, bevorzugt 4 Kohlenstoffatomen in der Kette ist;

$R^8$ Wasserstoff, eine gegebenenfalls substituierte Cycloalkylgruppe mit 3 bis 6 Kohlenstoffatomen, eine verzweigte oder unverzweigte Alkyl-, Alkenyl- oder Alkinylgruppe mit bis zu 10 - bevorzugt eine Alkylgruppe mit 1 - 4 - Kohlenstoffatomen, die gegebenenfalls durch Hydroxy, Phenyl, substituiertes Phenyl, Benzyl, substituiertes Benzyl, Amino, substituiertes Amino, $C_1$ bis $C_8$ - bevorzugt $C_1$ bis $C_4$-Alkyloxy substituiert sein kann, $-(CH_2)_m-NHCOOR^{10}$ mit m = 1, 2, 3 oder 4;

$R^8$ ein direkt oder über eine Alkylkette mit 1 bis 4 C-Atomen C-verknüpfter 5, 6 oder 7-gliedriger Heterocyclus, der ein oder mehrere Heteroatome aus der Gruppe Stickstoff, Sauerstoff oder Schwefel enthält und gegebenenfalls ein oder mehrfach, bevorzugt einfach, durch Benzyl, gegebenenfalls substituiertes Benzyl, $C_1$-$C_4$-Alkyl, Halogen, $-OR^{10}$, -CN, $-NO_2$, $-NH_2$, -OH, =O, -COOH, $-SO_3H$, $-COOR^{10}$ substituiert sein kann;

$R^8$ ein bicyclischer direkt oder über eine Alkylkette mit 1 bis 4 C-Atomen C-verknüpfter Heterocyclus, der ein oder mehrere Heteroatome aus der Gruppe Stickstoff, Sauerstoff oder Schwefel enthält und gegebenenfalls ein oder mehrfach, bevorzugt einfach, durch Benzyl, gegebenenfalls substituiertes Benzyl, $C_1$-$C_4$-Alkyl, Halogen, $-OR^{10}$, -CN, $-NO_2$, $-NH_2$, -OH, =O, -COOH, $-SO_3H$, $-COOR^{10}$ substituiert sein kann;

$R^9$ Wasserstoff, eine gegebenenfalls substituierte Cycloalkylgruppe mit 3 bis 6 Kohlenstoffatomen, eine verzweigte oder unverzweigte Alkyl-, Alkenyl- oder Alkinylgruppe mit bis zu 10 - bevorzugt 1 - 4 - Kohlenstoffatomen, die gegebenenfalls durch Hydroxy, Phenyl, substituiertes Phenyl, Benzyl, substituiertes Benzyl, Amino, substituiertes Amino, $C_1$ bis $C_8$, bevorzugt $C_1$ bis $C_4$-Alkoxy substituiert sein kann, $-(CH_2)_m-NHCOOR^{10}$ mit m 1, 2, 3 oder 4;

$R^9$ ein direkt oder über eine Alkylkette mit 1 bis 4 C-Atomen C-verknüpfter 5, 6 oder 7-gliedriger Heterocyclus, der ein oder mehrere Heteroatome aus der Gruppe Stickstoff, Sauerstoff oder Schwefel enthält und gegebenenfalls ein oder mehrfach, bevorzugt einfach, durch Benzyl, gegebenenfalls substituiertes Benzyl, $C_1$-$C_4$-Alkyl, Halogen, $-OR^{10}$, -CN, $-NO_2$, $-NH_2$, -OH, =O, -COOH, $-SO_3H$, $-COOR^{10}$ substituiert sein kann;

$R^9$ ein bicyclischer, direkt oder über eine Alkylkette mit 1 bis 4 C-Atomen C-verknüpfter Heterocyclus, der ein oder mehrere Heteroatome aus der Gruppe Stickstoff, Sauerstoff oder Schwefel enthält und gegebenenfalls ein oder mehrfach, bevorzugt einfach, durch Benzyl, gegebenenfalls substituiertes Benzyl, $C_1$-$C_4$-Alkyl, Halogen, $-OR^{10}$, -CN, $-NO_2$, $-NH_2$, -OH, =O, -COOH, $-SO_3H$, $-COOR^{10}$ substituiert sein kann, oder

$R^8$ und $R^9$ bilden zusammen mit dem Stickstoffatom einen gesättigten oder ungesättigten 5- oder 6-Ring, der als weitere Heteroatome Stickstoff, Sauerstoff oder Schwefel enthalten kann, wobei der Heterocyclus durch eine verzweigte oder unverzweigte Alkylgruppe mit 1 bis 4 Kohlenstoffatomen, bevorzugt Methyl, substituiert sein kann oder einen der folgenden Reste tragen kann

- $(CH_2)_n$-Phenyl,
- $(CH_2)_n$-$NH_2$, = O, ein Ketal - bevorzugt -O-$CH_2$-$CH_2$-O-,
- $(CH_2)_n$-NH-$C_1$-$C_4$-Alkyl,
- $(CH_2)_n$-N($C_1$-$C_8$-Alkyl)$_2$,
- $(CH_2)_n$-NHCOOR$^{10}$, (n = 2, 3, 4,), Halogen,
- OR$^{10}$, -CN, -$NO_2$, -$NH_2$, -$CH_2$NR$^8$R$^9$,
- OH, -COOH, -$SO_3$H, -COOR$^{10}$, -CONR$^8$R$^9$, -$SO_2$-R$^{10}$;

$R^{10}$ Wasserstoff, $C_1$-$C_4$-Alkyl, $C_2$-$C_4$-Alkenyl, $C_2$-$C_4$-Alkinyl, ein Benzyl- oder Phenyl-Rest, der gegebenenfalls ein- oder mehrfach durch $OCH_3$ substituiert ist, bedeuten können, gegebenenfalls in Form ihrer Racemate, ihrer Enantiomere, ihrer Diastereomere und ihrer Gemische, sowie gegebenenfalls ihrer pharmakologisch unbedenklichen Säureadditionssalze.

Die gestrichelten Linien zwischen den Stickstoffatomen der zuvor genannten allgemeinen Formel I sollen die Existenz einer Doppelbindung in einer von zwei möglichen Positionen beschreiben, sodaß die Reste $R^1$ und $R^3$ beziehungsweise $R^4$ und $R^5$ nicht gleichzeitig vorhanden sein können.

Die Verbindungen der allgemeinen Formel (I) bilden die folgenden Isomere:

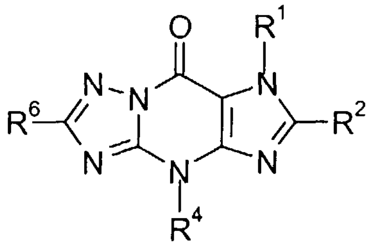

(Ia)

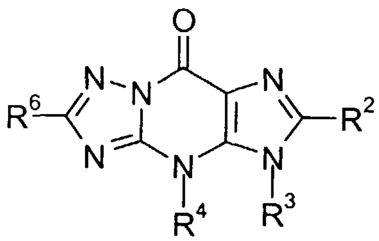

(Ib)

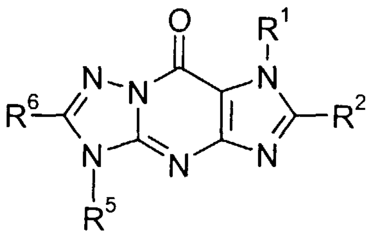

(Ic)

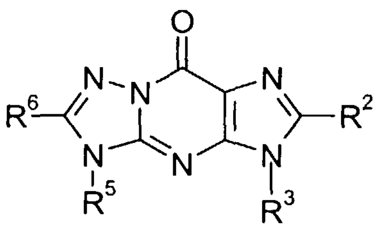

(Id)

wobei die Isomere der allgemeinen Formeln (Ia) und (Ib) bevorzugt sind, insbesondere solche worin $R^1$ oder $R^3$ Wasserstoff oder $C_1$-$C_4$-Alkyl sind. Besonders bevorzugt sind Verbindungen der allgemeinen Formel (Ia) und (Ib), worin $R^1$ und $R^3$ Wasserstoff bedeuten - in diesem Fall sind die Isomere (Ia) und (Ib) Tautomere.

Bevorzugt sind Verbindungen der allgemeinen Formel (Ia) bis (Id) worin

$R^1$ oder $R^3$ Wasserstoff, $C_1$-$C_4$-Alkyl, Benzyl, bevorzugt Wasserstoff;

$R^2$ Wasserstoff, ein $C_1$-$C_8$-Alkyl-, $C_2$-$C_8$-Alkenyl- oder $C_2$-$C_8$-Alkinyl-Rest, der durch -CN, -$CH_2$NR$^8$R$^9$, OH (auch Mehrfachsubstitution möglich), -OR$^{10}$, -NR$^8$R$^9$, -NHCOR$^{10}$, -NHCONR$^8$R$^9$, -NHCOOR$^{10}$, Halogen, -OCOR$^{10}$, -OCOPyridyl, -$OCH_2$COOH, -$OCH_2$COOR$^{10}$, -$SO_2$R$^7$, -S-R$^7$, -NHCONHPhenyl, -$OCH_2$-CONR$^8$R$^9$,-$OCH_2CH_2$OH, -$SO_2$-$CH_2$-$CH_2$-O-COR$^{10}$, -$OCH_2$-$CH_2$-NR$^8$R$^9$, -$SO_2$-$CH_2$-$CH_2$-OH, -CONH$SO_2$R$^{10}$, -$CH_2$CONH$SO_2$R$^{10}$, -$OCH_2CH_2$OR$^{10}$, -COOH, -COOR$^{10}$,-CONR$^8$R$^9$, -CHO, -SR$^{10}$, -SOR$^{10}$, -$SO_2$R$^{10}$, -$SO_3$H, -$SO_2$NR$^8$R$^9$, -$OCH_2$-$CH_2$OCOR$^{10}$, -CH=NOH, -CH=NOR$^{10}$, =O, -COR$^{11}$, -CH(OH)R$^{11}$, -CH(OR$^{10}$)$_2$, -CH=CH-R$^{12}$, OCONR$^8$R$^9$ gegebenenfalls ein- oder mehrfach - bevorzugt einfach - durch Methylsubstituiertes 1,3-Dioxolan oder 1,3-Dioxan substituiert ist;

$R^2$ Phenyl-$C_1$-$C_6$-alkyl-, bevorzugt Phenyl-$C_1$-$C_4$-alkyl-, Phenyl-$C_2$-$C_6$-alkenyl- oder Phenyl-$C_2$-$C_6$-alkinyl-,

wobei der Phenylring gegebenenfalls entweder direkt oder über eine Alkylenbrücke mit 1 bis 4 C-Atomen substituiert ist durch einen oder mehrere, bevorzugt einen, der Reste $-C_1-C_3$-Alkyl, -CN, $NR^8R^9$, $-NO_2$, -OH, $-OR^{10}$, $-CH_2-NH-SO_2-R^{10}$, $-NHCOR^{10}$, $-NHCONR^8R^9$, Halogen, $-OCOR^{10}$, -OCOPyridyl, $-OCH_2COOH$, $-OCH_2COOR^{10}$, $-CH_2OCOR^{10}$, $-SO_2R^7$, $-OCH_2-CONR^8R^9$, $-OCH_2CH_2OH$, $-OCH_2-CH_2-NR^8R^9$, $-CONHSO_2R^{10}$, $-OCH_2CH_2OR^{10}$, -COOH, $-COOR^{10}$, $-CF_3$, Cyclopropyl, $-CONR^8R^9$, $-CH_2OH$, $-CH_2OR^{10}$, -CHO, $-SR^{10}$, $-SOR^{10}$, $-SO_2R^{10}$, $-SO_3H$, $-SO_2NH^8R^9$ $-OCH_2-CH_2OCOR^{10}$, -CH=NOH, $-CH=NOR^{10}$, $-COR^{11}$, $-CH(OH)R^{11}$, $-CH(OR^{10})_2$, $-NHCOOR^{10}$, $-CH_2CONHSO_2R^{10}$, $-CH=CH-R^{12}$, $-OCONR^8R^9$, $-CH_2-O-CONR^8R^9$, $-CH_2-CH_2-O-CONR^8R^9$, gegebenenfalls ein- oder mehrfach - bevorzugt einfach - durch Methyl substituiertes 1,3-Dioxolan oder 1,3-Dioxan;

$R^2$ $C_3-C_7$-Cycloalkyl-$C_1-C_6$-alkyl-, $C_3-C_7$-Cycloalkyl-$C_2-C_6$-alkenyl-, $C_3-C_7$-Cycloalkyl-$C_2-C_6$-alkinyl-, wobei der Cycloalkylrest gegebenenfalls entweder direkt oder über eine Alkylenbrücke mit 1 bis 4 C-Atomen substituiert ist durch einen oder mehrere - bevorzugt einen-der Reste -CN, $NR^8R^9$, =O, -OH, $-OR^{10}$, $-NR^8R^9$, $-NHCOR^{10}$, $-NHCONR^8R^9$, Halogen, $-OCOR^{10}$, -OCOPyridyl, $-OCH_2COOH$, $-OCH_2COOR^{10}$, $-CH_2OCOR^{10}$, $-SO_2R^7$, $-OCH_2-CONR^8R^9$, $-OCH_2CH_2OH$, $-OCH_2-CH_2-NR^8R^9$, $-OCH_2CH_2OR^{10}$, -COOH, $-COOR^{10}$, $-CONR^8R^9$, $-CH_2OH$, $-CH_2OR^{10}$, -CHO, $-SR^{10}$, $-SOR^{10}$, $-SO_2R^{10}$, $-SO_3H$, $-SO_2NR^8R^9$, $-OCH_2-CH_2OCOR^{10}$, -CH=NOH, $-CH=NOR^{10}$, $-COR^{11}$, $-CH(OH)R^{11}$, $-CONHSO_2R^{10}$, $-CH(OR^{10})_2$, $-NHCOOR^{10}$, $-CH=CH-R^{12}$, $-OCONR^8R^9$, $-CH_2-O-CONR^8R^9$, $-CH_2-CH_2-O-CONR^8R^9$, durch Methyl substituiertes 1,3-Dioxolan oder 1,3-Dioxan;

$R^2$ einen Rest der Formel A-$C_1$ -$C_6$-Alkyl-, A-CONH-$C_1-C_6$-Alkyl-, A-CONH-$C_2-C_6$-Alkenyl-, A-CONH-$C_2-C_6$-Alkinyl-, A-NH-CO-$C_1-C_6$-Alkyl, A-NH-CO-$C_2-C_6$-Alkenyl, A-NH-CO-$C_2-C_6$-Alkinyl, A-$C_2-C_6$-Alkenylen, A-$C_2-C_6$-Alkinylen oder A-, wobei A ein C- oder N-verknüpfter 5, 6 oder 7-gliedriger Heterocyclus ist, der ein oder mehrere Heteroatome aus der Gruppe Stickstoff, Sauerstoff oder Schwefel enthält und gegebenenfalls ein oder mehrfach, bevorzugt einfach durch Benzyl, gegebenenfalls substituiertes Benzyl, $C_1-C_4$-Alkyl, Halogen, $-OR^{10}$, -CN, $-NO_2$, $-NH_2$, $-CH_2NR^8R^9$, -OH, =O, ein Ketal, Ethylenketal, -COOH, $-SO_3H$, $-COOR^{10}$, $-CONR^8R^9$, $-COR^{1\ 1}$, $-SO_2R^{10}$ oder $-CONR^8R^9$ substituiert sein kann;

$R^2$ $C_3$ - $C_7$-Cycloalkyl, bevorzugt Cyclopentyl oder Cyclohexyl, das gegebenenfalls durch = O, -OH, $-OR^{10}$, $OCOR^{10}$ oder -OCOPyridyl substituiert ist;

$R^2$ Phenyl, das gegebenenfalls durch -OH, Halogen, $-OR^{10}$, $C_1-C_4$-Alkyl, - bevorzugt $-CH_3$-, $-NH_2$, -COOH, $-SO_3H$, $-COOR^{10}$, $-OCH_2COOR^{10}$, -CN oder $-OCH_2CONR^8R^9$ substituiert ist;

$R^2$ einen gegebenenfalls durch $C_1-C_4$- Alkyl, bevorzugt Methyl substituierten Norbornan-, Norbornen-, $C_3-C_6$-Dicycloalkyl-methyl-, bevorzugt Dicyclopropylmethyl-, Adamantan- oder Noradamantan-Rest;

$R^2$ -CH=CH-Phenyl, wobei der Phenylring ein- oder mehrfach durch Methoxy, Hydroxy oder Halogen substituiert ist;

$R^2$ ein [3,3,0]-Bicyclooctan-, bevorzugt ein [3,3,0]-Bicyclooctan-2-yl;

$R^2$ ein C-verknüpftes Piperidin oder Furan;

$R^2$ ein Amin der allgemeinen Formel $NR^8R^9$;

$R^4$ oder $R^5$ Wasserstoff, ein gegebenfalls verzweigter $C_1-C_8$-Alkyl-, $C_2-C_8$-Alkenyl- oder $C_2-C_8$-Alkinyl-Rest, der durch -CN, $-CH_2NR^8R^9$, OH (auch Mehrfachsubstitution möglich), $-OR^{10}$, $-NR^8R^9$, $-NHCOR^{10}$, $-NHCONR^8R^9$, Halogen, $-OCOR^{10}$, -OCOPyridyl, $-OCH_2COOH$, $-OCH_2COOR^{10}$, $-SO_2R^7$, $-S-R^7$, -NHCONHPhenyl, $-OCH_2-CONR^8R^9$,$-OCH_2CH_2OH$, $-SO_2-CH_2-CH_2-O-COR^{10}$, $-OCH_2-CH_2-NR^8R^9$, $-SO_2-CH_2-CH_2-OH$, $-CONHSO_2R^{10}$, $-CH_2CONHSO_2R^{10}$, $-OCH_2CH_2OR^{10}$, -COOH, $-COOR^{10}$, $-CONR^8R^9$, -CHO, $-SR^{10}$, $-SOR^{10}$, $-SO_2R^{10}$, $-SO_3H$, $-SO_2NR^8R^9$, $-OCH_2-CH_2OCOR^{10}$, =O, -CH=NOH, $-CH=NOR^{10}$, $-COR^{11}$, $-CH(OH)R^{11}$, $-CH(OR^{10})_2$, $-CH=CH-R^{12}$, $OCONR^8R^9$, gegebenenfalls ein- oder mehrfach - bevorzugt einfach - durch Methylsubstituiertes 1,3-Dioxolan oder 1,3-Dioxan substituiert ist;

$R^4$ oder $R^5$ Phenyl-$C_1-C_6$-alkyl-, bevorzugt Phenyl-$C_1-C_4$-alkyl-, Phenyl-$C_2-C_6$-alkenyl- oder Phenyl-$C_2-C_6$-alkinyl-, wobei der Phenylring gegebenenfalls entweder direkt oder über eine Alkylenbrücke mit 1 bis 4 C-Atomen substituiert ist durch einen oder mehrere, bevorzugt einen, der Reste, $-C_1-C_3$-Alkyl, -CN, $NR^8R^9$, $-NO_2$,

-OH, $-OR^{10}$, $-CH_2-NH-SO_2-R^{10}$, $-NHCOR^{10}$, $-NHCONR^8R^9$, Halogen, $-OCOR^{10}$, -OCOPyridyl, $-OCH_2COOH$, $-OCH_2COOR^{10}$, $-CH_2OCOR^{10}$, $-SO_2R^7$, $-OCH_2-CONR^8R^9$, $-OCH_2CH_2OH$, $-OCH_2-CH_2-NR^8R^9$, $-CONHSO_2R^{10}$, $-OCH_2CH_2OR^{10}$, -COOH, $-COOR^{10}$, $-CF_3$, Cyclopropyl, $-CONR^8R^9$, $-CH_2OH$, $-CH_2OR^{10}$, -CHO, $-SR^{10}$, $-SOR^{10}$, $-SO_2R^{10}$, $-SO_3H$, $-SO_2NR^8R^9$, $-OCH_2-CH_2OCOR^{10}$, -CH=NOH, $-CH=NOR^{10}$, $-COR^{11}$, $-CH(OH)R^{11}$, $-CH(OR^{10})_2$, $-NHCOOR^{10}$, $-CH_2CONHSO_2R^{10}$, $-CH=CH-R^{12}$, $-OCONR^8R^9$, $-CH_2-O-CONR^8R^9$, $-CH_2-CH_2-O-CONR^8R^9$, gegebenenfalls ein- oder mehrfach - bevorzugt einfach - durch Methyl substituiertes 1,3-Dioxolan oder 1,3-Dioxan;

$R^4$ oder $R^5$ ein gegebenenfalls substituierter $C_3$-$C_7$-Cycloalkylrest;

$R^4$ oder $R^5$ $C_3$-$C_7$-Cycloalkyl-$C_1$-$C_6$-alkyl-, $C_3$-$C_7$-Cycloalkyl-$C_2$-$C_6$-alkenyl-, $C_3$-$C_7$-Cycloalkyl-$C_2$-$C_6$-alkinyl-, wobei der Cycloalkylrest gegebenenfalls entweder direkt oder über eine Alkylenbrücke mit 1 bis 4 C-Atomen durch -CN, $NR^8R^9$, =O, -OH, $-OR^{10}$, $-NR^8R^9$, $-NHCOR^{10}$, $-NHCONR^8R^9$, Halogen, $-OCOR^{10}$, -OCOPyridyl, $-OCH_2COOH$, $-OCH_2COOR^{10}$, $-CH_2OCOR^{10}$, $-SO_2R^7$, $-OCH_2-CONR^8R^9$, $-OCH_2CH_2OH$, $-OCH_2-CH_2-NR^8R^9$, $-OCH_2CH_2OR^{10}$, -COOH, $-COOR^{10}$, $-CONR^8R^9$, $-CH_2OH$, $-CH_2OR^{10}$, -CHO, $-SR^{10}$, $-SOR^{10}$, $-SO_2R^{10}$, $-SO_3H$, $-SO_2NR^8R^9$, $-OCH_2-CH_2OCOR^{10}$, -CH=NOH, $-CH=NOR^{10}$, $-COR^{11}$, $-CH(OH)R^{11}$, $-CONHSO_2R^{10}$, $-CH(OR^{10})_2$, $-NHCOOR^{10}$, $-CH=CH-R^{12}$, $-OCONR^8R^9$, $-CH_2-O-CONR^8R^9$, $-CH_2-CH_2-O-CONR^8R^9$, gegebenenfalls ein- oder mehrfach - bevorzugt einfach - durch Methyl substituiertes 1,3-Dioxolan oder 1,3-Dioxan substituiert ist;

$R^4$ oder $R^5$ einen Rest der Formel A-$C_1$-$C_6$-Alkyl, A-CONH-$C_1$-$C_6$-Alkyl, A-CONH-$C_2$-$C_6$-Alkenyl, A-CONH-$C_2$-$C_6$-Alkinyl, A-NH-CO-$C_1$-$C_6$-Alkyl, A-NH-CO-$C_2$-$C_6$-Alkenyl, A-NH-CO-$C_2$-$C_6$-Alkinyl, A-$C_2$-$C_6$-Alkenyl oder A-$C_2$-$C_6$-Alkinyl, wobei A ein C- oder N- verknüpfter Heterocyclus ist, der ein oder mehrere Heteroatome aus der Gruppe Stickstoff, Sauerstoff oder Schwefel enthält und gegebenenfalls ein oder mehrfach, bevorzugt einfach, durch $C_1$-$C_4$-Alkyl, Halogen, $-OR^{10}$, -CN, $-NO_2$, $-NH_2$, $-CH_2NR^8R^9$, -OH, =O, ein Ketal, Ethylenketal, -COOH, $-SO_3H$, $-COOR^{10}$, $-CONR^8R^9$, $-COR^{11}$, $-SO_2-R^{10}$ oder $-CONR^8R^9$ substituiert sein kann;

$R^6$ Wasserstoff, ein $C_1$-$C_8$-Alkyl-, $C_2$-$C_8$-Alkenyl- oder $C_2$-$C_8$-Alkinyl-Rest, der durch -CN, $-CH_2NR^8R^9$, OH (auch Mehrfachsubstitution möglich), $-OR^{10}$, $-NR^8R^9$, $-NHCOR^{10}$, $-NHCONR^8R^9$, $-NHCOOR^{10}$, Halogen, $-OCOR^{10}$, -OCOPyridyl, $-OCH_2COOH$, $-OCH_2COOR^{10}$, $-SO_2R^7$, $-S-R^7$, -NHCONHPhenyl, $-OCH_2-CONR^8R^9$, $-OCH_2CH_2OH$, $-SO_2-CH_2-CH_2-O-COR^{10}$, $-OCH_2-CH_2-NR^8R^9$, $-SO_2-CH_2-CH_2-OH$, $-CONHSO_2R^{10}$, $-CH_2CONHSO_2R^{10}$, $-OCH_2CH_2OR^{10}$, -COOH, $-COOR^{10}$, $-CONR^8R^9$, -CHO, $-SR^{10}$, $-SOR^{10}$, $-SO_2R^{10}$, $-SO_3H$, $-SO_2NR^8R^9$, $-OCH_2-CH_2OCOR^{10}$, -CH=NOH, $-CH=NOR^{10}$, $-COR^{11}$, $-CH(OH)R^{11}$, $-CH(OR^{10})_2$, $-CH=CH-R^{12}$, $OCONR^8R^9$ gegebenenfalls ein- oder mehrfach - bevorzugt einfach - durch Methylsubstituiertes 1,3-Dioxolan oder 1,3-Dioxan substituiert ist;

$R^6$ Phenyl-$C_1$-$C_6$-alkyl-, bevorzugt Phenyl-$C_1$-$C_4$-alkyl-, Phenyl-$C_2$-$C_6$-alkenyl- oder Phenyl-$C_2$-$C_6$-alkinyl-, wobei der Phenylring gegebenenfalls entweder direkt oder über eine Alkylenbrücke mit 1 bis 4 C-Atomen substituiert ist durch einen oder mehrere der Reste -$C_1$-$C_3$-Alkyl, -CN, $NR^8R^9$, $-NO_2$, -OH, $-OR^{10}$, $-CH_2-NH-SO_2-R^{10}$, $-NHCOR^{10}$, $-NHCONR^8R^9$, Halogen, $-OCOR^{10}$, -OCOPyridyl, $-OCH_2COOH$, $-OCH_2COOR^{10}$, $-CH_2OCOR^{10}$, $-SO_2R^7$, $-OCH_2-CONR^8R^9$, $-OCH_2CH_2OH$, $-OCH_2-CH_2-NR^8R^9$, $-CONHSO_2R^{10}$, $-OCH_2CH_2OR^{10}$, -COOH, $-COOR^{10}$, $-CF_3$, Cyclopropyl, $-CONR^8R^9$, $-CH_2OH$, $-CH_2OR^{10}$, -CHO, $-SR^{10}$, $-SOR^{10}$, $-SO_2R^{10}$, $-SO_3H$, $-SO_2NR^8R^9$, $-OCH_2-CH_2OCOR^{10}$, -CH=NOH, $-CH=NOR^{10}$, $-COR^{11}$, $-CH(OH)R^{11}$, $-CH(OR^{10})_2$, $-NHCOOR^{10}$, $-CH_2CONHSO_2R^{10}$, $-CH=CH-R^{12}$, $-OCONR^8R^9$, $-CH_2-O-CONR^8R^9$, $-CH_2-CH_2-O-CONR^8R^9$, $-CO-R^{10}$, -CO-$C_1$-$C_4$-Alkyl-$NR^8R^9$, gegebenenfalls ein- oder mehrfach - bevorzugt einfach - durch Methyl substituiertes 1,3-Dioxolan oder 1,3-Dioxan;

$R^6$ $C_3$-$C_7$-Cycloalkyl-$C_1$-$C_6$-alkyl-, $C_3$-$C_7$-Cycloalkyl-$C_2$-$C_6$-alkenyl-, $C_3$-$C_7$-Cycloalkyl-$C_2$-$C_6$-alkinyl-, wobei der Cycloalkylrest gegebenenfalls entweder direkt oder über eine Alkylenbrücke mit 1 bis 4 C-Atomen substituiert ist durch einen oder mehrere - bevorzugt einen-der Reste -CN, $NR^8R^9$, =O, -OH, $-OR^{10}$, $-NR^8R^9$, $-NHCOR^{10}$, $-NHCONR^8R^9$, Halogen, $-OCOR^{10}$, -OCOPyridyl, $-OCH_2COOH$, $-OCH_2COOR^{10}$, $-CH_2OCOR^{10}$, $-SO_2R^7$, $-OCH_2-CONR^8R^9$, $-OCH_2CH_2OH$, $-OCH_2-CH_2-NR^8R^9$, $-OCH_2CH_2OR^{10}$, -COOH, $-COOR^{10}$, $-CONR^8R^9$, $-CH_2OH$, $-CH_2OR^{10}$, -CHO, $-SR^{10}$, $-SOR^{10}$, $-SO_2R^{10}$, $-SO_3H$, $-SO_2NR^8R^9$, $-OCH_2-CH_2OCOR^{10}$, -CH=NOH, $-CH=NOR^{10}$, $-COR^{11}$, $-CH(OH)R^{11}$, $-CONHSO_2R^{10}$, $-CH(OR^{10})_2$, $-NHCOOR^{10}$, $-CH=CH-R^{12}$, $-OCONR^8R^9$, $-CH_2-O-CONR^8R^9$, $-CH_2-CH_2-O-CONR^8R^9$, durch Methyl substituiertes 1,3-Dioxolan oder 1,3-Dioxan;

$R^6$ einen Rest der Formel A-$C_1$-$C_6$-Alkyl-, A-CONH-$C_1$-$C_6$-Alkyl-, A-CONH-$C_2$-$C_6$-Alkenyl-, A-CONH-$C_2$-$C_6$-Alkinyl-, A-NH-CO-$C_1$ -$C_6$-Alkyl-, A-NH-CO-$C_2$-$C_6$-Alkenyl-, A-NH-CO-$C_2$-$C_6$-Alkinyl-, A-$C_2$-$C_6$-Alkenyl-, A-$C_2$-$C_6$-Alkinyl oder A-, wobei A ein C- oder N-verknüpfter 5, 6 oder 7-gliedriger Heterocyclus ist, der ein oder mehrere Heteroatome aus der Gruppe Stickstoff, Sauerstoff oder Schwefel enthält und gegebenenfalls ein oder mehrfach, bevorzugt einfach, durch Benzyl, gegebenenfalls substituiertes Benzyl, $C_1$-$C_4$-Alkyl, Halogen, -$OR^{10}$, -CN, -$NO_2$, -$NH_2$, -$CH_2NR^8R^9$, -OH, =O, ein Ketal, Ethylenketal, -COOH, -$SO_3H$, -$COOR^{10}$, -$CONR^8R^9$, -$COR^{11}$, -$SO_2$-$R^{10}$ oder -$CONR^8R^9$ substituiert sein kann;

$R^6$ -CHO, -$COOR^{10}$, -$CONR^8R^9$;

$R^6$ $C_3$-$C_7$-Cycloalkyl, bevorzugt Cyclopentyl oder Cyclohexyl, das gegebenenfalls durch = O,-OH, -$OR^{10}$, $OCOR^{10}$ oder -OCOPyridyl substituiert ist;

$R^6$ Phenyl, das gegebenenfalls durch -OH, Halogen, -$OR^{10}$, $C_1$-$C_4$-Alkyl, - bevorzugt -$CH_3$-, -$NH_2$, -COOH, -$SO_3H$, -$COOR^{10}$, -$OCH_2COOR^{10}$, -CN oder -$OCH_2CONR^8R^9$ substituiert ist;

$R^6$ einen gegebenenfalls durch $C_1$ - $C_4$ - Alkyl, bevorzugt Methyl substituierten Norbornan-, Norbornen-, $C_3$-$C_6$-Dicycloalkyl-methyl-, bevorzugt Dicyclopropylmethyl-, Adamantan- oder Noradamantan-Rest;

$R^6$ -CH=CH-Phenyl, wobei der Phenylring ein- oder mehrfach durch Methoxy, Hydroxy oder Halogen substituiert sein kann;

$R^6$ ein [3,3,0]-Bicyclooctan-, bevorzugt ein [3,3,0]-Bicyclooctan-2-yl;

$R^6$ ein C-verknüpftes Piperidin oder Furan;

$R^7$ $C_1$ - $C_4$-Alkyl, das gegebenfalls durch OH, $OCOR^{10}$, -OCOPyridyl, $NH_2$, $NR^8R^9$ oder $NHCOR^{10}$ substituiert ist, bevorzugt -$CH_2$-$CH_2$-OH, -$CH_2CH_2OCOR^{10}$, -$CH_2$-$CH_2$-$CH_2$-OH oder -$CH_2$-$CH_2CH_2OCOR^{10}$;

$R^8$ Wasserstoff, eine gegebenenfalls substituierte Cycloalkylgruppe mit 3 bis 6 Kohlenstoffatomen, eine verzweigte oder unverzweigte Alkyl-, Alkenyl- oder Alkinylgruppe mit bis zu 10 - bevorzugt eine Alkylgruppe mit 1 - 4 - Kohlenstoffatomen, die gegebenenfalls durch Hydroxy, Phenyl, substituiertes Phenyl, Benzyl, substituiertes Benyl, Amino, substituiertes Amino oder $C_1$-$C_8$-Alkoxy, bevorzugt $C_1$-$C_4$-Alkoxy substituiert sein kann, -$(CH_2)_m$-$NHCOOR^{10}$ mit m = 1, 2, 3 oder 4;

$R^8$ ein direkt oder über eine Alkylkette mit 1 bis 4 C-Atomen C-verknüpfter 5, 6 oder 7-gliedriger Heterocyclus, der ein oder mehrere Heteroatome aus der Gruppe Stickstoff, Sauerstoff oder Schwefel enthält und gegebenenfalls ein oder mehrfach, bevorzugt einfach, durch Benzyl, gegebenenfalls substituiertes Benzyl, $C_1$-$C_4$-Alkyl, Halogen, -$OR^{10}$, -CN, -$NO_2$, -$NH_2$, -OH, =O, -COOH, -$SO_3H$ oder -$COOR^{10}$ substituiert sein kann;

$R^8$ ein bicyclischer direkt oder über eine Alkylkette mit 1 bis 4 C-Atomen C-verknüpfter Heterocyclus, der ein oder mehrere Heteroatome aus der Gruppe Stickstoff, Sauerstoff oder Schwefel enthält und gegebenenfalls ein oder mehrfach, bevorzugt einfach, durch Benzyl, gegebenenfalls substituiertes Benzyl, $C_1$-$C_4$-Alkyl, Halogen, -$OR^{10}$, -CN, -$NO_2$, -$NH_2$, -OH, =O, -COOH, -$SO_3H$ oder -$COOR^{10}$ substituiert sein kann;

$R^9$ Wasserstoff, eine gegebenenfalls substituierte Cycloalkylgruppe mit 3 bis 6 Kohlenstoffatomen, eine verzweigte oder unverzweigte Alkyl-, Alkenyl- oder Alkinylgruppe mit bis zu 10 - bevorzugt eine Alkylgruppe mit 1 - 4 - Kohlenstoffatomen, die gegebenenfalls durch Hydroxy, Phenyl, substituiertes Phenyl, Benzyl, substituiertes Benzyl, Amino, substituiertes Amino oder $C_1$-$C_8$-Alkoxy, bevorzugt $C_1$-$C_4$-Alkoxy substituiert sein kann, -$(CH_2)_m$-$NHCOOR^{10}$ mit m 1, 2, 3 oder 4, bevorzugt Wasserstoff;

$R^9$ ein direkt oder über eine Alkylkette mit 1 bis 4 C-Atomen C-verknüpfter 5, 6 oder 7-gliedriger Heterocyclus, der ein oder mehrere Heteroatome aus der Gruppe Stickstoff, Sauerstoff oder Schwefel enthält und gegebenenfalls ein oder mehrfach, bevorzugt einfach, durch Benzyl, gegebenenfalls substituiertes Benzyl, $C_1$ -$C_4$-Alkyl, Halogen, -$OR^{10}$, -CN, -$NO_2$, -$NH_2$, -OH, =O, -COOH, -$SO_3H$ oder -$COOR^{10}$ substituiert sein kann;

$R^9$ ein bicyclischer direkt oder über eine Alkylkette mit 1 bis 4 C-Atomen C-verknüpfter Heterocyclus, der ein oder mehrere Heteroatome aus der Gruppe Stickstoff, Sauerstoff oder Schwefel enthält und gegebenenfalls ein oder mehrfach, bevorzugt einfach, durch Benzyl, gegebenenfalls substituiertes Benzyl, $C_1$-$C_4$-Alkyl, Halogen, $-OR^{10}$, -CN, $-NO_2$, $-NH_2$, -OH, =O, -COOH, $-SO_3H$ oder $-COOR^{10}$ substituiert sein kann, oder

$R^8$ und $R^9$ bilden zusammen mit dem Stickstoffatom einen gesättigten oder ungesättigten 5- oder 6-Ring, der als weitere Heteroatome Stickstoff, Sauerstoff oder Schwefel enthalten kann, wobei der Heterocyclus durch eine verzweigte oder unverzweigte Alkylgruppe mit 1 bis 4 Kohlenstoffatomen, bevorzugt Methyl substituiert sein kann oder einen der folgenden Reste tragen kann

- $(CH_2)_n$-Phenyl,
- $(CH_2)_n-NH_2$, = O, ein Ketal - bevorzugt $-O-CH_2-CH_2-O-$,
- $(CH_2)_nNH$-$C_1$-$C_4$-Alkyl,
- $(CH_2)_n-N(C_1$-$C_8$-Alkyl$)_2$,
- $(CH_2)_n-NHCOOR^{10}$, (n = 1, 2, 3, 4,), Halogen,
- $OR^{10}$, -CN, $-NO_2$, $-NH_2$, $-CH_2NR^8R^9$,
- OH, -COOH, $-SO_3H$, $-COOR^{10}$, $-CONR^8R^9$, $-SO_2-R^{10}$;

$R^{10}$ Wasserstoff, $C_1$-$C_4$-Alkyl, $C_2$-$C_4$-Alkenyl, $C_2$-$C_4$-Alkinyl, einen Benzyl- oder Phenyl-Rest, der gegebenenfalls ein- oder mehrfach durch $OCH_3$ substituiert ist;

$R^{11}$ $C_1$ -$C_4$-Alkyl, $C_2$-$C_4$-Alkenyl, $C_2$-$C_4$-Alkinyl, gegebenenfalls substituiertes Phenyl, gegebenenfalls substituiertes Benzyl, $C_3$-$C_6$-Cycloalkyl;

$R^{12}$ $-COOR^{10}$, $-CH_2OR^{10}$, $-CONR^8R^9$, Wasserstoff, $C_1$-$C_3$-Alkyl, gegebenenfalls substituiertes Phenyl, -$CH_2NR^8R^9$, bedeuten können, gegebenenfalls in Form ihrer Racemate, ihrer Enantiomere, ihrer Diastereomere und ihrer Gemische, sowie gegebenenfalls ihrer pharmakologisch unbedenklichen Säureadditionssalze.

Bevorzugt sind ferner Verbindungen der allgemeinen Formel (Ia) bis (Id)

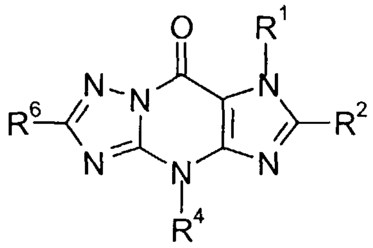

(Ia)

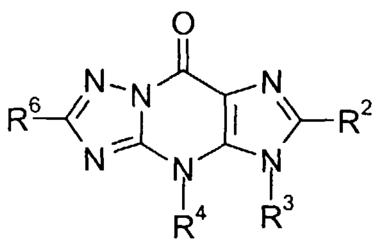

(Ib)

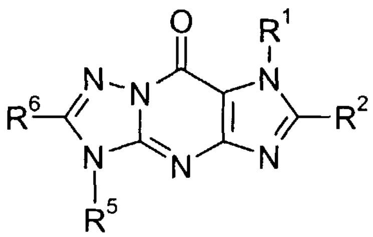

(Ic)

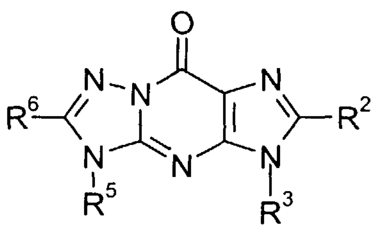

(Id)

worin

$R^1$ oder $R^3$ Wasserstoff, $C_1$-$C_4$-Alkyl, Benzyl;

$R^2$ Wasserstoff, $C_1$-$C_8$-Alkyl, bevorzugt $C_1$-$C_6$-Alkyl;

$R^2$ Phenyl, welches gegebenenfalls durch Halogen, bevorzugt Fluor oder Chlor, $C_1$-$C_4$-Alkyl, $C_1$-$C_4$-Alkyloxy, Hydroxy oder $NR^8R^9$ substituiert ist;

$R^2$ Phenyl-$C_1$-$C_6$-alkyl-, bevorzugt Benzyl, Phenyl-$C_2$-$C_6$-alkenyl- oder Phenyl-$C_2$-$C_6$-alkinyl-, wobei der Phenylring gegebenenfalls durch Halogen, bevorzugt Fluor oder Chlor, $C_1$-$C_4$-Alkyl, $C_1$-$C_4$-Alkyloxy, Hydroxy oder $NR^8R^9$ substituiert ist;

$R^2$ ein gegebenenfalls substituiertes Amin, bevorzugt $NR^8R^9$;

$R^2$ ein gegebenenfalls entweder direkt oder über eine Alkylenbrücke mit 1 bis 4 C-Atomen C- oder N-verknüpfter 5 oder 6-gliedriger Heterocyclus, der ein oder mehrere Heteroatome aus der Gruppe Stickstoff oder Sauerstoff enthält und gegebenenfalls durch Benzyl oder $C_1$-$C_4$-Alkyl substituiert sein kann;

$R^2$ ein $C_3$-$C_6$-Cycloalkyl welches gegebenenfalls durch =O, Hydroxy, $C_1$-$C_4$-Alkyl oder $C_1$-$C_4$-Alkyloxy substituiert sein kann;

$R^2$ gegebenenfalls durch $C_1$-$C_4$-Alkyl, bevorzugt Methyl substituiertes Norbornan, Norbornen, Adamantan oder Noradamantan;

$R^4$ oder $R^5$ Wasserstoff, $C_1$-$C_8$-Alkyl, Phenyl-$C_1$-$C_4$-alkyl, bevorzugt Benzyl, Phenyl-$C_2$-$C_6$-alkenyl oder Phenyl-$C_2$-$C_6$-alkinyl, wobei der Phenylring gegebenenfalls durch Halogen, bevorzugt Chlor oder Fluor, Hydroxy, $C_1$-$C_4$-Alkyl, $C_1$-$C_4$-Alkyloxy oder $NR^8R^9$ substituiert sein kann;

$R^6$ Wasserstoff, $C_1$-$C_8$-Alkyl, wobei die Alkylkette durch Halogen, Hydroxy, =O, $C_1$-$C_4$-Alkyloxy, $NR^8R^9$, Phenyloxy, -O-Phenyl-$C_1$-$C_4$-Alkyloxy, Benzyloxy, -O-Benzyl-O-$C_1$-$C_4$-Alkyloxy, -OCO-$C_1$-$C_4$-Alkyl, -OCO-Phenyl, -OCO-Benzyl, -OCO-Pyridyl, -O-$C_2$-$C_4$-Alkylen, -CO-$C_1$-$C_4$-Alkyl, -CHO, =NOH, -COOH, -COO-$C_1$-$C_4$-Alkyl, -COO-Phenyl, -COO-Benzyl, -$CONR^8R^9$, -NHCO-$C_1$-$C_4$-Alkyl, -NHCO-Phenyl, -CO-$C_1$-$C_4$-Alkyl-$NR^8R^9$, -$SO_2OH$, -$SO_2$-$C_1$-$C_4$-Alkyl, oder -$SO_2$-Phenyl substituiert sein kann;

$R^6$ Phenyl, welches gegebenenfalls durch Halogen, bevorzugt Chlor oder Fluor, Hydroxy, $C_1$-$C_4$-Alkyl, $C_1$-$C_4$-Alkyloxy, Benzyloxy, Phenyloxy, $NR^8R^9$, -OCO-$C_1$-$C_4$-Alkyl, -OCO-Phenyl, -OCO-Benzyl, -OCO-Pyridyl, -O-$C_2$-$C_4$-Alkylen, -CO-$C_1$-$C_4$-Alkyl, -$C_1$-$C_4$-Alkyl-$NH_2$, -$C_1$-$C_4$-Alkyl-OH, -$C_1$-$C_4$-Alkyl=NOH, -COOH, -COO-$C_1$-$C_4$-Alkyl, -COO-Phenyl, -COO-Benzyl, -$CONR^8R^9$, -CO-$C_1$-$C_4$-Alkyl-$NH_2$, -$SO_2OH$, -$SO_2$-$C_1$-$C_4$-Alkyl oder -$SO_2$-Phenyl substituiert sein kann;

$R^6$ Phenyl-$C_1$-$C_4$-alkyl-, bevorzugt Benzyl, Phenyl-$C_2$-$C_6$-alkenyl- oder Phenyl-$C_2$-$C_6$-alkinyl-, wobei der Phenylring gegebenenfalls durch Halogen, bevorzugt Chlor oder Fluor, Hydroxy, $C_1$-$C_4$-Alkyl, $C_1$-$C_4$-Alkyloxy, Benzyloxy, Phenyloxy, $NR^8R^9$, -OCO-$C_1$-$C_4$-Alkyl, -OCO-Phenyl, -OCO-Benzyl, -OCO-Pyridyl, -O-$C_2$-$O_4$-Alkylen, -CO-$C_1$-$C_4$-Alkyl, -$C_1$-$C_4$-Alkyl-$NR^8R^9$, -$C_1$-$C_4$-Alkyl-OH, -$C_1$-$C_4$-Alkyl=NOH, -COOH, -COO-$C_1$-$C_4$-Alkyl, -COO-Phenyl, -COO-Benzyl, -$CONR^8R^9$, -CO-$C_1$-$C_4$-Alkyl-$NH_2$, -$SO_2OH$, -$SO_2$-$C_1$-$C_4$-Alkyl oder -$SO_2$-Phenyl substituiert sein kann;

$R^6$ ein gegebenenfalls entweder direkt oder über eine Alkylenbrücke mit 1 bis 4 C-Atomen C- oder N-verknüpfter 5 oder 6-gliedriger Heterocyclus, der ein, zwei oder drei Heteroatome aus der Gruppe Stickstoff oder Sauerstoff enthält und gegebenenfalls ein oder mehrfach durch Benzyl oder $C_1$-$C_4$-Alkyl substituiert sein kann;

$R^6$ ein $C_3$-$C_6$-Cycloalkyl- oder $C_3$-$C_6$-Cycloalkyl-$C_1$-$C_4$-Alkyl-Rest, der gegebenenfalls durch =O, Hydroxy, $C_1$-$C_4$-Alkyl oder $C_1$-$C_4$-Alkyloxy substituiert sein kann;

$R^6$ gegebenenfalls durch $C_1$-$C_4$-Alkyl, bevorzugt Methyl substituiertes Norbornyl, Norbornenyl, Adamantyl oder Noradamantyl;

$R^6$ -CHO, -COOH, -COO-$C_1$-$C_4$-Alkyl, -COO-Phenyl, -COO-Benzyl oder -$CONR^8R^9$;

$R^6$ ein Amin der allgemeinen Formel $NR^8R^9$;

$R^8$ Wasserstoff, eine verzweigte oder unverzweigte Alkylgruppe mit 1 -4 - Kohlenstoffatomen;

$R^8$ ein C-verknüpfter 5 oder 6-gliedriger Heterocyclus, der ein, zwei oder drei Heteroatome aus der Gruppe Stickstoff, Sauerstoff oder Schwefel enthält und gegebenenfalls substituiert ist durch Benzyl, $C_1$-$C_4$-Alkyl, $C_1$-$C_4$-Alkyloxy, Halogen, -CN, -$NO_2$, -$NH_2$, -OH oder =O;

$R^9$ Wasserstoff, eine verzweigte oder unverzweigte Alkylgruppe mit 1 - 4 - Kohlenstoffatomen;

$R^9$ ein C-verknüpfter 5 oder 6-gliedriger Heterocyclus, der ein, zwei oder drei Heteroatome aus der Gruppe Stickstoff, Sauerstoff oder Schwefel enthält und gegebenenfalls substituiert ist durch Benzyl, $C_1$-$C_4$-Alkyl, $C_1$-$C_4$-Alkyloxy, Halogen, -CN, -$NO_2$, -$NH_2$, -OH oder =O; oder
$R^8$ und $R^9$ bilden zusammen mit dem Stickstoffatom einen gesättigten oder ungesättigten 5- oder 6-Ring, der als weitere Heteroatome Stickstoff oder Sauerstoff enthalten kann, wobei der Heterocyclus durch eine verzweigte oder unverzweigte Alkylgruppe mit 1 bis 4 Kohlenstoffatomen, bevorzugt Methyl, oder durch einen Rest -$(CH_2)_{1-4}$-Phenyl, bevorzugt Benzyl substituiert sein kann, bedeuten können, gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

Bevorzugt sind ferner Verbindungen der allgemeinen Formeln (Ia) und (Ib) worin

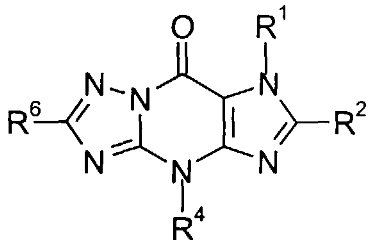
(Ia)

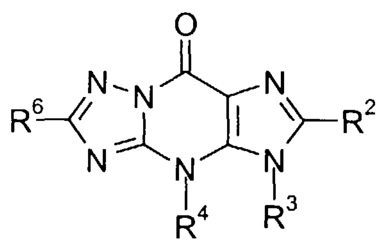
(Ib)

$R^1$ oder $R^3$ Wasserstoff, $C_1$-$C_4$-Alkyl, bevorzugt $C_1$-$C_3$-Alkyl, Benzyl;

$R^2$ Wasserstoff, $C_1$-$C_6$-Alkyl, bevorzugt $C_1$-$C_4$-Alkyl;

$R^2$ Cyclopentyl, Cyclohexyl, Cyclopentanon, Cyclohexanon, Hydroxycyclopentan oder Hydroxycyclohexan;

$R^2$ ein Morpholinrest, der gegebenenfalls durch $C_1$-$C_4$-Alkyl, bevorzugt Methyl substituiert ist, ein Piperidinylrest, ein Piperazinylrest, der gegebenenfalls durch Benzyl oder $C_1$-$C_4$-Alkyl, bevorzugt Methyl, substituiert ist, Pyridyl, Tetrahydrofuranyl, Tetrahydropyranyl oder Furyl;

$R^2$ ein Phenylrest, der gegebenenfalls durch $C_1$-$C_4$-Alkyl, Halogen oder Hydroxy substituiert ist;

$R^2$ ein Phenyl-$C_1$-$C_4$-Alkyl, bevorzugt Benzyl, wobei der Phenylring gegebenenfalls durch Halogen, bevorzugt Fluor oder Chlor, $C_1$-$C_4$-Alkyl, $C_1$-$C_4$-Alkyloxy, Hydroxy oder $NR^8R^9$ substituiert ist;

$R^2$ ein Amin der allgemeinen Formel $NR^8R^9$;

$R^2$ ein gegebenenfalls durch $C_1$-$C_4$-Alkyl, bevorzugt Methyl substituiertes Norbornenyl, Norbornyl, Adamantyl oder Noradamantyl;

$R^4$ Wasserstoff, $C_1$-$C_7$-Alkyl, bevorzugt $C_1$-$C_5$-Alkyl, Phenyl-$C_1$-$C_3$-alkyl, bevorzugt Benzyl, wobei der Phenylring durch Halogen, bevorzugt Chlor oder Fluor, Hydroxy, $C_1$-$C_4$-Alkyl, $C_1$-$C_4$-Alkyloxy oder $NR^8R^9$ substituiert sein kann;

$R^6$ Wasserstoff, $C_1$-$C_6$-Alkyl, bevorzugt $C_1$-$C_4$-Alkyl, wobei die Alkylkette durch Halogen, Hydroxy, =O, $C_1$-$C_4$-Alkyloxy, $NR^8R^9$, Phenyloxy, -O-Phenyl-O-$C_1$-$C_4$-Alkyloxy, Benzyloxy, -O-Benzyl-O-$C_1$-$C_4$-Alkyloxy, -OCO-$C_1$-$C_4$-Alkyl, -OCO-Phenyl, -OCO-Pyridyl, -OCO-Benzyl, -O-$C_2$-$C_4$-Alkylen, -CO-$C_1$-$C_4$-Alkyl, -CHO, =NOH, -COOH, -COO-$C_1$-$C_4$-Alkyl, -COO-Phenyl, -COO-Benzyl, -$CONR^8R^9$, -NHCO-$C_1$-$C_4$-Alkyl, -NHCO-Phenyl, -CO-$C_1$-$C_4$-Alkyl-$NR^8R^9$, -$SO_2OH$, -$SO_2$-$C_1$-$C_4$-Alkyl oder -$SO_2$-Phenyl, substituiert sein kann;

$R^6$ Phenyl, wobei der Phenylring durch Halogen, bevorzugt Fluor oder Chlor, Hydroxy, $C_1$-$C_4$-Alkyl, $C_1$-$C_4$-Alkyloxy oder $NR^8R^9$ substituiert sein kann;

$R^6$ Phenyl-$C_1$-$C_3$-Alkyl, bevorzugt Benzyl, wobei der Phenylring durch Halogen, bevorzugt Fluor oder Chlor, Hydroxy, $C_1$-$C_4$-Alkyl, $C_1$-$C_4$-Alkyloxy, Benzyloxy, Phenyloxy, $NR^8R^9$, -OCO-$C_1$-$C_4$-Alkyl, -OCO-Phenyl, -OCO-Benzyl, -OCO-Pyridyl, -O-$C_2$-$C_4$-Alkylen, -CO-$C_1$-$C_4$-Alkyl, -$C_1$-$C_4$-Alkyl-$NR^8R^9$, -$C_1$-$C_4$-Alkyl-OH, -$C_1$-$C_4$-Alkyl=NOH, -COOH, -COO-$C_1$-$C_4$-Alkyl, -COO-Phenyl, -COO-Benzyl, -$CONR^8R^9$, -CO-$C_1$-$C_4$-Alkyl-$NR^8R^9$, -$SO_2OH$, -$SO_2$-$C_1$-$C_4$-Alkyl oder -$SO_2$-Phenyl substituiert sein kann;

$R^6$ ein über eine Einfachbindung oder über eine Alkylenkette mit 1 bis 4 C-Atomen verknüpftes Cyclopentyl, Cyclohexyl, Cyclohexyl-$C_1$-$C_3$-alkyl, bevorzugt Cyclohexylmethyl, Cyclopentanon, Cyclohexanon, Hydroxycyclopentan oder Hydroxycyclohexan;

$R^6$ ein über eine Einfachbindung oder über eine Alkylenkette mit 1 bis 4 C-Atomen verknüpftes Furan, Tetrahydrofuran, $\alpha$-Pyran, $\gamma$-Pyran, Dioxolan, Tetrahydropyran, Dioxan, Thiophen, Thiolan, Dithiolan, Pyrrol, Pyrrolin, Pyrrolidin, Pyrazol, Pyrazolin, Imidazol, Imidazolin, Imidazolidin, Triazol, Pyridin, Piperidin, Pyridazin, Pyrimidin, Pyrazin, Piperazin, Triazin, Morpholin, Thiomorpholin, Oxazol, Isoxazol, Oxazin, Thiazol, Isothiazol, Thiadiazol, Oxadiazol oder Pyrazolidin;

$R^6$ -CHO, -COOH, -COO-$C_1$-$C_4$-Alkyl, -COO-Phenyl, -COO-Benzyl, -CO-NH-$C_1$-$C_4$-Alkyl, -CO-N($C_1$-$C_4$-Alkyl)$_2$ oder -CO-NH-Phenyl;

$R^6$ ein Amin der allgemeinen Formel $NR^8R^9$;

$R^8$ Wasserstoff, eine verzweigte oder unverzweigte Alkylgruppe mit 1 - 4 - Kohlenstoffatomen;

$R^8$ ein gegebenenfalls durch Chlor, Brom, $C_1$-$C_4$-Alkyl, $C_1$-$C_4$-Alkyloxy, $NO_2$, $NH_2$, OH substituiertes C-verknüpftes Pyrrol, Pyrrolin, Pyrrolidin, Pyrazol, Pyrazolin, Imidazol, Imidazolin, Imidazolidin, Triazol, Pyridin, Piperidin, Pyridazin, Pyrimidin, Pyrazin, Piperazin, Morpholin, Oxazol, Isoxazol, Thiazol, Isothiazol oder Thiadiazol;

$R^9$ Wasserstoff, eine verzweigte oder unverzweigte Alkylgruppe mit 1 - 4 - Kohlenstoffatomen;

$R^9$ ein gegebenenfalls durch Chlor, Brom, $C_1$-$C_4$-Alkyl, $C_1$-$C_4$-Alkyloxy, $NO_2$, $NH_2$, OH substituiertes C-verknüpftes Pyrrol, Pyrrolin, Pyrrolidin, Pyrazol, Pyrazolin, Imidazol, Imidazolin, Imidazolidin, Triazol, Pyridin, Piperidin, Pyridazin, Pyrimidin, Pyrazin, Piperazin, Morpholin, Oxazol, Isoxazol, Thiazol, Isothiazol oder Thiadiazol,
oder
$R^8$ und $R^9$ bilden zusammen mit dem Stickstoffatom einen gesättigten oder ungesättigten 5- oder 6-Ring, der als weitere Heteroatome Stickstoff oder Sauerstoff enthalten kann, wobei der Heterocyclus durch eine verzweigte oder unverzweigte Alkylgruppe mit 1 bis 4 Kohlenstoffatomen, bevorzugt Methyl, oder durch einen Rest -$(CH_2)_{1-4}$-Phenyl, bevorzugt Benzyl substituiert sein kann, bedeuten können, gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

Bevorzugt sind ferner Verbindungen der allgemeinen Formel (Ib) worin

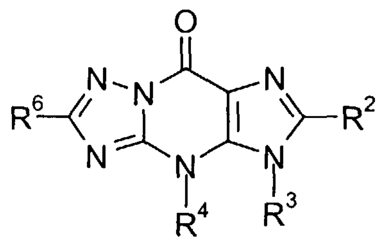

(Ib)

$R^2$ Wasserstoff, $C_1$-$C_4$-Alkyl, Phenyl, Benzyl, wobei der Phenylring gegebenenfalls durch Fluor substituiert ist, Pyridyl, Piperidinyl, Morpholinyl, Piperazinyl, 4-Benzylpiperazinyl, Furyl, Tetrahydrofuranyl, Tetrahydropyranyl, $NR^8R^9$, Cyclopentyl, Cyclohexyl, Adamantyl, Noradamantyl, Norbornyl oder Norbornenyl;

$R^3$ Wasserstoff, $C_1$-$C_3$-Alkyl oder Benzyl;

$R^4$ Wasserstoff, $C_1$-$C_5$-Alkyl oder Benzyl;

$R^6$ Wasserstoff, $C_1$-$C_4$-Alkyl, bevorzugt Methyl, welches gegebenenfalls durch OH, Chlor, Brom, $C_1$-$C_4$-Alkyloxy oder $NR^8R^9$ substituiert sein kann, -CHO, -COOH, -COO-$C_1$-$C_4$-Alkyl, bevorzugt -$COOCH_3$, Phenyl, gegebenenfalls durch Fluor oder Benzyloxy substituiertes Phenyl-$C_1$-$C_3$-Alkyl, bevorzugt Benzyl, gegebenenfalls durch Methoxy substituiertes Phenyloxy-$C_1$-$C_3$-Alkyl, bevorzugt Phenyloxymethyl, gegebenenfalls durch Methoxy substituiertes Benzyloxy-$C_1$-$C_3$-Alkyl, bevorzugt Benzyloxymethyl, Benzyloxybenzyl, Benzoyloxymethyl, Pyridylcarbonyloxymethyl, Cyclopentyl, Furyl, Cyclohexylmethyl, Pyridylmethyl, N-Pyrrolylmethyl oder N-Morpholinomethyl;

$R^8$ Wasserstoff, $C_1$-$C_4$-Alkyl oder Pyridyl;

$R^9$ Wasserstoff, $C_1$-$C_4$-Alkyl oder Pyridyl bedeuten können, gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

Bevorzugt sind ferner Verbindungen der allgemeinen Formel (Ia)

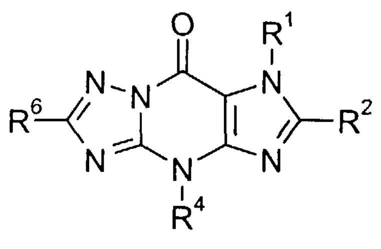

(Ia)

worin

$R^1$ Wasserstoff, $C_1$-$C_3$-Alkyl oder Benzyl;

$R^2$ Wasserstoff, $C_1$-$C_4$-Alkyl, Phenyl, Benzyl, wobei der Phenylring gegebenenfalls durch Fluor substituiert ist, Pyridyl, Piperidinyl, Morpholinyl, Piperazinyl, 4-Benzylpiperazinyl, Furyl, Tetrahydrofuranyl, Tetrahydropyranyl, $NR^8R^9$, Cyclopentyl, Cyclohexyl, Adamantyl, Noradamantyl, Norbornyl oder Norbornenyl;

$R^4$ Wasserstoff, $C_1$-$C_5$-Alkyl oder Benzyl;

$R^6$ Wasserstoff, $C_1$-$C_4$-Alkyl, bevorzugt Methyl, welches gegebenenfalls durch OH, Chlor, Brom, $C_1$-$C_4$-Alkyloxy, $NR^8R^9$ substituiert sein kann, -CHO, -COOH, -COO-$C_1$-$C_4$-Alkyl, bevorzugt -$COOCH_3$, Phenyl, gegebenenfalls durch Fluor oder Benzyloxy substituiertes Phenyl-$C_1$-$C_3$-Alkyl, bevorzugt Benzyl, gegebenenfalls durch Methoxy substituiertes Phenyloxy-$C_1$-$C_3$-Alkyl, bevorzugt Phenyloxymethyl, gegebenenfalls durch Methoxy substituiertes Benzyloxy-$C_1$-$C_3$-Alkyl, bevorzugt Benzyloxymethyl, Benzyloxybenzyl, Benzoyloxymethyl, Pyridylcarbonyloxymethyl, Cyclopentyl, Furyl, Cyclohexylmethyl, Pyridylmethyl, N-Pyrrolylmethyl oder N-Morpholinomethyl;

$R^8$ Wasserstoff, $C_1$-$C_4$-Alkyl oder Pyridyl;

$R^9$ Wasserstoff, $C_1$-$C_4$-Alkyl oder Pyridyl bedeuten können, gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

Von Interesse sind ferner Verbindungen der allgemeinen Formel (Ic) worin

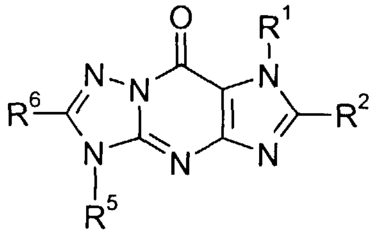

(Ic)

$R^1$ Wasserstoff oder $C_1$-$C_3$-Alkyl;

$R^2$ Wasserstoff, $C_1$-$C_4$-Alkyl, Cyclopentyl, Cyclopentanon, Hydroxycyclopentan, Furan oder Benzyl;

$R^5$ $C_1$-$C_6$-Alkyl, bevorzugt $C_1$-$C_4$-Alkyl, besonders bevorzugt Methyl, Ethyl oder tert.-Butyl;

$R^6$ Wasserstoff, Benzyl, oder Cyclopentyl
bedeuten können,
gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

Von Interesse sind ferner Verbindungen der allgemeinen Formel (Id) worin

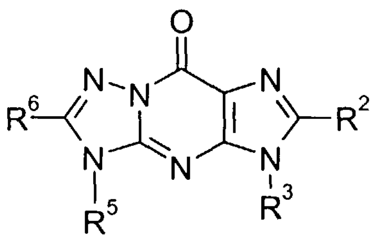

(Id)

$R^1$ Wasserstoff oder $C_1$-$C_3$-Alkyl;

$R^2$ Wasserstoff, $C_1$-$C_4$-Alkyl, Cyclopentyl, Cyclopentanon, Hydroxycyclopentan, Furan oder Benzyl;

$R^5$ $C_1$-$C_6$-Alkyl, bevorzugt $C_1$-$C_4$-Alkyl, besonders bevorzugt Methyl, Ethyl oder tert.-Butyl;

$R^6$ Wasserstoff, Benzyl, oder Cyclopentyl
bedeuten können,
gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

Bevorzugt sind ferner Verbindungen der allgemeinen Formel (Ib) worin

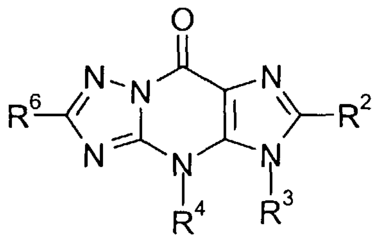

(Ib)

$R^2$ Wasserstoff, Methyl, Ethyl, n-Propyl, i-Propyl, n-Butyl, tert-Butyl, Phenyl, Benzyl, 4-Fluorbenzyl, Pyridyl, N-Piperidinyl, N-Morpholinyl, N-Piperazinyl, 4-Benzylpiperazinyl, 2-Furyl, 3-Tetrahydrofuranyl, 4-Tetrahydropyranyl, -$NMe_2$, Cyclopentyl, Cyclohexyl, Adamantan-1-yl, Noradamantan-3-yl, Norbornan-2-yl oder 5-Norbornen-2-yl;

$R^3$ Wasserstoff, Methyl, Ethyl, n-Propyl oder Benzyl;

$R^4$ Wasserstoff, Methyl, Ethyl, n-Propyl, i-Propyl, n-Butyl, tert.-Butyl, n-Pentyl oder Benzyl;

$R^6$ Wasserstoff, Methyl, Ethyl, n-Propyl, i-Propyl, n-Butyl, tert.-Butyl, Phenyl, Benzyl, Cyclopentyl, 2-Furyl, 2-Pyridylmethyl, 3-Pyridylmethyl, 4-Pyridylmethyl, Cyclohexylmethyl, Phenylethyl, N-Morpholinomethyl, N-Pyrrolylmethyl, (3-Pyridyl)-NH-$CH_2$-, PhCO-O-$CH_2$-, Pyridyl-CO-O-$CH_2$-, Ph-O-$CH_2$-, (4-MeO-Ph)-O-$CH_2$-, (4-MeO-Ph)-$CH_2$-O-$CH_2$-, (4-Ph-$CH_2$-O-Ph)-$CH_2$-, 4-F-Ph-$CH_2$-, 3,4-F-Ph-$CH_2$-, -COOH, -COOMe, -$CH_2$-OH, -$CH_2$-OMe, -$CH_2$-OEt oder -$CH_2$-$NMe_2$ bedeuten können,
gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

Von besonderem Interesse sind ferner Verbindungen der allgemeinen Formel (Ia) worin

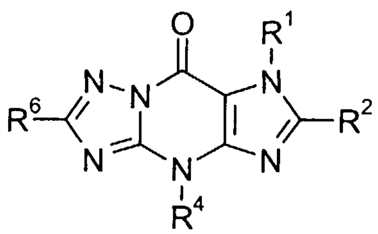

(Ia)

$R^1$ Wasserstoff, Methyl, Ethyl, n-Propyl oder Benzyl;

$R^2$ Wasserstoff, Methyl, Ethyl, n-Propyl, i-Propyl, n-Butyl, tert-Butyl, Phenyl, Benzyl, 4-Fluorbenzyl, Pyridyl, N-Piperidinyl, N-Morpholinyl, N-Piperazinyl, 4-Benzylpiperazin, 2-Furyl, 3-Tetrahydrofuranyl, 4-Tetrahydropyranyl, -$NMe_2$, Cyclopentyl, Cyclohexyl, Adamantan-1-yl, Noradamantan-3-yl, Norbornan-2-yl oder 5-Norbornen-2-yl;

$R^4$ Wasserstoff, Methyl, Ethyl, n-Propyl, i-Propyl, n-Butyl, tert.-Butyl, n-Pentyl oder Benzyl;

$R^6$ Wasserstoff, Methyl, Ethyl, n-Propyl, i-Propyl, n-Butyl, tert.-Butyl, Phenyl, Benzyl, Cyclopentyl, 2-Furyl, 2-Pyridylmethyl, 3-Pyridylmethyl, 4-Pyridylmethyl, Cyclohexylmethyl, Phenylethyl, N-Morpholinomethyl, N-Pyrrolylmethyl, (3-Pyridyl)-NH-$CH_2$-, PhCO-O-$CH_2$-, Pyridyl-CO-O-$CH_2$-, Ph-O-$CH_2$-, (4-MeO-Ph)-O-$CH_2$-, (4-MeO-Ph)-$CH_2$-O-$CH_2$-, (4-Ph-$CH_2$-O-Ph)-$CH_2$-, 4-F-Ph-$CH_2$-, 3,4-F-Ph-$CH_2$-, -COOH, -COOMe, -$CH_2$-OH, -$CH_2$-OMe, -$CH_2$-OEt oder -$CH_2$-$NMe_2$ bedeuten können,
gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

Bevorzugt sind ferner Verbindungen der allgemeinen Formel (Ib)

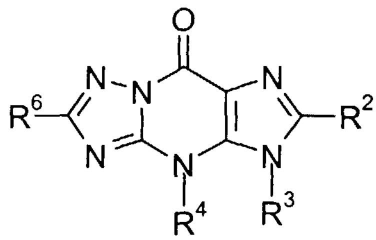

(Ib)

worin

$R^2$ Wasserstoff, Ethyl, n-Propyl, i-Propyl, n-Butyl, tert-Butyl, Phenyl, Benzyl, 2-Pyridyl, 3-Pyridyl, 4-Pyridyl, N-Piperidinyl, N-Morpholinyl, N-Piperazinyl, 4-Benzylpiperazinyl, 3-Tetrahydrofuranyl, 4-Tetrahydropyranyl, -$NMe_2$, Cyclopentyl, Cyclohexyl, Adamantan-1-yl, Noradamantan-3-yl, Norbornan-2-yl oder 5-Norbornen-2-yl;

$R^3$ Wasserstoff;

$R^4$ Wasserstoff, Methyl, Ethyl, n-Propyl, i-Propyl, n-Butyl, tert.-Butyl, n-Pentyl oder Benzyl;

$R^6$ Wasserstoff, Methyl, Ethyl, n-Propyl, i-Propyl, n-Butyl, tert-Butyl, Phenyl, Benzyl, Cyclopentyl, 2-Furyl, 2-Pyridylmethyl, 3-Pyridylmethyl, 4-Pyridylmethyl, Cyclohexylmethyl, 2-Phenylethyl, N-Morpholinomethyl, N-Pyrrolylmethyl, (3-Pyridyl)-NH-$CH_2$-, Ph-COO-$CH_2$-, 3-Pyridyl-COO-$CH_2$-, Ph-O-$CH_2$-, (4-MeO-Ph)-O-$CH_2$-, (4-MeO-Ph)-$CH_2$-O-$CH_2$-, 4-F-Ph-$CH_2$-, 3,4-F-Ph-$CH_2$-, -$CH_2$-OH, -$CH_2$-OMe, -$CH_2$-OEt, -$CH_2$-$NMe_2$, -COOMe oder -COOH bedeuten können, gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

Von besonderem Interesse sind ferner Verbindungen der allgemeinen Formel (Ia)

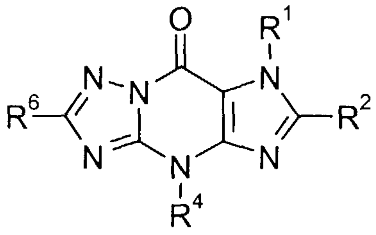

(Ia)

worin

$R^1$ Wasserstoff;

$R^2$ Wasserstoff, Ethyl, n-Propyl, i-Propyl, n-Butyl, tert-Butyl, Phenyl, Benzyl, 2-Pyridyl, 3-Pyridyl, 4-Pyridyl, N-Piperidinyl, N-Morpholinyl, N-Piperazinyl, 4-Benzylpiperazinyl, 3-Tetrahydrofuranyl, 4-Tetrahydropyranyl, $-NMe_2$, Cyclopentyl, Cyclohexyl, Adamantan-1-yl, Noradamantan-3-yl, Norbornan-2-yl oder 5-Norbornen-2-yl;

$R^4$ Wasserstoff, Methyl, Ethyl, n-Propyl, i-Propyl, n-Butyl, tert.-Butyl, n-Pentyl oder Benzyl;

$R^6$ Wasserstoff, Methyl, Ethyl, n-Propyl, i-Propyl, n-Butyl, tert.-Butyl, Phenyl, Benzyl, Cyclopentyl, 2-Furyl, 2-Pyridylmethyl, 3-Pyridylmethyl, 4-Pyridylmethyl, Cyclohexylmethyl, 2-Phenylethyl, N-Morpholinomethyl, N-Pyrrolylmethyl, (3-Pyridyl)-NH-$CH_2$-, Ph-COO-$CH_2$-, 3-Pyridyl-COO-$CH_2$-, Ph-O-$CH_2$-, (4-MeO-Ph)-O-$CH_2$-, (4-MeO-Ph)-$CH_2$-O-$CH_2$-, 4-F-Ph-$CH_2$-, 3,4-F-Ph-$CH_2$-, -$CH_2$-OH, -$CH_2$-OMe, -$CH_2$-OEt, -$CH_2$-$NMe_2$, -COOMe oder -COOH bedeuten können, gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

Zudem betrifft die Erfindung eine neue Verbindungsklasse, die Grundkörper der allgemeinen Formeln (Ia) bis (Id) enthält.

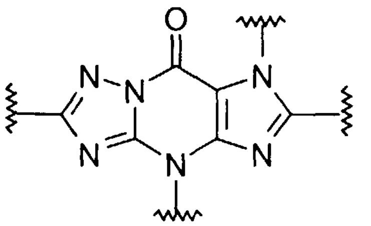

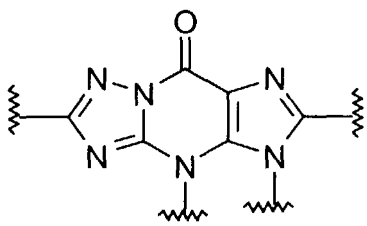

(Ia) (Ib)

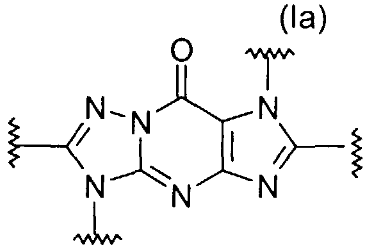

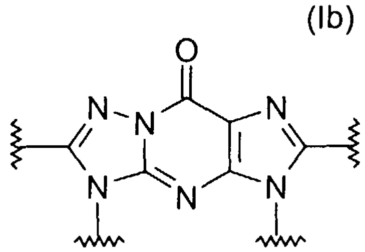

(Ic) (Id)

Bevorzugt sind Derivate der allgemeinen Formel

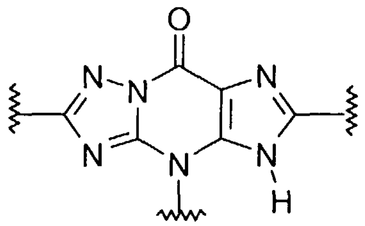

die in den Positionen 2, 4 und 6 einen Substituenten tragen.

Gegebenenfalls können die Verbindungen der allgemeinen Formel (Ia) bis (Id) in ihre Salze, insbesondere für die pharmazeutische Anwendung, in ihre physiologisch verträglichen Salze mit einer anorganischen oder organischen Säure, überführt werden. Als Säuren kommen hierfür beispielsweise Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure oder Maleinsäure in Betracht. Ferner können Mischungen der vorgenannten Säuren eingesetzt werden.

Als Alkylgruppen (auch soweit sie Bestandteil anderer Reste sind) werden verzweigte und unverzweigte Alkylgruppen mit 1 bis 10 Kohlenstoffatomen bevorzugt 1 - 4 Kohlenstoffatomen betrachtet, beispielsweise werden genannt: Methyl, Ethyl, n-Propyl, iso-Propyl, Butyl, iso-Butyl, sec. Butyl, tert.-Butyl, Pentyl, iso-Pentyl, Hexyl, Heptyl und Octyl.

Substituierte Alkylgruppen können, sofern nicht anders beschrieben (auch soweit sie Bestandteil anderer Reste sind), beispielsweise einen oder mehrere der nachfolgend genannten Substituenten tragen: Halogen, Hydroxy, Mercapto, $C_1$-$C_6$-Alkyloxy, Amino, Alkylamino, Dialkylamino, Cyano, Nitro, =O, -CHO, -COOH,

Als Alkenylgruppen (auch soweit sie Bestandteil anderer Reste sind) werden verzweigte und unverzweigte Alkenylgruppen mit 2 bis 10 Kohlenstoffatomen, bevorzugt 2 bis 3 Kohlenstoffatomen genannt, soweit sie mindestens eine Doppelbindung aufweisen, beispielsweise auch oben genannte Alkylgruppen bezeichnet, soweit sie mindestens eine Doppelbindung aufweisen, wie zum Beispiel Vinyl (soweit keine unbeständigen Enamine oder Enolether gebildet werden), Propenyl, iso-Propenyl, Butenyl, Pentenyl, Hexenyl.

Substituierte Alkenylgruppen können, sofern nicht anders beschrieben (auch soweit sie Bestandteil anderer Reste sind), beispielsweise einen oder mehrere der nachfolgend genannten Substituenten tragen: Halogen, Hydroxy, Mercapto, $C_1$-$C_6$-Alkyloxy, Amino, Alkylamino, Dialkylamino, Cyano, Nitro, =O, -CHO, -COOH, -COO-$C_1$-$C_6$-Alkyl, -S-$C_1$-$C_6$-Alkyl.

Als Alkinylgruppen (auch soweit sie Bestandteil anderer Reste sind) werden Alkinylgrupppen mit 2 bis 10 Kohlenstoffatomen bezeichnet, soweit sie mindestens eine Dreifachbindung aufweisen, beispielsweise Ethinyl, Propargyl, Butinyl, Pentinyl, Hexinyl.

Substituierte Alkinylgruppen können, sofern nicht anders beschrieben (auch soweit sie Bestandteil anderer Reste sind), beispielsweise einen oder mehrere der nachfolgend genannten Substituenten tragen: Halogen, Hydroxy, Mercapto, $C_1$-$C_6$-Alkyloxy, Amino, Alkylamino, Dialkylamino, Cyano, Nitro, =O, -CHO, -COOH, -COO-$C_1$-$C_6$-Alkyl, -S-$C_1$-$C_6$-Alkyl.

Als Cycloalkylreste mit 3 - 6 Kohlenstoffatomen werden beispielsweise Cyclopropyl, Cyclobutyl, Cyclopentyl oder Cyclohexyl bezeichnet, die auch durch verzweigtes oder unverzweigtes Alkyl mit 1 bis 4 Kohlenstoffatomen, Hydroxy, und/oder Halogen oder wie zuvor definiert substituiert sein können. Als Halogen wird im allgemeinen Fluor, Chlor, Brom oder Jod bezeichnet.

Der Begriff Aryl steht für ein aromatisches Ringsystem mit 6 bis 10 Kohlenstoffatomen, das, soweit nicht anders beschrieben, beispielsweise einen oder mehrere der nachfolgend genannten Substituenten tragen kann: $C_1$-$C_6$-Alkyl, $C_1$-$C_6$-Alkyloxy, Halogen, Hydroxy, Mercapto, Amino, Alkylamino, Dialkylamino, $CF_3$, Cyano, Nitro, -CHO, -COOH, -COO-$C_1$-$C_6$-Alkyl, -S-$C_1$-$C_6$-Alkyl. Bevorzugter Arylrest ist Phenyl.

Als Beispiele für N-verknüpfte cyclische Reste der allgemeinen Formel $NR^8R^9$ werden genannt: Pyrrol, Pyrrolin, Pyrrolidin, 2-Methylpyrrolidin, 3-Methylpyrrolidin, Piperidin, Piperazin, N-Methylpiperazin, N-Ethylpiperazin, N-(n-Propyl)-piperazin, N-Benzylpiperazin, Morpholin, Thiomorpholin, Imidazol, Imidazolin, Imidazolidin, Pyrazol, Pyrazolin, Pyrazolidin, bevorzugt Morpholin, N-Benzylpiperazin, Piperazin, und Piperidin, wobei die genannten Heterocyclen auch durch Alkyl mit 1 bis 4 Kohlenstoffatomen, bevorzugt Methyl, oder wie in den Definitionen angegeben substituiert sein können.

Als C-verknüpfte 5- oder 6-gliedrige heterocyclische Ringe, die als Heteroatome Stickstoff, Sauerstoff oder Schwefel enthalten können, werden beispielsweise Furan, Tetrahydrofuran, 2-Methyltetrahydrofuran, 2-Hydroxymethylfuran, Tetrahydrofuranon, γ-Butylrolacton, α-Pyran, γ-Pyran, Dioxolan, Tetrahydropyran, Dioxan, Thiophen, Dihydrothiophen, Thiolan, Dithiolan, Pyrrol, Pyrrolin, Pyrrolidin, Pyrazol, Pyrazolin, Imidazol, Imidazolin, Imidazolidin, Triazol,Tetrazol, Pyridin, Piperidin, Pyridazin, Pyrimidin, Pyrazin, Piperazin, Triazin, Tetrazin, Morpholin, Thiomorpholin,

Oxazol, Isoxazol, Oxazin, Thiazol, Isothiazol, Thiadiazol, Oxadiazol, Pyrazolidin genannt, wobei der Heterocyclus wie in den Definitionen angegeben substituiert sein kann.

"=O" bedeutet ein über eine Doppelbindung verknüpftes Sauerstoffatom.

Die erfindungsgemäßen Verbindungen weisen eine Affinität zu Adenosin-Rezeptoren auf und stellen somit eine neue Klasse von Adenosin-Antagonisten dar. Generell können Adenosin-Antagonisten in den Fällen eine therapeutisch nutzbare Wirkung entfalten, in denen Krankheiten oder pathologische Situationen mit einer Aktivierung von Adenosin-Rezeptoren verbunden sind.

Adenosin ist ein endogener Neuromodulator mit überwiegend hemmenden (inhibitorischen) Wirkungen im ZNS, im Herzen, in den Nieren und anderen Organen. Die Effekte von Adenosin werden über mindestens drei Rezeptor-Subtypen vermittelt: Adenosin $A_1$-, $A_2$- und $A_3$- Rezeptoren.

Im ZNS entfaltet Adenosin inhibitorische Wirkungen vorwiegend über die Aktivierung von $A_1$-Rezeptoren: praesynaptisch durch Hemmung der synaptischen Übertragung (Hemmung der Freisetzung von Neurotransmittern wie Acetylcholin, Dopamin, Noradrenalin, Serotonin, Glutamat u.a.), postsynaptisch durch Hemmung der neuronalen Aktivität.

$A_1$-Antagonisten heben die inhibitorischen Wirkungen von Adenosin auf und fördern die neuronale Transmission und die neuronale Aktivität.

$A_1$ Antagonisten sind deshalb von großem Interesse für die Therapie zentralnervöser degenerativer Erkrankungen wie senile Demenz vom Morbus Alzheimer Typ und altersassoziierte Störungen der Gedächtnis- und Lernleistungen.

Die Krankheit umfaßt neben der Vergeßlichkeit in der milden Form und der völligen Hilflosigkeit und absoluten Pflegebedürftigkeit bei der schwersten Form eine Reihe anderer Begleitsymptome wie Schlafstörungen, Moto-Koordinationsstörungen bis zum Bild eines Morbus Parkinson, ferner eine erhöhte Affektlabilität sowie auch depressive Symptome. Die Krankheit ist progredient und kann zum Tode führen. Die bisherige Therapie ist unbefriedigend. Spezifische Therapeutika fehlen bis jetzt vollständig. Therapieversuche mit Acetylcholinesterase-Inhibitoren zeigen nur bei einem geringen Teil der Patienten eine Wirkung, sind jedoch mit einer hohen Nebenwirkungsrate verbunden.

Die Pathophysiologie des M. Alzheimer und SDAT ist charakterisiert durch eine schwere Beeinträchtigung des cholinergen Systems, jedoch sind auch andere Transmittersysteme betroffen. Durch den Verlust praesynaptischer cholinerger und anderer Neurone und der daraus resultierenden mangelnden Bereitstellung von Neurotransmittern ist die neuronale Übertragung und die neuronale Aktivität in den für Lernen und Gedächtnis essentiellen Hirnarealen empfindlich vermindert.

Selektive Adenosin $A_1$-Rezeptor Antagonisten fördern die neuronale Transmission durch vermehrte Bereitstellung von Neurotransmittern, erhöhen die Erregbarkeit postsynaptischer Neurone und können damit der Krankheit symptomatisch entgegenwirken.

Die hohe Rezeptoraffinität und -Selektivität einiger der beanspruchten Verbindungen sollte es erlauben, M. Alzheimer und SDAT mit niedrigen Dosen zu therapieren, so daß kaum mit Nebenwirkungen zu rechnen ist, die nicht auf die Blockade von $A_1$-Rezeptoren zurückzuführen sind.

Eine weitere Indikation für zentralwirksame Adenosin-$A_1$-Antagonisten ist die Depression. Der Therapieerfolg antidepressiver Substanzen scheint mit einer Aufregulation von $A_1$-Rezeptoren verbunden zu sein. $A_1$-Antagonisten können zur Aufregulierung von Adenosin-$A_1$-Rezeptoren führen und somit einen neuen Therapieansatz zur Behandlung von depressiven Patienten bieten.

Weitere Einsatzgebiete insbesondere für $A_2$-selektive Adenosinantagonisten sind neurodegenerative Erkrankungen wie Morbus Parkinson und darüberhinaus die Migräne. Adenosin hemmt die Freisetzung von Dopamin aus zentralen synaptischen Endigungen durch Interaktionen mit Dopamin-$D_2$-Rezeptoren. $A_2$ Antagonisten steigern die Freisetzung und die Verfügbarkeit von Dopamin und bieten damit ein neues therapeutisches Prinzip zur Behandlung des M. Parkinson.

Bei der Migräne scheint eine über $A_2$-Rezeptoren mediierte Vasodilatation cerebraler Gefäße mitbeteiligt zu sein. Selektive $A_2$-Antagonisten hemmen die Vasodilatation und können somit nützlich zur Behandlung der Migräne sein.

Auch zur Therapie peripherer Indikationen können Adenosinantagonisten Verwendung finden.

Beispielsweise kann die Aktivierung von $A_1$-Rezeptoren in der Lunge zu einer Bronchokonstriktion führen. Selektive Adenosin $A_1$- Antagonisten relaxieren die tracheale glatte Muskulatur, bewirken eine Brochodilatation und können dadurch als Antiasthmamittel nützlich sein.

Über die Aktivierung von $A_2$-Rezeptoren kann Adenosin unter anderem eine respiratorische Depression und Atemstillstand hervorrufen. $A_2$-Antagonisten bewirken eine respiratorische Stimulation. Beispielsweise werden Adenosin-Antagonisten (Theophyllin) zur Behandlung der Atemnot und zur Vorbeugung des "plötzlichen Kindstodes" bei Frühgeburten eingesetzt.

Wichtige Therapiefelder für Adenosin-Antagonisten sind ferner kardiovaskuläre Erkrankungen und Nierenerkrankungen.

Am Herzen entfaltet Adenosin über die Aktivierung von $A_1$-Rezeptoren eine Hemmung der elektrischen und kontraktilen Aktivität. Verbunden mit einer über $A_2$-Rezeptoren mediierten koronaren Vasodilatation wirkt Adenosin negativ chronotrop,-inotrop,-dromotrop, -bathmotrop, bradykard und erniedrigt das Herzminutenvolumen.

Adenosin $A_1$-Rezeptor-Antagonisten vermögen durch Ischämie und nachfolgende Reperfusion bedingte Schädigungen am Herzen und an der Lunge zu verhindern. Deshalb könnten Adenosinantagonisten zur Prävention oder frühen Behandlung von Ischämie-Reperfusions bedingten Schädigungen des Herzenz z.B. nach coronar Bypass-Chirurgie, Herztransplantation, Angioplastie oder thrombolytischer Therapie des Herzens und ähnlicher Eingriffe eingesetzt werden. Entsprechendes gilt für die Lunge.

An den Nieren bewirkt die Aktivierung von $A_1$-Rezeptoren eine Vasokonstriktion afferenter Arteriolen und dadurch bedingt einen Abfall des renalen Blutflusses und der glomerulären Filtration.
$A_1$ Antagonisten wirken an der Niere wie starke kaliumsparende Diuretika und können somit zur Nierenprotektion sowie zur Behandlung von Oedemen, Niereninsuffizienz und akutem Nierenversagen eingesetzt werden.

Aufgrund des Adenosin-Antagonismus am Herzen und der diuretischen Wirkung können $A_1$-Antagonisten bei verschiedenen kardiovaskulären Erkrankungen therapeutisch wirksam eingesetzt werden wie z.B. bei Herzinsuffizienz, Arrhytmien (Bradyarrhytmien) assoziiert mit Hypoxie oder Ischämie, Überleitungsstörungen, Hypertonie, Ascites bei Leberversagen (hepato-renales Syndrom) und als Analgetikum bei Durchblutungsstörungen.

Überraschenderweise zeigen einige der erfindungsgemäßen Verbindungen eine Affinität zum $A_3$-Adenosin-Rezeptor. $A_3$ Antagonisten hemmen die durch $A_3$-Rezeptor-Aktivierung bedingte Degranulation von Mastzellen und sind daher therapeutisch nützlich bei allen Krankheiten und pathologischen Situationen, die in Zusammenhang mit Mastzellen-Degranulation stehen: z.B. als antiinflammatorische Substanzen, bei Überempfindlichkeitsreaktionen wie z.B. Asthma, allergischer Rhinitis, Urticaria, bei myocardialer reperfusion injury, Scleroderma, Arthritis, Autoimmun-Krankheiten, entzündlichen Darmkrankheiten u.a..
Die zystische Fibrose - auch als Mukoviszidose bekannt - ist eine erbliche Stoffwechselstörung, hervorgerufen durch einen genetischen Defekt eines bestimmten Chromosoms. Durch eine vermehrte Produktion und erhöhte Viskosität des Sekrets der mukösen Drüsen in den Bronchien kann es zu schweren Komplikationen im Bereich der Atemwege kommen. Erste Untersuchungen haben gezeigt, daß $A_1$-Antagonisten den Efflux von Chloridionen z.B. bei CF PAC Zellen erhöhen. Ausgehend von diesen Befunden kann erwartet werden, daß bei Patienten, die an zystischer Fibrose (Mukovizidose) erkrankt sind, die erfindungsgemäßen Verbindungen den gestörten Elektrolythaushalt der Zellen regulieren und die Symptome der Erkrankung gemildert werden.

Die ermittelten $A_1$-Rezeptorbindungswerte wurden in Analogie zu Ensinger et al. in "Cloning and functional characterisation of human $A_1$ adenosine Receptor - Biochemical and Biophysical Communications, Vol 187, No. 2, 919-926, 1992" bestimmt und sind in Tabelle 20 zusammengefaßt.

Die in Tabelle 21 zusammengefassten $A_3$-Rezeptorbindungswerte wurden in Analogie zu Salvatore et al. "Molecular cloning and characterization of the human $A_3$-adenosine receptor" (Proc. Natl. Acad. Sci. USA 90, 10365-10369, 1993) ermittelt.

Die neuen Verbindungen der allgemeinen Formel (Ia) bis (Id) können oral, transdermal, inhalativ oder parenteral verabreicht werden. Die erfindungsgemäßen Verbindungen liegen hierbei als aktive Bestandteile in üblichen Darreichungsformen vor, beispielsweise in Zusammensetzungen, die im wesentlichen aus einem inerten pharmazeutischen Träger und einer effektiven Dosis des Wirkstoffs bestehen, wie beispielsweise Tabletten, Dragées, Kapseln, Oblaten, Pulver, Lösungen, Suspensionen, Emulsionen, Sirupe, Suppositorien, transdermale Systeme etc.. Eine wirksame Dosis der erfindungsgemäßen Verbindungen liegt bei einer oralen Anwendung zwischen 1 und 100, vorzugsweise zwischen 1 und 50, besonders bevorzugt zwischen 5-30 mg/Dosis, bei intravenöser oder intramuskulärer Anwendung zwischen 0,001 und 50, vorzugsweise zwischen 0,1 und 10 mg/Dosis. Für die Inhalation sind erfindungsgemäß Lösungen geeignet, die 0,01 bis 1,0, vorzugsweise 0,1 bis 0,5 % Wirkstoff enthalten. Für die inhalative Applikation ist die Verwendung von Pulvern bevorzugt. Gleichfalls ist es möglich, die erfindungsgemäßen Verbindungen als Infusionslösung, vorzugsweise in einer physiologischen Kochsalzlösung oder Nährsalzlösung einzusetzen.

Die erfindungsgemäßen Verbindungen können nach folgenden Verfahren hergestellt werden. Zur Synthese der beiden Isomeren

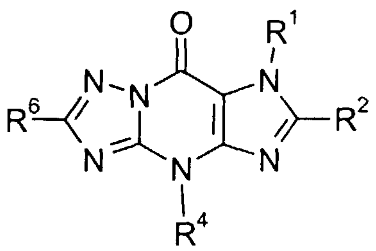

(Ia)

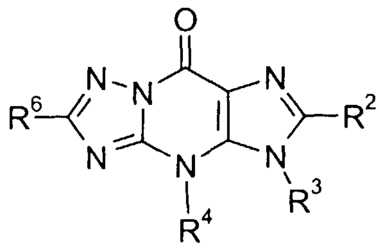

(Ib)

der allgemeinen Formel (Ia) und (Ib), wobei die Substituenten die zuvor beschriebenen Bedeutung besitzen, geht man wie folgt vor.

In einer ersten Stufe wird Aminoguanidin (1) mit einem Carbonsäurederivat der allgemeinen Formel (2) zu einem Triazol der allgemeinen Formel (3) umgesetzt (Schema 1). Diese Reaktion kann gemäß den in J. Chem. Soc. 1929, 816; J. Org. Chem. 1926, 1729 oder Org. Synthesis 26 11 veröffentlichten Vorschriften erfolgen.

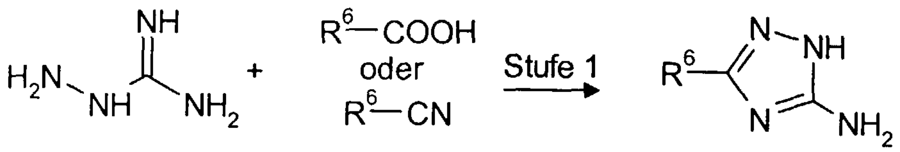


(1) (2) (3)

Schema 1:

Anschließend wird in einer zweiten Stufe das Triazol der allgemeinen Formel (3) mit Cyanessigsäurealkylester in einer Ringschlußreaktion unter alkalischen Bedingungen zu einem Triazolopyrimidin der allgemeinen Formel (4) umgesetzt (Schema 2).

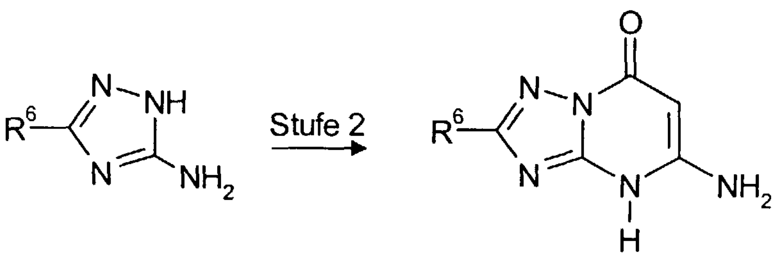


(3) (4)

Schema 2:

Als Base kommen Alkali- oder Erdalkalialkoholate beispielsweise des Methanols, Ethanols, Isopropanols, n-, sec-, tert-Butylalkohols in Betracht. Geeignete Alkali- und Erdalkalimetalle sind beispielsweise Lithium, Natrium, Kalium, Magnesium, Calzium. Natriummethanolat, Natriumethanolat, Natriumisopropanolat und Kalium-tert-butylat sind als Base besonders bevorzugt. Weiterhin kommen erfindungsgemäß als Basen Alkali- oder Erdalkalihydride in Betracht. Die Hydride des Natriums, Lithiums, Kaliums sowie des Magnesiums, Calziums sind bevorzugt. Geeignete

inerte Lösungsmittel sind Dimethylformamid, Dimethylacetamid, Methylenchlorid, Tetrahydrofuran. Ferner können Alkali- oder Erdalkalihydroxide des Lithiums, Natriums, Kaliums sowie des Magnesiums, Calziums, bevorzugt jedoch Natriumhydroxid, Kaliumhydroxid, Lithiumhydroxid und Calziumhydroxid in alkoholischer oder wässriger Lösung zum Einsatz kommen.

Neben den Cyanessigsäurealkylestern kann gleichfalls Cyanessigsäure eingesetzt werden. Die so erhaltene Mischung wird über 0,5 bis 4 Stunden, bevorzugt 1 bis 2 Stunden, bei Raumtemperatur gerührt und anschließend mit einer Verbindung der allgemeinen Formel (3) versetzt und über 2 bis 12, bevorzugt 4 bis 6 Stunden gerührt, bevorzugt unter Rückfluß erhitzt. Das Reaktionsgemisch wird dann bei Raumtemperatur mit Wasser versetzt und sauer gestellt, anschließend wird der Feststoff abfiltriert, gewaschen und getrocknet. Geeignete Säuren sind beispielsweise Ameisensäure, Essigsäure oder mineralische Säuren, wie beispielsweise Salzsäure oder Schwefelsäure.

In einer dritten Stufe werden die Reste $R^4$ und $R^5$ in das Triazolopyrimidin der allgemeinen Formel (4) eingeführt und man gelangt zu den Verbindungen der allgemeinen Formeln (5) und (12) (Schema 3).

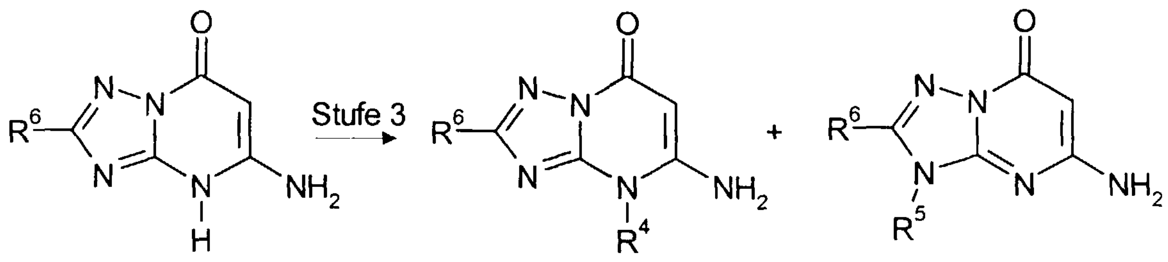


(4) (5) (12)

Schema 3:

Hierzu wird eine Verbindung der allgemeinen Formel (4) mit der 5 bis 40-fachen, bevorzugt 10 bis 20-fachen Menge eines polaren Lösungsmittels, wie Dimethylformamid, Dimethylactamid, Methylenchlorid, Tetrahydrofuran, bevorzugt Dimethylformamid und besonders bevorzugt wasserfreies, gegebenenfalls absolutes Dimethylformamid gelöst. Die so erhaltene Lösung wird mit einer Base und einem entsprechenden Alkylierungsmittel versetzt. Als Base kommen die Alkali- oder die Erdalkalicarbonate des Lithiums, Natriums, Kaliums, Calziums wie Natriumcarbonat, Lithiumcarbonat, Kaliumcarbonat, Calziumcarbonat und bevorzugt Kaliumcarbonat in Betracht. Ferner können die Hydrogencarbonate des Lithiums, Natriums und Kaliums eingesetzt werden. Ferner sind die Alkali- oder Erdalkalihydroxide des Lithiums, Natriums, Kaliums, Magnesiums, Calziums, bevorzugt jedoch Natriumhydroxid, Kaliumhydroxid, Lithiumhydroxid und Calziumhydroxid in Alkoholen oder Wasser einsetzbar. Als weitere Basen kommen die in Stufe 2 bereits aufgeführten Alkoholate der Alkali- und Erdalkalimetalle in Betracht. Weiterhin können die zuvor genannten Alkali- und Erdalkalihydride bevorzugt in inerten Lösungsmitteln wie Dimethylformamid, Dimethylacetamid, Methylenchlorid, Ethern, Tetrahydofuran und Toluol eingesetzt werden. Als Alkylierungsmittel kommen Alkylhalogenide, wie Alkylchlorid, Alkylbromid, besonders Alkyljodid sowie Alkyltosylate,- Mesylate, -Triflate, -Dialkylsulfate in Betracht. Die Alkylreste der Alkylierungsmittel entsprechen den zuvor getroffenen Definitionen für $R^4$ und $R^5$. Das Reaktionsgemisch wird 0,5 bis 4 Tage, bevorzugt 1 bis 2 Tage bei Raumtemperatur gerührt und zur Trockne eingeengt. Die Aufarbeitung kann zum einen dadurch erfolgen, daß der Rückstand mit Wasser und einem Lösemittel, beispielsweise einem halogenierten Lösungsmittel, wie Tetrachlorkohlenstoff, Methylenchlorid, bevorzugt Methylenchlorid verrührt wird, wobei die Verbindung der allgemeinen Formel (5) sich als Feststoff durch Abfiltrieren isolieren läßt. Aus dem Filtrat kann durch Entfernung des Lösungsmittels die Verbindung der allgemeinen Formel (12) gewonnen werden. Ferner kann die Aufarbeitung, für den Fall, daß kein Feststoff anfällt, erfolgen, indem zur Isolierung der Verbindung der allgemeinen Formel (12) aus dem Filtrat die Phasen des Filtrats getrennt, die wässrige Lösung mit einem halogenierten Lösungsmittel, bevorzugt Methylenchlorid, extrahiert und die vereinigten organischen Phasen getrocknet und aufgearbeitet werden. Durch chromatographische Reinigung des Rückstands erhält man die Verbindungen der allgemeinen Formeln (5) und (12) (Schema 3).

Durch Nitrosierung einer Verbindung der allgemeinen Formel (5) erhält man in der vierten Stufe eine Verbindung der allgemeinen Formel (6) (Schema 4).

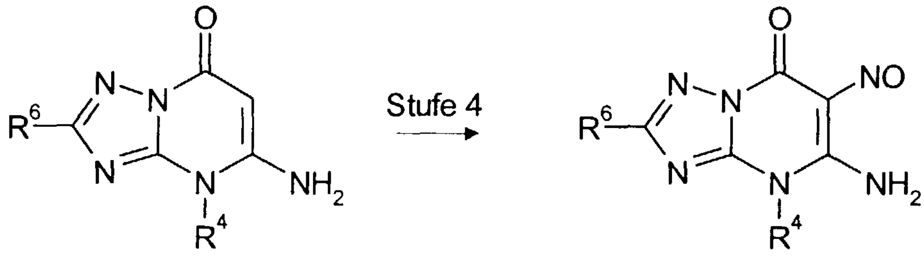

(5)

(6)

Schema 4:

Hierzu bieten sich erfindungsgemäß 2 Verfahrensvarianten an.

Stufe 4, Variante A:

Eine Verbindung der allgemeinen Formel (5) wird in einem polaren Lösungsmittel gelöst oder suspendiert, wobei als polares Lösungsmittel die zuvor genannten Lösungsmittel in Frage kommen können und Dimethylacetamid oder ein Alkohol, beispielsweise Methanol, Ethanol, Propanol, Isopropanol und Butanol bevorzugt sind. Dimethylformamid ist besonders bevorzugt. Es empfiehlt sich, das gewählte Lösungsmittel als wasserfreies, gegebenenfalls absolutes Lösungsmittel einzusetzen. Die so erhaltene Mischung wird auf unter 0° Celsius, bevorzugt -5°C, abgekühlt. Zu dieser Mischung wird ein Nitrosierungsmittel, beispielsweise ein Alkylnitrit wie Methyl-, Ethyl-, n-Propyl-, Isopropyl-, n-Butyl-, sec-Butyl-, tert-Butyl-, und Pentylnitrit, jedoch bevorzugt Isoamylnitrit gegeben, wobei die Temperatur nicht über 0°C steigen soll. Bei dieser Temperatur wird über 2 bis 48, bevorzugt 4 bis 24, besonders bevorzugt 8 bis 12 Stunden gerührt Anschließend wird gegebenenfalls bis zur Vervollständigung der Umsetzung bei Raumtemperatur weiter gerührt Das Lösungsmittel wird im Vakuum entfernt, der Rückstand mit einem Lösungsmittel, beispielsweise Diethylether, aufgenommen, filtriert, mit Wasser gewaschen und der so erhaltene Rückstand (im Vakuum) getrocknet.

Stufe 4, Variante B:

Eine Verbindung der allgemeinen Formel (5) wird mit einer wässerigen Säure versetzt, wobei als Säure organische Säuren, Ameisensäure, Essigsäure, Propionsäure, bevorzugt Essigsäure und anorganische Säuren, beispielsweise Salzsäure oder verdünnte Schwefelsäure, bevorzugt Salzsäure sowie deren Mischungen verwendet werden. Die Mischung wird auf 30 bis 100, bevorzugt 50 bis 80, besonders bevorzugt 70°C erwärmt und mit einem Nitrosierungsmittel, bevorzugt Natriumnitrit, besonders bevorzugt Natriumnitrit in Wasser, versetzt und 0,5 -2 Stunden, bevorzugt eine Stunde bei der zuvor genannten Temperatur gehalten und 0,5 -2 Stunden, bevorzugt eine Stunde bei 0 bis 20, bevorzugt 5 bis 15, besonders bevorzugt 10°C gehalten. Das Produkt wird filtriert, der Rückstand gewaschen und im Vakuum getrocknet.

In der fünften Stufe erfolgt die Reduktion der Nitrosoverbindung (6) zu einem Diamin-Derivat der allgemeinen Formel (7) (Schema 5).

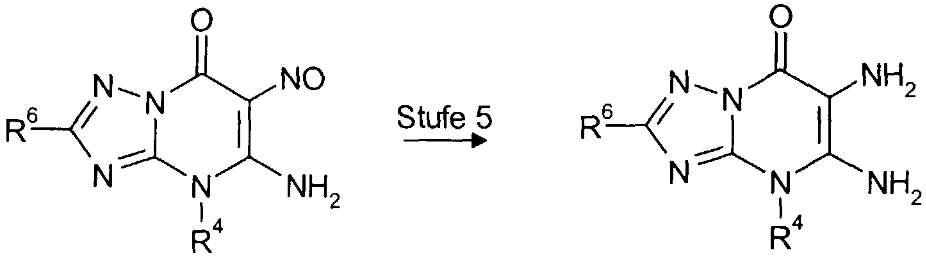

(6)

(7)

Schema 5:

Folgende Verfahrensvarianten sind hierzu u.a. anwendbar.

Stufe 5, Variante A:

Die Nitrosoverbindung (6) wird unter Rühren im Wasser suspendiert und mit einem geeigneten Reduktionsmittel versetzt. Geeignete Reduktionsmittel sind Dithionit in saurer oder alkalischer Lösung. Auch Ammoniumsulfit, Lithium-, Natrium-, Kaliumhydrogensulfit, Triethylphosphit, Triphenylphosphin oder Lithiumaluminiumhydrid können verwendet werden. Weiterhin kann die Nitrosogruppe mit Hilfe von Übergangsmetallkatalysatoren auf Basis von Palladium, Platin, Nickel oder Rhodium katalytisch mit Wasserstoffgas oder durch Transferhydrierung, beispielsweise mit Cyclohexen oder Ammoniumformiat als Wasserstoffquelle hydriert werden. Bevorzugt ist jedoch der Einsatz von Natriumdithionit. Als Basen können neben der bevorzugt eingesetzten wässrigen Ammoniaklösung gleichfalls Alkali- oder Erdalkalihydroxidlösungen, beispielsweise Lithium-, Natrium-, Kaliumhydoxid oder Magnesium-, Calzium-, Bariumhydroxidlösungen, bevorzugt jedoch die verdünnten Lösungen eingesetzt werden. Weiterhin können die Methyl-, Ethyl-, Isopropyl-, n-Propyl-, iso-Butyl-, tert-Butyl-, n-Butyl-Alkoholate der zuvor genannten Alkali- und Erdalkalimetalle verwendet werden. Diese Suspension wird auf 20 bis 100, bevorzugt 50 bis 90, besonders bevorzugt 60 bis 80°C erhitzt und mit einer Säure versetzt, bevorzugt zu Beginn, besonders bevorzugt innerhalb der ersten 30 Minuten. Mögliche Säuren sind anorganische Säuren, beispielsweise Salzsäure und Schwefelsäure sowie organische Säuren beispielsweise Ameisensäure und Essigsäure sowie deren Mischungen, wobei Schwefelsäure bevorzugt und eine 50%ige Schwefelsäure besonders bevorzugt ist. Die so erhaltene Mischung wird bis zur vollständigen Umsetzung auf Rückflußtemperatur erhitzt. Beim Abkühlen fällt das Produkt als Semisulfat aus. Weiterhin kann das Produkt durch Neutralisieren mit einer wässrigen Base, beispielsweise Lithiumhydroxid, Natriumhydroxid, Kaliumhydroxid, bevorzugt Natronlauge, besonders bevorzugt 30 Gew.-%ige Natronlauge durch Neutralisieren ausgefällt und als freie Base abfiltriert werden.

Stufe 5, Variante B:

Zu einer Mischung aus wässriger Base und einem Alkohol wird unter Rühren die Nitrosoverbindung (6) zugegeben. Anschließend wird, nachdem die Nitrosoverbindung gelöst oder suspendiert ist, das Reduktionsmittel, bevorzugt in Lösung, zugegeben. Als Reduktionsmittel kommen die in Methode A genannten Reduktionsmittel in Frage, wobei eine wässrige Lösung von Natriumdithionit besonders bevorzugt ist. Anschließend wird 0,5 bis 6, bevorzugt 1 bis 4, besonders bevorzugt 1,5 bis 3 Stunden bei Raumtemperatur gerührt. Das ausgefallene Produkt wird durch Abfiltrieren isoliert.

In der sechsten Stufe werden aus den Diaminen der allgemeinen Formel (7) die isomeren Verbindungen (8) hergestellt (Schema 6).

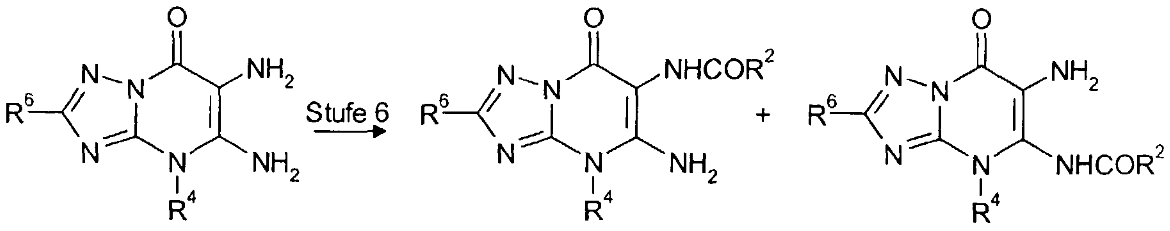


(7) (8)

Schema 6:

Hierzu wird die Aminoverbindung der allgemeinen Formel (7) in einem organischen Lösungsmittel suspendiert und wird mit einer organischen Base versetzt. Nachdem die Mischung 40 bis 60 Minuten, bevorzugt 20 bis 50 Minuten, besonders bevorzugt 25 bis 45 Minuten bei Raumtemperatur gehalten wurde, wird die Mischung auf 0 bis 15°C, bevorzugt 5 bis 10°C abgekühlt und mit einem Carbonsäurederivat, worin der Rest $R^2$ wie zuvor definiert ist, versetzt. Geeignete organische Lösungsmittel sind Dimethylformamid, Dimethylacetamid, Methylenchlorid, Toluol und Tetrahydrofuran, wobei Dimethylformamid bevorzugt und wasserfreies, gegebenenfalls absolutes Dimethylformamid besonders bevorzugt ist. Geeignete organische Basen sind Dimethylaminopyridin, Pyridin, tert. Amine, beispielsweise Trimethylamin, Triethylamin, Diisopropylethylamin, DBU (Diazabicycloundecen) oder eine der zuvor genannten anorganischen Basen, insbesondere Alkali- oder Erdalkalicarbonate oder -hydrogencarbonate. Besonders geeignet als Base ist jedoch Dimethylaminopyridin. Erfindungsgemäße Carbonsäurederivate sind Carbonsäurehalogenide, bevorzugt Carbonsäurechloride oder auch Carbonsäuren, wenn sie in geeigneter Weise aktiviert werden. Dieses kann beispielsweise durch Umsetzung mit Chlorameisensäureestern, Carbonyldiimidazol, Carbodiimiden, beispielsweise Dicyclohexylcarbodiimid, EDAC, oder Benzotriazolen, beispielsweise HOBt (1-Hydroxy-1-H-benzotriazol) und TBTU erfolgen. Gleichfalls kann an Stelle des Carbonsäurederivats auch der entsprechende Aldehyd eingesetzt werden und das Zwischenprodukt mit Eisen-(II)-chlorid, Azodicarbonsäureestern und anderen Oxidationsmitteln oxidiert werden. Anschließend wird die Mischung über 0,5 bis 4 Stunden, bevorzugt 1 bis 3 Stunden, besonders bevorzugt 2 Stunden, bei 5 bis 20, bevorzugt 7 bis 15, besonders bevorzugt 10°C und über Nacht bei Raumtemperatur gerührt und das Lösungsmittel anschließend im Vakuum entfernt. Der Rückstand wird in Wasser aufgenommen, der Feststoff abfiltriert, gewaschen und gegebenenfalls durch Umkristallisation oder Chromatographie, bevorzugt Säulenchromatographie, gereinigt.

In der siebten Stufe erfolgt die Ringschlußreaktion einer Verbindung der allgemeinen Formel (6), (7) oder (8) zur Verbindung der allgemeinen Formel (9) gemäß den Methoden A bis C (Schema 7).

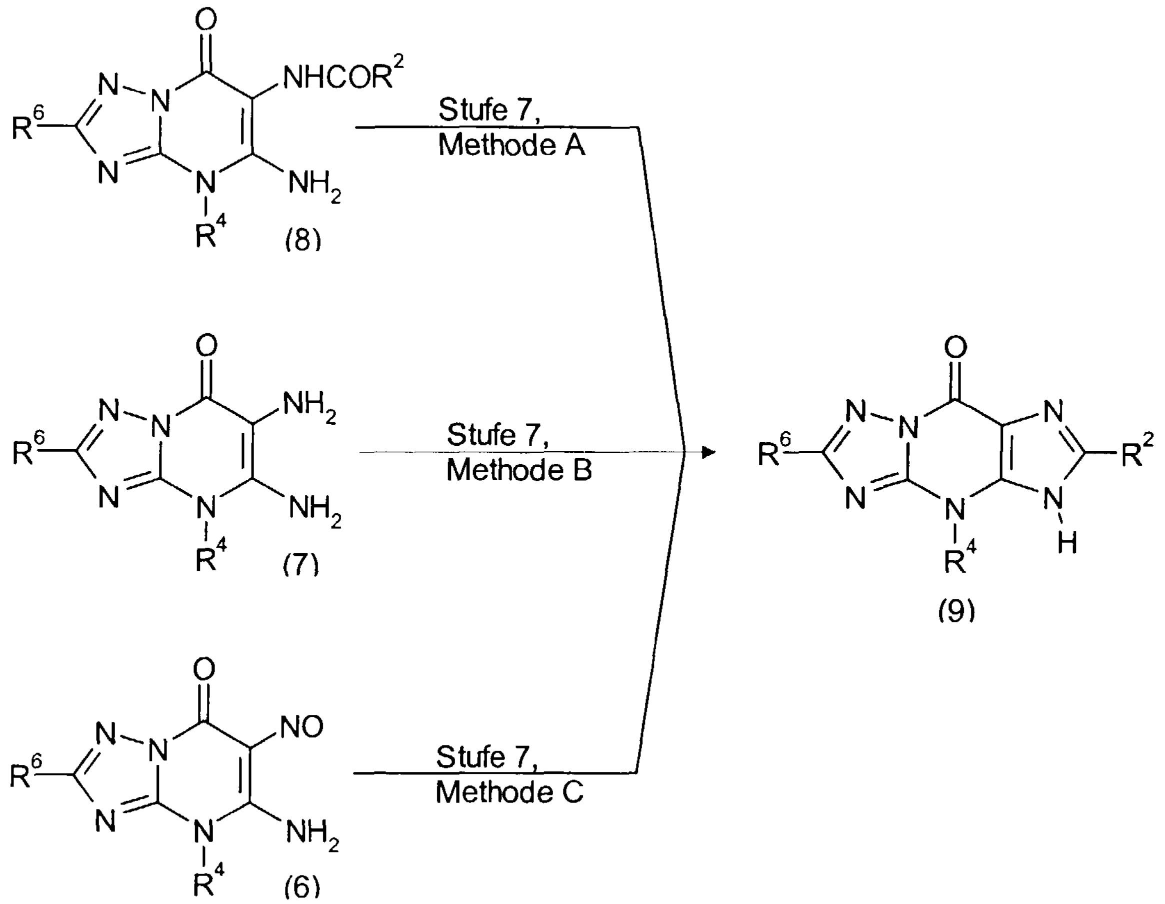


Schema 7:

Stufe 7, Methode A:

Eine Verbindung der allgemeinen Formel (8) wird in einem Alkohol suspendiert und mit einer Base 24 Stunden unter Rückfluß erhitzt. Nach dem Abkühlen wird der Ansatz sauer gestellt, der Feststoff abfiltriert und getrocknet. Gegebenenfalls wird das Produkt durch Umkristallisation oder Chromatographie, bevorzugt Säulenchromatographie, gereinigt. Als Base kommen hier die Alkali- und Erdalkalihydroxide, beispielsweise Hydroxide des Lithiums, Natriums, Kaliums, Calziums und Bariums sowie deren Mischungen als wäßrige Lösung oder gegebenenfalls in Gemischen mit einem Alkohol und/oder mit einem wassermischbaren Ether versetzt, in Frage. Geeignete Alkohole sind Methanol, Ethanol, n-Propanol, Isopropanol, n-Butanol und tert.-Butanol. Als Ether eignen sich insbesondere cyclische Ether, beispielsweise Tetrahydrofuran, Dioxan.

Nach Analogieverfahren kann der Ringschluß auch mit anorganischen Säurechloriden wie Thionylchlorid, Phosphoroxychlorid oder Phosphorpentachlorid sowie mit Polyphosphorsäure durchgeführt werden.

Ein weiteres Analogieverfahren besteht darin, das Carbonsäureamid unter sauren Bedingungen mit Mineralsäure/Methanol, bevorzugt Salzsäure/Methanol oder basischen Bedingungen, Alkalimetall/Methanol, bevorzugt Natrium/Methanol umzusetzen und anschließend thermisch zu cyclisieren.

Stufe 7, Methode B:

In einem weiteren Verfahren ist es möglich, ausgehend von dem Diamin (7) die Verbindung (9) mit $R^2$ gleich Wasserstoff herzustellen. Hierzu wird (7) mit einer equimolaren Menge, bevorzugt mit einem Überschuß Formamid über 0,5 bis 3 Stunden, bevorzugt 1 bis 2 Stunden, besonders bevorzugt 1,5 Stunden bei 50 bis 250°C, bevorzugt 100 bis 225°C, besonders bevorzugt 180 - 200°C gerührt. Das verbleibende Formamid wird im Vakuum abdestilliert und der

Rückstand in Wasser aufgenommen. Der so erhaltene Feststoff wird abfiltriert, mit Wasser gewaschen und das so erhaltene Produkt gegebenenfalls durch Umkristallisation oder Chromatographie, bevorzugt Säulenchromatographie, gereinigt.

Stufe 7, Methode C:

Ferner ist die Verbindung mit der allgemeinen Formel (9) aus der Nitrosoverbindung der allgemeinen Formel (6) direkt zugänglich, wenn $R^2$ für einen Rest $NR^8R^9$ steht, wobei $R^8$ und $R^9$ die zuvor genannte Bedeutung besitzen. Zu einer Lösung des gewünschten N-Formylamins in Diglyme gibt man bei minus 10 bis plus 20°C, bevorzugt minus 5 bis plus 5°C, besonders bevorzugt 0°C, Phosphoroxychlorid. Anschließend wird im Eisbad gerührt und die Nitrosoverbindung (6) zugesetzt. Die Mischung wird über 15 bis 120 Minuten, bevorzugt 30 bis 80 Minuten, besonders bevorzugt 40 bis 60 Minuten, bei 30 bis 120°C, bevorzugt 50 bis 100°C, besonders bevorzugt 70 bis 80°C gerührt und dann auf Eiswasser gegeben. Das feste Produkt wird abfiltriert, gewaschen und getrocknet und gegebenenfalls zur Reinigung umkristallisiert oder durch Chromatographie, bevorzugt Säulenchromatographie, gereinigt.

Die Verbindungen mit den allgemeinen Formel (Ia) und (Ib) sind in einer achten Stufe aus der Verbindung mit der allgemeinen Formel (9) zugänglich (Schema 8).

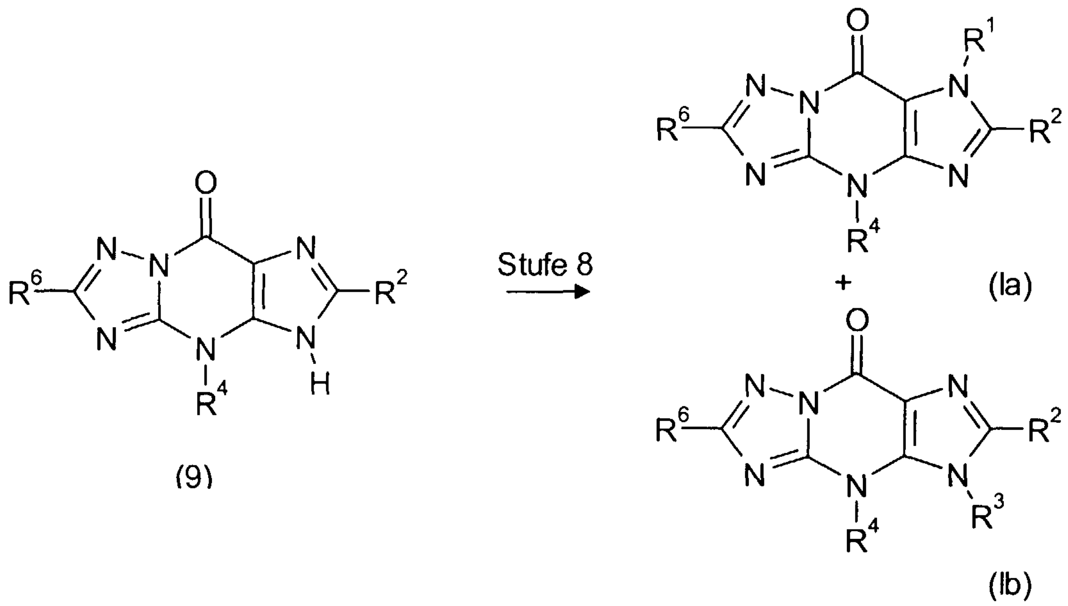


Schema 8:

Hierzu wird die Ausgangsverbindung in einem organischen Lösungsmittel aufgenommen und mit einer Base und einem geeigneten Alkylierungsmittel umgesetzt. Die Reaktionsmischung wird über Nacht bei Raumtemperatur gerührt. Gegebenenfalls kann auch bei erhöhter Temperatur gearbeitet werden. Die Reaktionsdauer kann unter Umständen bis zu einer Woche betragen. Nach vollständiger Reaktion wird der Ansatz bis zur Trockne eingeengt. Der Rückstand wird in einer wässerigen alkalischen Lösung aufgenommen, wobei als Base Lithiumhydroxid, Natriumhydroxid, Kaliumhydroxid, bevorzugt Natriumhydroxid und besonders bevorzugt 2N Natronlauge in Frage kommt. Anschließend wird die Lösung mit einem unpolaren organischen Lösungsmittel, wie Benzol, Toluol, Xylol, bevorzugt Toluol gewaschen und auf einen pH-Wert von 6 eingestellt. Der ausgefallene Feststoff wird abfiltriert und gegebenenfalls durch Chromatographie, bevorzugt Säulenchromatographie gereinigt. Auf diese Weise lassen sich sowohl die Dialkylierungsprodukte als auch die Monoalkylierungsprodukte der Verbindungen der allgemeinen Formel (Ia) und (Ib) herstellen und isolieren.

Als organische Lösungsmittel für die Alkylierungsreaktion kommen inerte Lösungsmittel, wie Dimethylformamid, Dimethylacetamid, Methylenchlorid, Alkylether, bevorzugt Diethylether, Tetrahydrofuran, Benzol, Toluol, Xylol und besonders bevorzugt Dimethylformamid in Frage. Als Basen kommen Carbonate wie Lithiumcarbonat, Natriumcarbonat, Kaliumcarbonat, Calziumcarbonat sowie Hydrogencarbonate der zuvor genannten Alkali- und Erdalkalimetalle als auch deren Hydroxide und Alkoholate in Frage. Die Alkoholate der zuvor genannten Alkali- und Erdalkalimetalle mit

Methanol, Isopropanol, tert-Butanol sind bevorzugt, wobei Natriumethanolat besonders bevorzugt ist. Im Fall der Carbonate, Hydrogencarbonate, Alkoholate und Hydroxide kann es vorteilhaft sein, Alkohol oder Wasser als Lösungsmittel einzusetzen. Als weitere Basen kommen für die Alkylierung Natriumhydrid, Kaliumhydrid, Lithiumhydrid, Calziumhydrid in den zuvorgenannten inerten Lösungsmitteln in Betracht. Weiterhin sind als Alkylierungsmittel Kohlenwasserstoffhalogenide, wie Alkylchloride, Alkylbromide, Alkyljodide sowie Kohlenwasserstoff/ bzw. Alkyltosylate, -mesylate, -triflate und die Alkylsulfate geeignet, bevorzugt sind Alkyljodide.

Ferner betrifft die Erfindung ein Verfahren zur Herstellung von Verbindungen der allgemeinen Formel (Id) und (Ic).

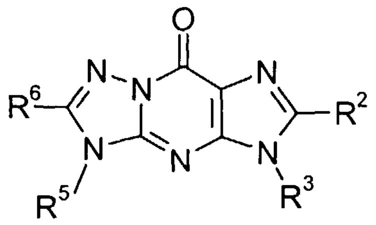

(Id)

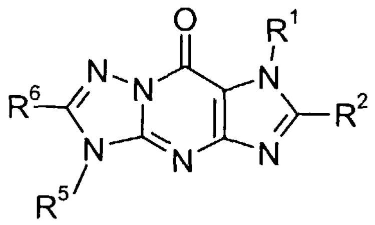

(Ic)

Ausgehend von einer Verbindung der allgemeinen Formel (12) läßt sich entsprechend der zuvor beschriebenen Umsetzung von (5) zu (6) (vgl. Schema 4, Stufe 4) eine Verbindung der allgemeinen Formel (13) erhalten (Schema 9).

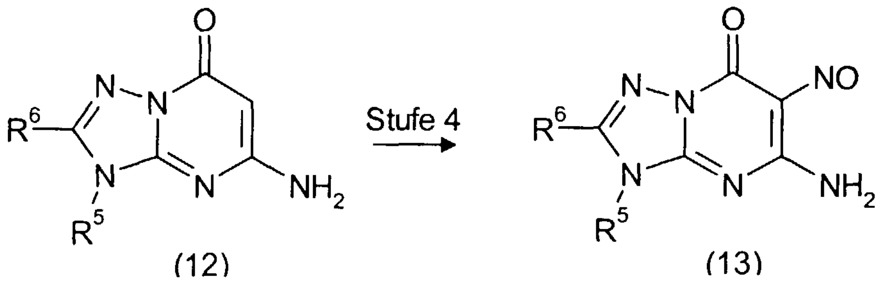


(12) (13)

Schema 9:

Diese läßt sich nach den Vorschriften der Stufe 5 (vgl. Schema 5) in eine Verbindung der allgemeinen Formel (14) überführen (Schema 10).

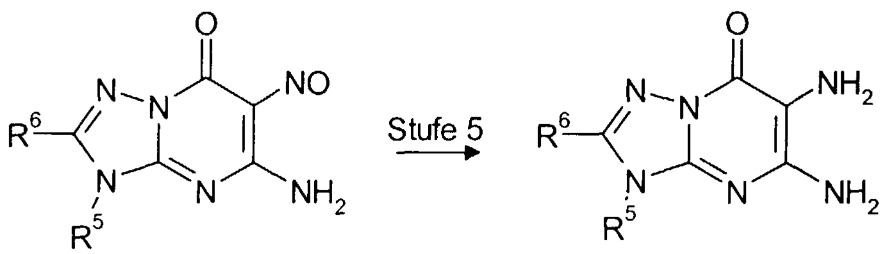


(13) (14)

Schema 10:

Entsprechend den zuvor für die Umsetzung der Verbindungen (7) zu (8) (vgl. Schema 6, Stufe 6) beschriebenen Verfahrensvarianten lassen sich die Verbindungen der allgemeinen Formel (14) in die Verbindungen der allgemeinen Formel (15) überführen (Schema 11).

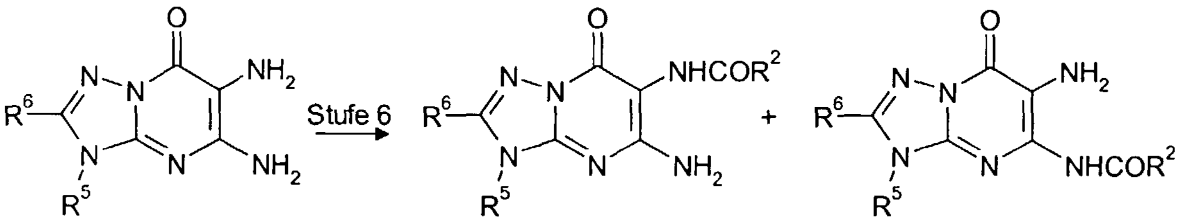


(14) (15)

Schema 11:

Daraus lassen sich in Stufe 7 entsprechend zuvor beschriebenen Ringschlußreaktionsvarianten (vgl. Schema 7) die Verbindungen der allgemeinen Formel (16) erhalten (Schema 12).

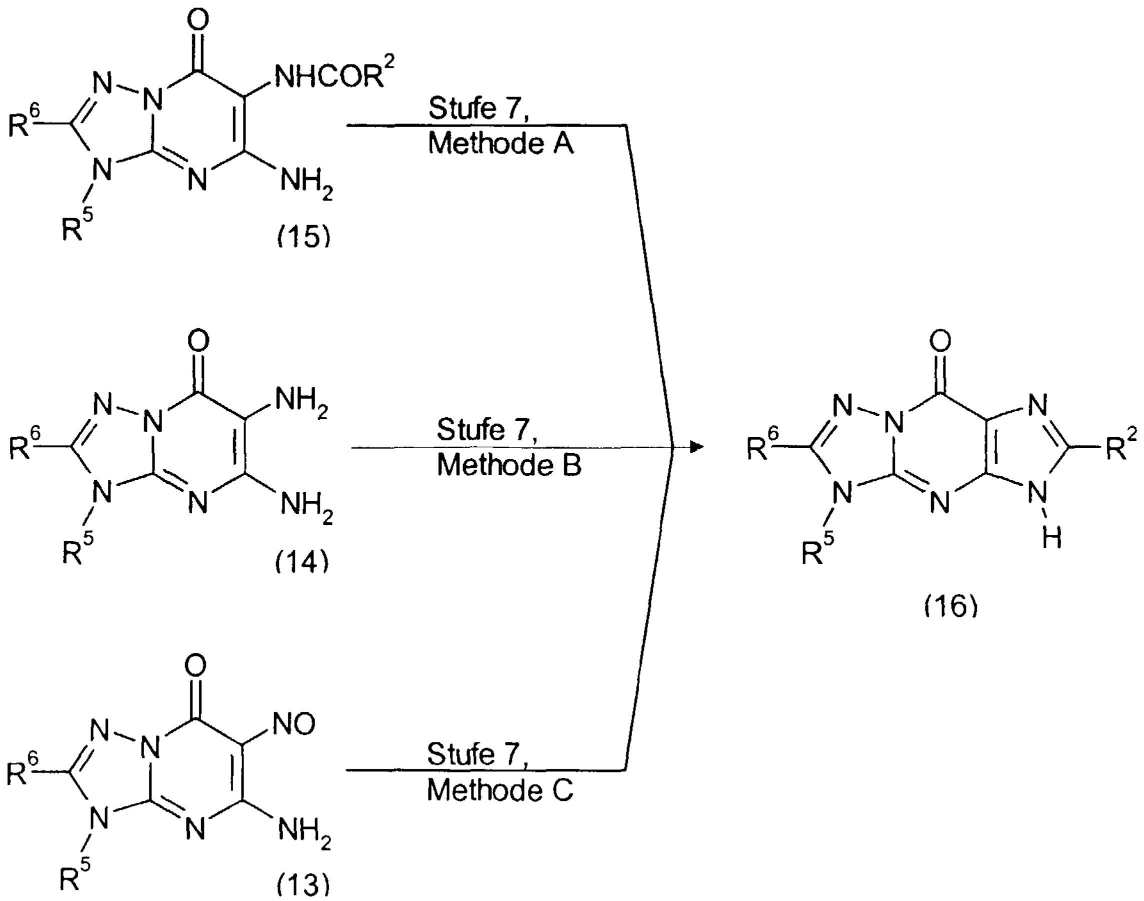


(15)

(14)

(16)

(13)

Schema 12:

Aus diesen lassen sich durch die in Stufe 3 und 8 beschriebenen Alkylierungsreaktionen die Verbindungen der allgemeinen Formeln (Ic) und (Id) herstellen, die gegebenenfalls durch geeignete Reinigungs- bzw. Trennverfahren, beispielweise Auskristallisieren oder Säulenchromatographie, isoliert werden (Schema 13).

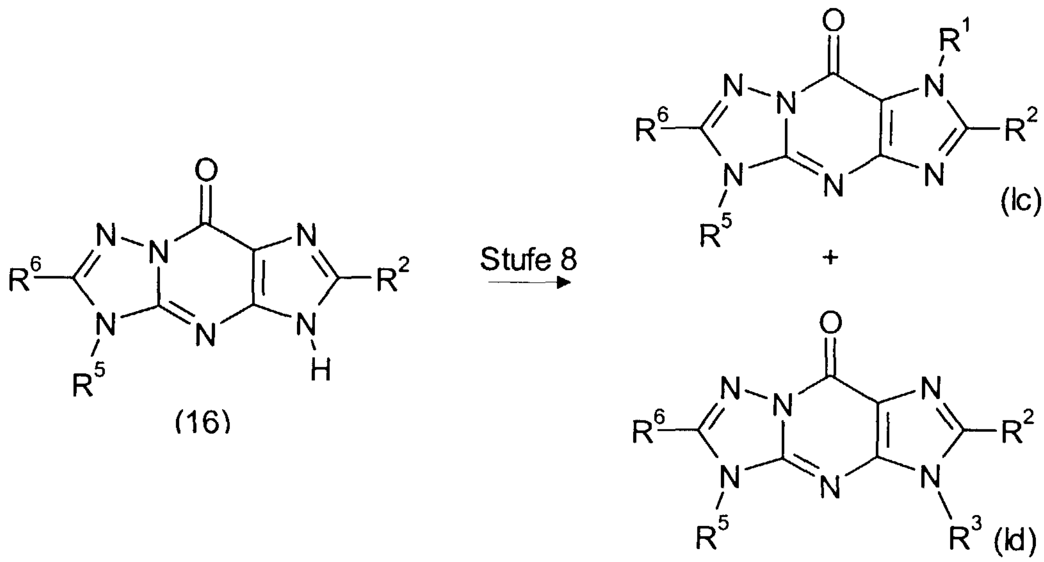


Schema 13:

Über Einführung geeignet substituierter Reste $R^1$, $R^2$, $R^3$, $R^4$, $R^5$ oder $R^6$ lassen sich die durch die zuvor beschriebenen Prozesse darstellbaren Triazolopurine (Ia), (Ib), (Ic) und (Id) unter Anwendung allgemein bekannter Verfahren weitergehend funktionalisieren. Hierzu bietet sich gegebenenfalls die Verwendung geeigneter Schutzgruppen an. Das nachfolgende Schema 14 erläutert beispielhaft daraus erwachsende Modifikationsmöglichkeiten der erfindungsgemäßen Verbindungen (Ib) am Rest $R^6$.

Die beispielhaft in Schema 14 skizzierten Reaktionen führen zu reaktiven Triazolopurinen, die als Ausgangsverbindungen für weitergehende Strukturvariationen dienen können (Veresterungen, Alkylierungen, Substitutionen etc.). Erfindungsgemäß sind Funktionalisierungen sämtlicher Reste $R^1$, $R^2$, $R^3$, $R^4$, $R^5$ oder $R^6$ durchführbar. Diese Modifizierungen sind nicht auf Verbindungen der allgemeinen Formel (Ib) beschränkt, sondern ebenfalls auf die Triazolopurine der allgemeinen Formeln (Ia), (Ic) und (Id) übertragbar. Schema 14 dient zur beispielhaften Erläuterung möglicher Strukturvariationen der Triazolopurine (Ia), (Ib), (Ic) und (Id), ohne die Erfindung auf die dort dargestellten Reaktionsfolgen zu beschränken.

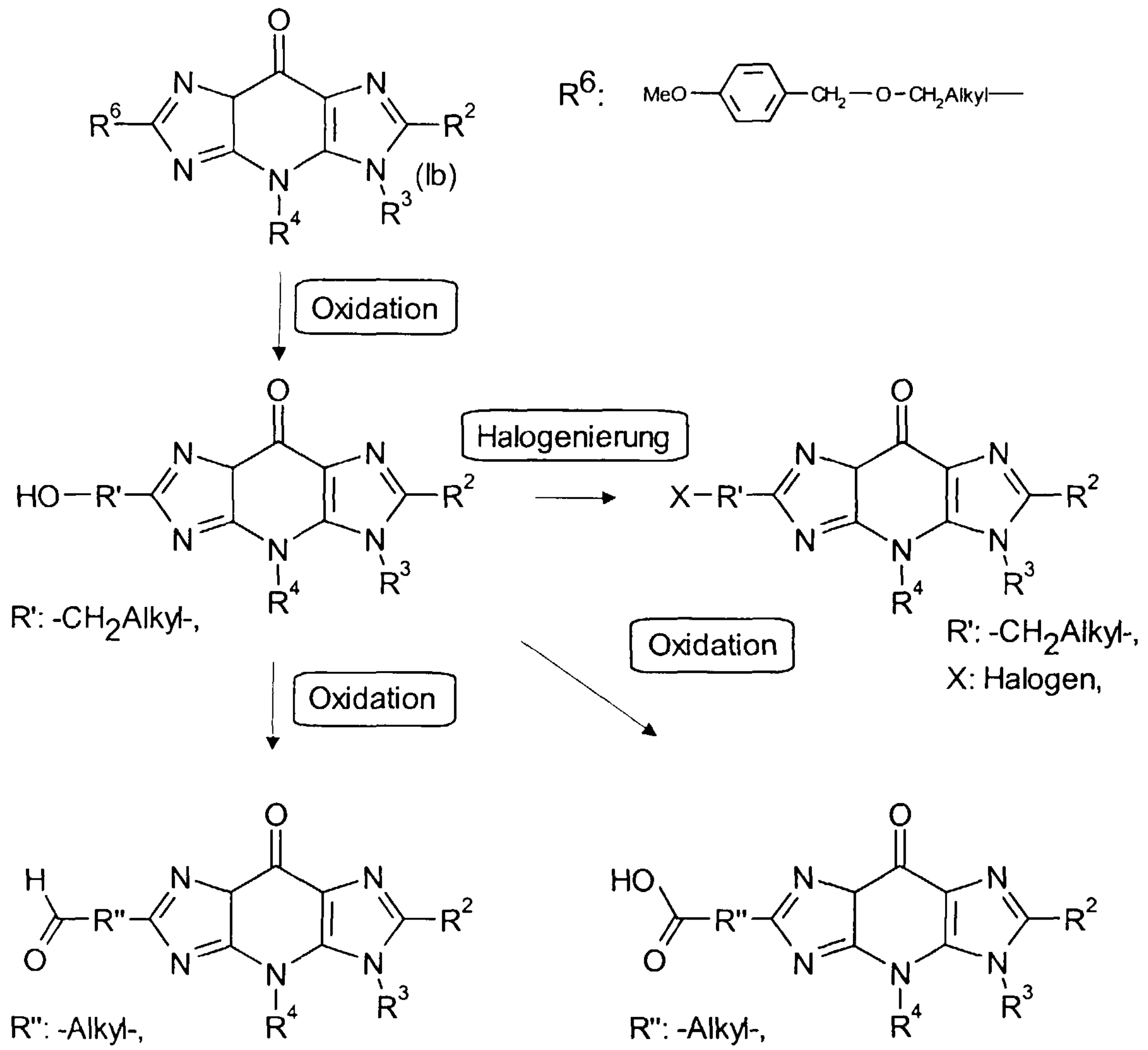


Schema 14:

Die vorliegende Erfindung wird anhand beispielhafter Synthesevorschriften für Triazolopurine näher erläutert. Diese Beispiele dienen der Illustration, ohne die Erfindung auf deren Umfang zu beschränken.

**Stufe 1: Synthese von 5-substituierten 3-Amino-1,2,4-triazolen (3):**

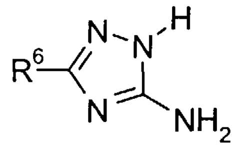

(3)

Das unsubstituierte 3-Amino-1,2,4-triazol ist käuflich, die benötigten 5-substituierten 3-Amino-1,2,4-triazole können entsprechend literaturbekannten Verfahren hergestellt werden, die beispielsweise in J. Chem. Soc. 1929, 816, J. Org. Chem. 1926, 1729 oder Org. Synthesis 26, 11 veröffentlicht sind. Die Carbonsäuren $R^6$-COOH bzw. Nitrile $R^6$-CN sind käuflich erhältlich oder können nach literaturbekannten Verfahren hergestellt werden.

Tabelle 1

| Nr. | $R^6$ | Ausbeute (%) | Fp (°C) |
|---|---|---|---|
| 1.1. | -H | käuflich | - |
| 1.2. | -Methyl | 76 | 145-148 |
| 1.3. | -Ethyl | 75 | 152 |
| 1.4. | -n-Propyl | 62 | 140-143 |
| 1.5. | -i-Propyl | 67 | 103-104 |
| 1.6. | -n-Butyl | 62 | 118 |
| 1.7. | -t-Butyl | 39 | 130-131 |
| 1.8. | Benzyl- | 70 | 167-169 |
| 1.9. | Cyclopentyl- | 64 | 168-172 |
| 1.10. | 2-Furyl-(Semisulfat) | 76 | 207-208 |
| 1.11. | Phenyl- | 68 | 185-186 |
| 1.12. | Cyclohexylmethyl- | 51 | 186-188 |
| 1.13. | 2-Phenylethyl- | 53 | 139-140 |
| 1.14. | (4-MeO-Ph)-O-$CH_2$- | 41 | - |
| 1.15. | (4-MeO-Ph)-$CH_2$-O-$CH_2$- | 54 | 153 |
| 1.16. | (4-Ph-$CH_2$O-Ph)-$CH_2$- | 57 | 196-200 |
| 1.17. | 4-Fluorbenzyl- | 100 | 163-164 |
| 1.18. | 3,4-Difluorbenzyl- | 81 | 135-137 |
| 1.19. | 3-Pyridylmethyl- | 58 | 192-196 |

**Stufe 2: Synthese von 2-substituierten 4*H*-5-Amino-1,2,4-triazolo [1,5 a] pyrimidin-7-on-Derivaten (4)**

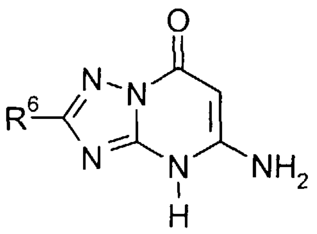

(4)

Allgemeine Arbeitsvorschrift:

2,3 g (0,1 Mol) Natrium werden in 10 ml abs. Ethanol gelöst, dann gibt man 14,7 g (0,13 Mol) Cyanessigsäureethylester zu. Die Mischung wird 1 -2 h bei Raumtemperatur gerührt, anschließend mit 0,1 Mol des gewünschten, 5-substituierten 3-Aminotriazols versetzt und 4 - 6 h unter Rückfluß erhitzt. Das abgekühlte Reaktionsgemisch wird mit Wasser versetzt und unter Rühren sauer gestellt, dann wird der Feststoff abfiltriert, gewaschen und getrocknet.
Nach diesem Verfahren wurden u.a. die folgenden Verbindungen hergestellt:

Tabelle 2

| Nr | $R^6$ | Ausbeute (%) | Fp (°C) |
|---|---|---|---|
| 2.1. | -H | 73 | >370 |
| 2.2. | -Methyl | 76 | >300 |
| 2.3. | -Ethyl | 81 | >300 |
| 2.4. | -n-Propyl | 80 | 290 (Zers.) |
| 2.5. | -i-Propyl | 81 | 294 (Zers.) |
| 2.6. | -n-Butyl | 77 | 256 |
| 2.7. | -t-Butyl | 78 | >300 |
| 2.8. | Benzyl- | 82 | 300 (Zers.) |
| 2.9. | Cyclopentyl- | 83 | 310 (Zers.) |
| 2.10. | 2-Furyl- | 61 | - |
| 2.11. | Phenyl- | 75 | - |
| 2.12. | Cyclohexylmethyl- | 81 | > 300 |
| 2.13. | 2-Phenylethyl- | 83 | 295-296 |
| 2.14. | (4-MeO-Ph)-O-$CH_2$- | 68 | 302 |
| 2.15. | (4-MeO-Ph)-$CH_2$-O-$CH_2$- | 89 | 245 |
| 2.16. | (4-$PhCH_2O$-Ph)-$CH_2$- | 96 | 296-298 |
| 2.17. | 4-Fluorbenzyl- | 79 | 300-302 |
| 2.18. | 3,4-Difluorbenzyl- | 62 | 290-292 |
| 2.19. | 3-Pyridylmethyl- | 82 | >300 |

**Stufe 3: Synthese der alkylierten Triazolopyrimidin-Derivate (5) und (12):**

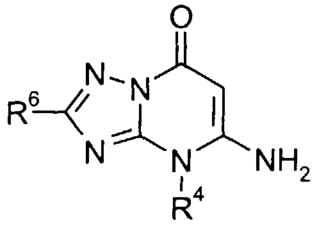

(5)

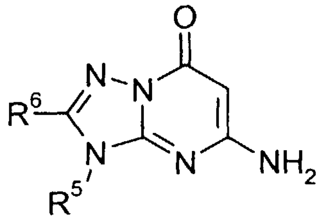

(12)

Allgemeine Arbeitsvorschrift:

66 mMol des betreffenden 5-Amino-1,2,4-triazolo [1,5-a]pyrimidin-7-ons (4) werden in der 10 - 20fachen Menge wasserfreiem Dimethylformamid gelöst und mit 76 mMol Kaliumcarbonat und 76 mMol des gewünschten Alkyljodids versetzt. Das Reaktionsgemisch wird 1 - 2 Tage bei Raumtemperatur gerührt und zur Trockne eingeengt. Der Rückstand wird mit Wasser und Methylenchlorid verrührt. In vielen Fällen läßt sich nach dieser Behandlung (5) als Feststoff abfiltrieren und dadurch isolieren. Aus dem Filtrat kann dann, wie nachfolgend beschrieben, das Isomere (12) gewonnen werden.
Falls beim Verrühren kein Feststoff anfällt bzw. zur Isolierung von (12) aus dem Filtrat werden die Phasen des Filtrats getrennt, die wäßrige Phase wird mit Methylenchlorid ausgeschüttelt, und die vereinigten organischen Phasen werden über Magnesiumsulfat getrocknet und zur Trockne eingeengt. Durch chromatographische Reinigung des Rückstand werden (5) und ggfs. (12) isoliert. Die benötigten Alkylhalogenide R'-X (mit $R'=R^4,R^5$) sind käuflich erhältlich oder können nach literaturbekannten Verfahren hergestellt werden.
Nach diesem Verfahren wurden u.a.die folgenden Substanzen hergestellt:

Tabelle 3

| Nr. | $R^6$ | $R^4$ | $R^5$ | Ausbeute (%) | Fp (°C) |
|---|---|---|---|---|---|
| 3.1 | -H | -Methyl | - | 80 | 310(Zers.) |
| 3.2 | -H | -n-Propyl | - | 58 | 184 - 186 |
| 3.3 | -H | - | -n-Propyl | 18 | 183 |
| 3.4 | -Methyl | -n-Propyl | - | 61 | 228 |
| 3.5 | -Ethyl | -n-Propyl | - | 65 | 194 |
| 3.6 | -n-Propyl | -n-Propyl | - | 69 | 170 |
| 3.7 | -i-Propyl | -n-Propyl | - | 50 | 138 |
| 3.8 | -Butyl | -n-Propyl | - | 62 | 167 |
| 3.9 | -t-Butyl | -n-Propyl | - | 64 | 164 - 165 |
| 3.10 | 2-Furyl- | -n-Propyl | - | 45 | 260 - 262 |
| 3.11 | Cyclopentyl- | -n-Propyl | - | 64 | 148 - 150 |
| 3.12 | Cyclopentyl | - | -n-Propyl | 22 | 173 |
| 3.13 | Benzyl- | -n-Propyl | - | 60 | 208 |
| 3.14 | Benzyl- | -Methyl | - | 52 | 296 |
| 3.15 | Benzyl- | -Ethyl | - | 58 | 213 |
| 3.16 | Benzyl | -i-Propyl | - | 29 | 232 |
| 3.17 | Benzyl- | -n-Butyl | - | 55 | 175 |
| 3.18 | Benzyl- | -Pentyl | - | 63 | 188 |
| 3.19 | Benzyl- | Benzyl- | - | 51 | 180 - 181 |
| 3.20 | Phenyl- | -n-Propyl | - | 30 | 270 - 271 |
| 3.21 | Cyclohexylmethyl- | -Ethyl | - | 67 | 210-212 |
| 3.22 | 2-Phenylethyl- | -Ethyl | - | 29 | 173-174 |
| 3.23 | (4-MeO-Ph)-O-$CH_2$- | -Ethyl | - | 58 | 177-180 |
| 3.24 | (4-MeO-Ph)-$CH_2$-O-$CH_2$- | -Ethyl | - | 100 | -* |
| 3.25 | (4-Ph$CH_2$-O-Ph)-$CH_2$- | -Ethyl | - | 63 | 240-242 |
| 3.26 | 4-Fluorbenzyl- | -Ethyl | - | 53 | - |
| 3.27 | 3,4-Difluorbenzyl- | -Ethyl | - | 61 | 228 |
| 3.28 | 3-Pyridylmethyl- | -Ethyl | - | 94 | 186-188 |

*Isomerentrennung auf Folgestufe

**Stufe 4: Synthese der Nitrosoverbindungen (6) bzw. (13)**

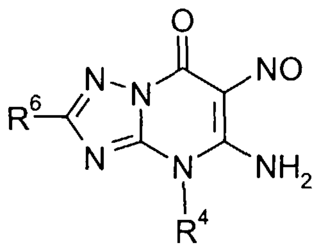

(6)

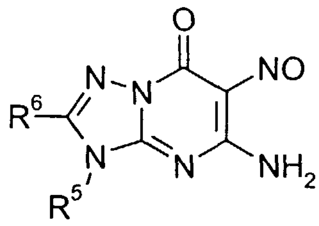

(13)

Allgemeine Arbeitsvorschriften (Variante A):

10 mMol 5-Amino-1,2,4-triazolo[1,5-a]pyrimidin-7-on (4), (5) oder (12) werden in 10-30 ml wasserfreiem Dimethylformamid gelöst oder suspendiert, und die Mischung wird auf -5°C abgekühlt. Man gibt 20 mMol Isoamylnitrit so zu, daß die Temperatur nicht über 0 °C steigt und rührt bei dieser Temperatur 4 - 24 h, anschließend bei Bedarf noch bis zur Vervollständigung der Reaktion bei Raumtemperatur. Das Lösungsmittel wird im Vakuum entfernt, der Rückstand mit Ether verrührt, abfiltriert, mit Wasser gewaschen und im Vakuum getrocknet.

Nach diesem Verfahren wurden u.a. die folgenden Substanzen hergestellt:

Tabelle 4a

| Nr. | $R^6$ | $R^4$ | $R^5$ | Ausbeute (%) | Fp (°C) |
|---|---|---|---|---|---|
| 4.1 | -H | -H | - | 85 | >350 |
| 4.2 | -H | -Methyl | - | 79 | 248 |
| 4.3 | -H | -n-Propyl | - | 81 | 211 (Zers.) |
| 4.4 | -Methyl | -n-Propyl | - | 88 | 226 (Zers.) |
| 4.5 | -Ethyl | -n-Propyl | - | 94 | 190 |
| 4.6 | -n-Propyl | -n-Propyl | - | 96 | 206 (Zers.) |
| 4.7 | -i-Propyl | -n-Propyl | - | 72 | 210 (Zers.) |
| 4.8 | -n-Butyl | -n-Propyl | - | 95 | 196 (Zers.) |
| 4.9 | -t-Butyl | -n-Propyl | - | 93 | 200 (Zers.) |
| 4.10 | 2-Furyl- | -n-Propyl | - | 85 | 261 |
| 4.11 | Cyclopentyl- | -n-Propyl | - | 97 | 224 (Zers.) |
| 4.12 | Cyclopentyl- | -H | - | 89 | 225 (Zers.) |
| 4.13 | Benzyl- | -H | - | 97 | 238 (Zers.) |
| 4.14 | Benzyl- | -n-Propyl | - | 95 | 208 (Zers.) |
| 4.15 | Benzyl- | -Methyl | - | 96 | 233 (Zers.) |
| 4.16 | Benzyl- | -Ethyl | - | 97 | 226 (Zers.) |
| 4.17 | Benzyl | -i-Propyl | - | 88 | 190 (Zers.) |
| 4.18 | Benzyl- | -n-Butyl | - | 93 | 196 |
| 4.19 | Benzyl- | -n-Pentyl | - | 74 | 190 (Zers.) |

Tabelle 4a (fortgesetzt)

| Nr. | $R^6$ | $R^4$ | $R^5$ | Ausbeute (%) | Fp (°C) |
|---|---|---|---|---|---|
| 4.20 | Benzyl- | Benzyl- | - | 96 | 236 |
| 4.21 | Phenyl- | -n-Propyl | - | 92 | 257 - 258 (Zers.) |
| 4.22 | -H | - | -n-Propyl | 63 | 206 (Zers.) |
| 4.23 | Cyclopentyl- | - | -n-Propyl | 74 | 146 |
| 4.24 | Cyclohexylmethyl- | -Ethyl | - | 83 | 208-209 |
| 4.25 | 2-Phenylethyl- | -Ethyl | - | 90 | 200-201 |
| 4.26 | (4-MeO-Ph)-O-$CH_2$- | -Ethyl | - | 93 | 214-216 |
| 4.27 | (4-MeO-Ph)-$CH_2$-O-$CH_2$- | -Ethyl | - | 56 | 156-158 |
| 4.28 | (4-Ph$CH_2$-O-Ph)-$CH_2$- | -Ethyl | - | 97 | 201 Zers. |
| 4.29 | 4-Fluorbenzyl- | -Ethyl | - | 89 | 241-242 |
| 4.30 | 3,4-Difluorbenzyl- | -Ethyl | - | 97 | 234-236 |
| 4.31 | 3-Pyridylmethyl- | -Ethyl | - | 44 | 229 |

Allgemeine Arbeitsvorschrift (Variante B):

20 mMol 5-Amino-1,2,4-triazolo[1,5-a]pyrimidin-7-on (4), (5) oder (12) werden in 80-100 ml Eisessig suspendiert. Die Mischung wird auf 70°C erwärmt, mit einer Lösung von 20 mMol Natriumnitrit in 2-4 ml $H_2O$ versetzt und 0,5-2 h bei 70°C, dann weitere 0,5-2 h bei 10°C gerührt. Das Produkt wird abfiltriert, gewaschen und im Vakuum getrocknet. Nach diesem Verfahren wurden u.a. die folgenden Verbindungen hergestellt!

Tabelle 4b

| Nr. | $R^6$ | $R^4$ | $R^5$ | Ausbeute (%) | Fp (°C) |
|---|---|---|---|---|---|
| 4.32 | Benzyl- | -H | - | 65 | 236 (Zers.) |

**Stufe 5: Synthese der Diamine (7) bzw. (14):**

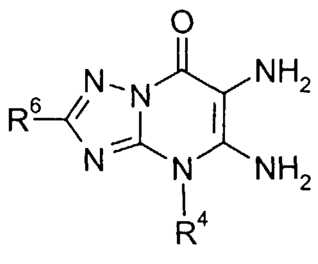

(7)

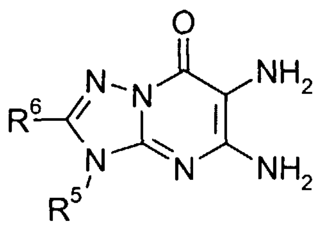

(14)

Allgemeine Arbeitsvorschrift (Variante A):

12,7 mMol der Nitrosoverbindung (6) bzw. (13) werden unter Rühren in 86 ml $H_2O$ suspendiert und mit 4,9 g (2,9 mMol) $Na_2S_2O_4$ versetzt. Die Suspension wird auf 80°C erhitzt, und innerhalb von 30 Min. werden 15 ml 50%ige $H_2SO_4$ zugegeben. Die Mischung wird bis zur vollständigen Umsetzung unter Rückfluß gekocht. Nach dem Abkühlen

fällt in den meisten Fällen das Produkt als Semisulfat aus. Sollte kein Feststoff ausfallen, neutralisiert man mit 30%iger Natronlauge und filtriert die ausgefallene freie Base ab.

Allgemeine Arbeitsvorschrift (Variante B):

Zu einer Mischung aus 175 ml 25%igem wäßrigem Ammoniak und 36 ml Ethanol gibt man unter Rühren 11,5 mMol Nitrosoverbindung (6) oder (13). Anschließend rührt man bei 30°C, bis sich die Nitrosoverbindung weitgehend gelöst hat, tropft eine Lösung von 6,1 g (34,7 mMol) $Na_2S_2O_4$ in 57 ml $H_2O$ zu und rührt weitere 1,5 - 3 h bei Raumtemperatur. Sollte anschließend noch Ausgangsverbindung vorhanden sein, gibt man 10% der o.a. Menge an $Na_2S_2O_4$ nach und rührt bis zur Beendigung der Umsetzung. Das Produkt wird durch Abfiltrieren isoliert.

Nach einer dieser Varianten (A oder B, s. Tabelle 5) wurden u.a. die in Tabelle 5 beschriebenen Verbindungen hergestellt:

Tabelle 5

| Nr. | $R^6$ | $R^4$ | $R^5$ | Variante | Ausbeute in % | Salzform | Schmelzpunkt in °C |
|---|---|---|---|---|---|---|---|
| 5.1 | -H | -H | - | A | 73 | Semisulfat | >300 |
| 5.2 | -H | -Methyl | - | A | 65 | - | 267 (Zers.) |
| 5.3 | -H | -n-Propyl | - | A | 65 | - | 185-188 |
| 5.4 | -Methyl | -n-Propyl | - | A | 70 | - | 174 |
| 5.5 | -Ethyl | -n-Propyl | - | A | 78 | - | 170-172 |
| 5.6 | -n-Propyl | -n-Propyl | - | A | 82 | - | 148-150 |
| 5.7 | -i-Propyl | -n-Propyl | - | A | 52 | - | 193 |
| 5.8 | -n-Butyl | -n-Propyl | - | A | 71 | - | 140-142 |
| 5.9 | -t-Butyl | -n-Propyl | - | B | 90 | - | 168 |
| 5.10 | Cyclopentyl- | -H | - | A | 74 | Semisulfat | 224 |
| 5.11 | Cyclopentyl- | -n-Propyl | - | A | 48 | Semisulfat | 195-197 |
| 5.12 | 2-Furyl- | -n-Propyl | - | B | 75 | - | 204-205 |
| 5.13 | Benzyl | -i-Propyl | - | A | 8 | Semisulfat | 223 |
| 5.14 | Benzyl- | -n-Propyl | - | A | 62 | Semisulfat | 217 |
| 5.15 | Benzyl- | -Methyl | - | B | 91 | - | 248 |
| 5.16 | Benzyl- | -Ethyl | | B | 95 | - | 175 |
| 5.17 | Benzyl- | -n-Butyl | - | A | 48 | Semisulfat | 203-205 |
| 5.18 | Benzyl- | -n-Pentyl | - | B | 92 | - | 146 |
| 5.19 | Benzyl- | -H | - | A | 47 | Semisulfat | 218-220 |
| 5.20 | Benzyl- | Benzyl- | - | B | 80 | - | 273 |
| 5.21 | Phenyl- | -n-Propyl | - | A | 79 | Semisulfat | 260 |
| 5.22 | -H | - | -n-Propyl | A | 81 | - | 222-224 |
| 5.23 | Cyclopentyl- | - | -n-Propyl | A | 86 | - | 175 |
| 5.24 | Cyclohexylmethyl- | -Ethyl | - | B | 94 | - | 158-160 |
| 5.25 | 2-Phenylethyl- | -Ethyl | - | B | 94 | - | 204-205 |
| 5.26 | (4-MeO-Ph)-O-$CH_2$- | -Ethyl | - | B | 86 | - | 154-156 |

Tabelle 5 (fortgesetzt)

| Nr. | $R^6$ | $R^4$ | $R^5$ | Variante | Ausbeute in % | Salzform | Schmelzpunkt in °C |
|---|---|---|---|---|---|---|---|
| 5.27 | (4-MeO-Ph)-$CH_2$-O-$CH_2$- | -Ethyl | - | B | 88 | - | 133-139 |
| 5.28 | (4-Ph$CH_2$-O-Ph)-$CH_2$- | -Ethyl | - | B | 84 | - | 155 |
| 5.29 | 4-Fluorbenzyl- | -Ethyl | - | B | 88 | - | 202-205 |
| 5.30 | 3,4-Difluorbenzyl- | -Ethyl | - | B | 95 | - | 209-212 |
| 5.30 | 3-Pyridylmethyl- | -Ethyl | - | B | 82 | - | 179-180 |

**Stufe 6: Synthese der Amide (8) bzw. (15):**

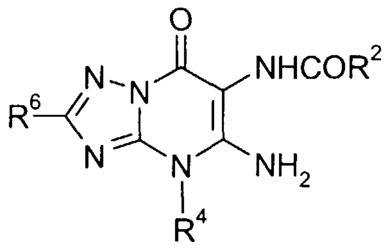

+

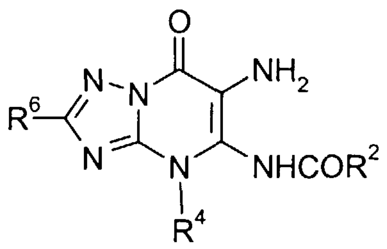

(8)

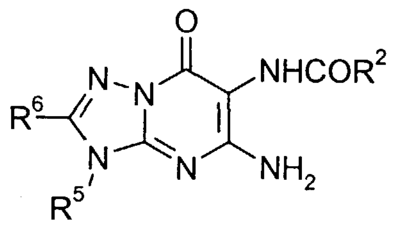

+

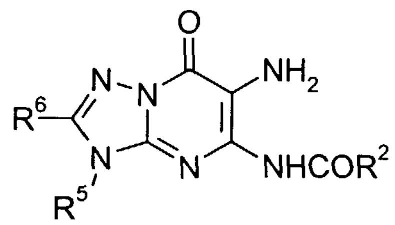

(15)

Allgemeine Arbeitsvorschrift (Variante A):

30 mMol Diaminoverbindung (7) bzw. (14) werden in 165 ml wasserfreiem Dimethylformamid suspendiert und mit 45 mMol (im Falle von Semisulfaten mit 78 mMol) 4-Dimethylaminopyridin versetzt. Man rührt 30 Min. bei Raumtemperatur, kühlt die Mischung auf 5 - 10°C und gibt eine Lösung aus 39 mMol des gewünschten Carbonsäurechlorids in 16 ml wasserfreiem Dimethylformamid zu. Anschließend wird 2 h bei 10°C und über Nacht bei Raumtemperatur gerührt, dann wird das Lösungsmittel im Vakuum entfernt. Der Rückstand wird auf Wasser gegeben, der Feststoff abfiltriert, gewaschen und ggfs. durch Umkristallisation oder Chromatographie gereinigt.

Die benötigten Carbonsäurechloride sind käuflich oder können nach literaturbekannten Verfahren hergestellt werden.

Nach diesem Verfahren wurden u.a. die in Tabelle 6a beschriebenen Verbindungen der Formel (8) und (15) hergestellt:

Tabelle 6a:

| Nr. | $R^6$ | $R^4$ | $R^5$ | $R^2$ | Ausbeute in % | Schmelzpunkt in °C |
|---|---|---|---|---|---|---|
| 6.1 | -H | -H | - | Cyclopentyl- | 86 | 284 (Zers.) |
| 6.2 | -H | -Methyl | - | Benzyl- | 35 | 306 |
| 6.3 | -H | -n-Propyl | - | Benzyl- | 65 | 178 |
| 6.4 | -H | -n-Propyl | - | Cyclopentyl- | 95 | 192 |
| 6.5 | -Methyl | -n-Propyl | - | Cyclopentyl- | 71 | 208 |
| 6.6 | -Ethyl | -n-Propyl | - | Cyclopentyl- | 72 | 209 |
| 6.7 | -Ethyl | -n-Propyl | - | -Ethyl | 77 | - |
| 6.8 | -n-Propyl | -n-Propyl | - | Cyclopentyl- | 90 | 210 |
| 6.9 | -n-Propyl | -n-Propyl | - | Phenyl- | 68 | 205 |
| 6.10 | -n-Propyl | -n-Propyl | - | 4-Fluorbenzyl- | 84 | 200 |
| 6.11 | -i-Propyl | -n-Propyl | - | Cyclopentyl- | 71 | 148 |
| 6.12 | -n-Butyl | -n-Propyl | - | Cyclopentyl- | 66 | 173 |
| 6.13 | -t-Butyl | -n-Propyl | - | Cyclopentyl- | 60 | 100 |
| 6.14 | Cyclopentyl- | -n-Propyl | - | -Ethyl | 76 | 205 |
| 6.15 | Cyclopentyl- | -n-Propyl | - | -Methyl | 70 | 243 |
| 6.16 | Cyclopentyl- | -n-Propyl | - | Benzyl- | 76 | 160 |
| 6.17 | 2-Furyl- | -n-Propyl | - | Cyclopentyl- | 74 | - |
| 6.18 | 2-Furyl- | -n-Propyl | - | -Ethyl | 73 | - |
| 6.19 | Benzyl- | -H | - | Cyclopentyl- | 51 | 254-257 |
| 6.20 | Benzyl- | Benzyl- | - | Cyclopentyl- | 90 | - |
| 6.21 | Benzyl- | -Ethyl | - | 3-Tetrahydrofuranyl- | 60 | 227 |
| 6.22 | Benzyl- | -Ethyl | - | 4-Tetrahydropyranyl- | 65 | 237 |

| Nr. | $R^6$ | $R^4$ | $R^5$ | $R^2$ | Ausbeute in % | Schmelzpunkt in °C |
|---|---|---|---|---|---|---|
| 6.23 | Benzyl- | -Ethyl | - | 2-Pyridyl- | 63 | 223 |
| 6.24 | Benzyl- | -Ethyl | - | 4-Pyridyl- | 55 | |
| 6.25 | Benzyl- | -n-Propyl | - | -Ethyl | 61 | 183 |
| 6.26 | Benzyl- | -n-Propyl | - | -n-Propyl | 62 | 166 |
| 6.27 | Benzyl- | -Methyl | - | Cyclopentyl- | 88 | 205-208 |
| 6.28 | Benzyl- | -Ethyl | - | Cyclopentyl- | 90 | 188-189 |
| 6.29 | Benzyl- | -n-Propyl | - | Cyclopentyl- | 77 | 170 |
| 6.30 | Benzyl- | -n-Butyl | - | Cyclopentyl- | 60 | 163 |
| 6.31 | Benzyl- | -n-Pentyl | - | Cyclopentyl- | 67 | 162 |
| 6.32 | Phenyl- | -n-Propyl | - | -Ethyl | 47 | 165-166 |
| 6.33 | Phenyl- | -n-Propyl | - | Cyclopentyl- | 68 | >300 |
| 6.34 | -H | - | -n-Propyl | Cyclopentyl- | 69 | 248 |
| 6.35 | Benzyl- | -Ethyl | - | 2-Pyridyl- | 40 | 223 |
| 6.36 | Benzyl- | -Ethyl | - | 4-Pyridyl- | 55 | - |
| 6.37 | Benzyl- | -Ethyl | - | 3-Pyridyl- | 88 | 206 |
| 6.38 | Benzyl- | -Ethyl | - | Cyclohexyl- | 86 | 186-187 |
| 6.39 | Benzyl- | n-Butyl- | - | -H | 84 | 231-232 |
| 6.40 | Benzyl- | -H | - | Phenyl- | 72 | 276-279 |
| 6.41 | Benzyl- | -H | - | 2-Furyl- | 69 | 305-307 |
| 6.42 | 3-Pyridylmethyl- | -Ethyl | - | Cyclopentyl- | 73 | 163-165 |
| 6.43 | 3-Pyridylmethyl- | -Ethyl | - | -Ethyl | 71 | 186-187 |
| 6.44 | 2-Furyl- | -n-Propyl | - | 2-Furyl- | 81 | 228 |

| Nr. | $R^6$ | $R^4$ | $R^5$ | $R^2$ | Ausbeute in % | Schmelzpunkt in °C |
|---|---|---|---|---|---|---|
| 6.45 | Cyclohexylmethyl- | -Ethyl | - | -Ethyl | 81 | 204-207 |
| 6.46 | Cyclohexylmethyl- | -Ethyl | - | Cyclopentyl- | 70 | 196-198 |
| 6.47 | 2-Phenylethyl- | -Ethyl | - | -Ethyl | 95 | 182-183 |
| 6.48 | 2-Phenylethyl- | -Ethyl | - | Cyclopentyl- | 81 | 155-156 |
| 6.49 | Cyclopentyl- | -n-Propyl | - | 3-Tetrahydrofuranyl- | 65 | 179-180 |
| 6.50 | Cyclopentyl- | -n-Propyl | - | Cyclopentyl- | 41 | 306-308 |
| 6.51 | (4-MeO-Ph)- $OCH_2$- | -Ethyl | - | -Ethyl | 93 | 160-164 |
| 6.52 | (4-MeO-Ph)- $OCH_2$- | -Ethyl | - | Cyclopentyl- | 76 | 176-178 |
| 6.53 | (4-MeO-Ph)-$CH_2$-O-$CH_2$- | -Ethyl | - | Cyclopentyl- | 91 | 133-136 |
| 6.54 | (4-$PhCH_2O$-Ph)-$CH_2$- | -Ethyl | - | Cyclopentyl- | 86 | 190 |
| 6.55 | Cyclohexylmethyl- | -Ethyl | - | 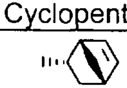 | 98 | - |
| 6.56 | 4-Fluorbenzyl- | -Ethyl | - | Cyclopentyl- | 62 | 206-208 |
| 6.57 | 4-Fluorbenzyl- | -Ethyl | - | tert.-Butyl- | 53 | - |
| 6.58 | 4-Fluorbenzyl- | -Ethyl | - | 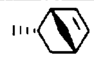 | 75 | - |
| 6.59 | 4-Fluorbenzyl- | -Ethyl | - | 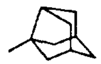 | 91 | - |
| 6.60 | 3,4-Difluorbenzyl- | -Ethyl | - | Cyclopentyl- | 100 | 222-224 |
| 6.61 | 3,4-Difluorbenzyl- | -Ethyl | - | 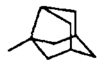 | 100 | - |
| 6.62 | Benzyl- | -Ethyl | - | 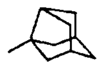 | 87 | - |
| 6.63 | 3-Pyridylmethyl- | -Ethyl | - | 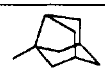 | 79 | 134-137 |

Allgemeine Arbeitsvorschrift (Variante B):

6,6 mMol eines Diamins (7) bzw. (14) werden mit 7,3 mMol der entsprechenden Carbonsäure in 15-25 ml Acetonitril suspendiert. 7,3 mMol N-Methylmorpholin werden unter Rühren langsam zugesetzt. Anschließend wird eine Lösung von 7,3 mMol Chlorameisensäureisobutylester in 6-7 ml Acetonitril bei 20-25°C binnen 10-20 Minuten zugetropft. Die erhaltene Suspension wird 4-6 h bei Raumtemperatur gerührt. Zur Aufarbeitung wird filtriert und mit Acetonitril gewaschen. Das erhaltene Filtrat wird konzentriert und an Kieselgel chromatographiert.

Nach diesem Verfahren wurden u.a. die in Tabelle 6b beschriebenen Verbindungen der Formel (8) und (15) hergestellt:

Tabelle 6b:

| Nr. | $R^6$ | $R^4$ | $R^5$ | $R^2$ | Ausbeute in % |
|---|---|---|---|---|---|
| 6.64 | 4-Fluorbenzyl- | Ethyl- | - | 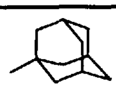 | 26 |

**Stufe 7: Ringschlußreaktionen zu den Triazolopurinen (9) bzw. (16)**

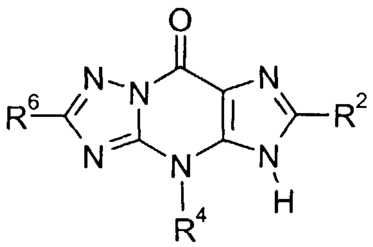

(9)

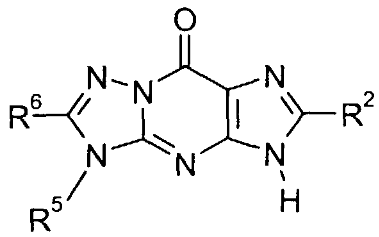

(16)

Allgemeine Arbeitsvorschrift (Methode A, Variante A):

14,4 mMol (8) bzw. (15) werden in 75,5 ml $H_2O$ und 37,8 ml Ethanol suspendiert und mit 17,4 ml 50%iger NaOH und 4,82 g (65 mMol) $Ca(OH)_2$ 24 h unter Rückfluß gekocht. Man läßt den Ansatz abkühlen, stellt ihn sauer, filtriert den Feststoff ab und trocknet ihn. Das Produkt wird gegebenenfalls durch Umkristallisation oder Chromatographie gereinigt.

Nach diesem Verfahren wurden u.a. die in Tabelle 7a beschriebenen Verbindungen der allgemeinen Formeln (9) und (16) hergestellt.

Tabelle 7a:

| Nr. | $R^6$ | $R^4$ | $R^5$ | $R^2$ | Ausbeute in % | Schmelzpunkt in °C |
|---|---|---|---|---|---|---|
| 7.1 | -H | -H | - | Cyclopentyl- | 80 | >360 |
| 7.2 | -H | -Methyl | - | Benzyl- | 35 | 306 |
| 7.3 | -H | -n-Propyl | - | Benzyl- | 68 | 222-223 |
| 7.4 | -H | -n-Propyl | - | Cyclopentyl- | 59 | 250 |
| 7.5 | -Methyl | -n-Propyl | - | Cyclopentyl- | 62 | 262-264 |
| 7.6 | -Ethyl | -n-Propyl | - | Cyclopentyl- | 58 | 253 |
| 7.7 | -Ethyl | -n-Propyl | - | -Ethyl | 68 | 229 |
| 7.8 | -n-Propyl | -n-Propyl | - | Cyclopentyl- | 75 | 256 |
| 7.9 | -n-Propyl | -n-Propyl | - | Phenyl- | 49 | 300 (Zers.) |
| 7.10 | -n-Propyl | -n-Propyl | - | 4-Fluorbenzyl- | 65 | 221 |
| 7.11 | -i-Propyl | -n-Propyl | - | Cyclopentyl- | 62 | 260-261 |
| 7.12 | -n-Butyl | -n-Propyl | - | Cyclopentyl- | 72 | 246 |
| 7.13 | -t-Butyl | -n-Propyl | - | Cyclopentyl- | 64 | 310 |
| 7.14 | Cyclopentyl- | -n-Propyl | - | -Ethyl | 85 | 245-248 |
| 7.15 | Cyclopentyl- | -n-Propyl | - | -Methyl | 56 | 248-250 |
| 7.16 | Cyclopentyl- | -n-Propyl | - | Benzyl- | 70 | 233 |
| 7.17 | 2-Furyl- | -n-Propyl | - | Cyclopentyl- | 61 | >300 (Zers.) |
| 7.18 | 2-Furyl- | -n-Propyl | - | -Ethyl | 44 | >300 (Zers.) |
| 7.19 | Benzyl- | -H | - | Cyclopentyl- | 40 | 270 (Zers.) |
| 7.20 | Benzyl- | Benzyl- | - | Cyclopentyl- | 13 | 270 |
| 7.21 | Benzyl- | -Ethyl | - | 3-Tetrahydrofuranyl- | 60 | 227 |

| Nr. | $R^6$ | $R^4$ | $R^5$ | $R^2$ | Ausbeute in % | Schmelzpunkt in °C |
|---|---|---|---|---|---|---|
| 7.22 | Benzyl- | -Ethyl | - | 4-Tetrahydropyranyl- | 64 | 237 |
| 7.23 | Benzyl- | -Ethyl | - | 2-Pyridyl- | 21 | 287 |
| 7.24 | Benzyl- | -Ethyl | - | 4-Pyridyl- | 8 | 346 |
| 7.25 | Benzyl- | -n-Propyl | - | -Ethyl | 57 | 223 |
| 7.26 | Benzyl- | -n-Propyl | - | -n-Propyl | 53 | 202 |
| 7.27 | Benzyl- | -Methyl | - | Cyclopentyl- | 13 | 265-267 |
| 7.28 | Benzyl- | -Ethyl | - | Cyclopentyl- | 56 | 242 |
| 7.29 | Benzyl- | -n-Propyl | - | Cyclopentyl- | 67 | 234-235 |
| 7.30 | Benzyl- | -n-Butyl | - | Cyclopentyl- | 53 | 238-240 |
| 7.31 | Benzyl- | -n-Pentyl | - | Cyclopentyl- | 56 | 231-236 |
| 7.32 | Phenyl- | -n-Propyl | - | -Ethyl | 97 | >300 (Zers.) |
| 7.33 | Phenyl- | -n-Propyl | - | Cyclopentyl- | 78 | >300 (Zers.) |
| 7.34 | -H | - | -n-Propyl | Cyclopentyl- | 36 | 217-220 |
| 7.35 | -H | - | -Ethyl | Cyclopentyl- | 2,4 | 260 |
| 7.36 | Benzyl- | -Ethyl | - | 3-Pyridyl- | 11 | 331-332 |
| 7.37 | Benzyl- | -Ethyl | - | Cyclohexyl- | 74 | 260-262 |
| 7.38 | 3-Pyridylmethyl- | -Ethyl | - | Cyclopentyl- | 60 | 261-263 |
| 7.39 | 3-Pyridylmethyl- | -Ethyl | - | -Ethyl_ | 62 | 225-227 |
| 7.40 | 2-Furyl- | -n-Propyl | - | 2-Furyl- | 9 | 385-387 |
| 7.41 | Benzyl- | -H | - | 2-Furyl- | 14 | 352-354 |
| 7.42 | Cyclohexylmethyl- | -Ethyl | - | -Ethyl | 73 | 230-232 |
| 7.43 | Cyclohexylmethyl- | -Ethyl | - | Cyclopentyl- | 62 | 273-274 |

| Nr. | $R^6$ | $R^4$ | $R^5$ | $R^2$ | Ausbeute in % | Schmelzpunkt in °C |
|---|---|---|---|---|---|---|
| 7.44 | 2-Phenylethyl- | -Ethyl | - | -Ethyl | 31 | 282-283 |
| 7.45 | 2-Phenylethyl- | -Ethyl | - | Cyclopentyl- | 53 | 292-294 |
| 7.46 | Cyclopentyl- | n-Propyl | - | 3-Tetrahydrofuranyl- | 56 | 289-290 |
| 7.47 | Cyclopentyl- | n-Propyl | - | Cyclopentyl- | 41 | 306-308 |
| 7.48 | (4-MeO-Ph)-$OCH_2$- | -Ethyl | - | -Ethyl | 47 | 225 |
| 7.49 | (4-MeO-Ph)-$OCH_2$- | -Ethyl | - | Cyclopentyl- | 20 | 234-236 |
| 7.50 | (4-MeO-Ph)-$CH_2$-O-$CH_2$- | -Ethyl | - | Cyclopentyl- | 70 | 182 |
| 7.51 | (4-$PhCH_2O$-Ph)-$CH_2$- | -Ethyl | - | Cyclopentyl- | 70 | 251 |
| 7.52 | 4-Fluorbenzyl- | -Ethyl | - | Cyclopentyl- | 71 | 252-254 |
| 7.53 | 4-Fluorbenzyl- | -Ethyl | - | -tert.-Butyl | 73 | 262-264 |
| 7.54 | 4-Fluorbenzyl- | -Ethyl | - | 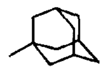 | 53 | 337-339 |
| 7.55 | 4-Fluorbenzyl- | -Ethyl | - | 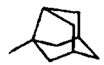 | 45 | 325-327 |
| 7.56 | 3,4-Difluorbenzyl- | -Ethyl | - | Cyclopentyl- | 60 | 264-266 |
| 7.57 | 3,4-Difluorbenzyl- | -Ethyl | - | 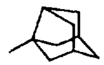 | 58 | 315-317 |
| 7.58 | Benzyl- | -Ethyl | - | 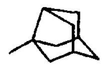 | 67 | 320-322 |
| 7.59 | 3-Pyridylmethyl- | -Ethyl | - | 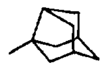 | 57 | 330-332 |

Allgemeine Arbeitsvorschrift (Methode A, Variante B):

4 mMol eines Amids (8) bzw. (15) werden in 10-15 ml Tetrahydrofuran und 25-35 ml $H_2O$ mit 46 mMol Lithiumhydroxyd-Monohydrat 14 Tage bei 45-65° gerührt. Danach wird mit 6 n Salzsäure angesäuert und abgesaugt. Das Rohprodukt wird aus Methanol umkristallisiert. Das erhaltene Produkt wird an einer Kieselgel-Säule chromatographisch getrennt.

Nach diesem Verfahren wurden u.a. die in Tabelle 7b beschriebenen Verbindungen der allgemeinen Formel (9) hergestellt.

Tabelle 7b:

| Nr. | $R^6$ | $R^4$ | $R^2$ | Ausbeute (%) | Fp (°C) |
|---|---|---|---|---|---|
| 7.62 | Cyclohexylmethyl- | Ethyl- | 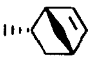 | 10 | 246-248 |
| 7.63 | Cyclohexylmethyl- | Ethyl- | 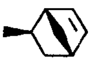 | 3 | 258-260 (Zers.) |

Allgemeine Arbeitsvorschrift (Methode B):

10,1 mMol (7) bzw. (14) werden in 34 ml Formamid 1,5 h bei 200°C gerührt. Das verbleibende Formamid wird im Vakuum abdestilliert, der Rückstand mit Wasser verrührt. Den Feststoff filtriert man ab, wäscht ihn mit Wasser und reinigt das Produkt durch Umkristallisation oder Chromatographie.

Nach diesem Verfahren wurden u.a. die in Tabelle 7c aufgeführten Verbindungen (9) und (16) hergestellt:

Tabelle 7c

| Nr. | $R^6$ | $R^4$ | $R^5$ | $R^2$ | Ausbeute (%) | Fp (°C) |
|---|---|---|---|---|---|---|
| 7.64 | -Ethyl | -n-Propyl | - | -H | 84 | >300 |
| 7.65 | Cyclopentyl- | -H | - | -H | 53 | 215 |
| 7.66 | Cyclopentyl- | -Methyl | - | -H | 7 | >300 |
| 7.67 | 3-Pyridylmethyl- | -Ethyl | - | -H | 58 | 328-330 |
| 7.68 | Cyclopentyl- | -n-Propyl | - | -H | 58 | 287 (Zers.) |
| 7.69 | 2-Furyl- | -n-Propyl | - | -H | 74 | >300 (Zers.) |
| 7.70 | Benzyl- | -Methyl | - | -H | 9 | >300 |
| 7.71 | Benzyl- | -n-Propyl | - | -H | 75 | >300 |
| 7.72 | Benzyl- | -i-Propyl | - | -H | 90 | >300 |
| 7.73 | Benzyl- | -n-Butyl | - | -H | 75 | >300 |
| 7.74 | Benzyl- | -H | - | -H | 63 | >300 |
| 7.75 | Phenyl- | -n-Propyl | - | -H | 75 | 295-296 (Zers.) |
| 7.76 | Cyclopentyl- | - | -n-Propyl | -H | 35 | 202-203 |
| 7.77 | 3-Pyridylmethyl- | -Ethyl | - | -H | 58 | 328-330 |
| 7.78 | Cyclohexylmethyl- | -Ethyl | - | -H | 87 | 328-330 |
| 7.79 | 2-Phenylethyl- | -Ethyl | - | -H | 84 | 273 |

Allgemeine Arbeitsvorschrift (Methode C):

Zu einer Lösung von 6,8 mMol des gewünschten N-Formylamins in 6 ml Diglyme gibt man bei 0°C 0,66 ml (7,2 mMol) Phosphoroxychlorid. Man rührt 30 Minuten im Eisbad und setzt dann 4,8 mMol Nitrosoverbindung (6) bzw. (13) zu. Die Mischung wird 30 - 60 Min. bei 80°C gerührt und auf Eiswasser gegeben, das feste Produkt wird abfiltriert, gewaschen und getrocknet. Zur Reinigung kristallisiert man um oder reinigt durch Chromatographie.

Nach diesem Verfahren wurden u.a. die in Tabelle 7d beschriebenen Verbindungen (9) hergestellt:

Tabelle 7d

| Nr. | $R^6$ | $R^4$ | $R^2$ | Ausbeute (%) | Fp (°C) |
|---|---|---|---|---|---|
| 7.80 | Benzyl- | -n-Propyl | N-Morpholinyl- | 21 | 298 |
| 7.81 | Benzyl- | -n-Propyl | 4-Benzyl-1-piperazinyl- | 21 | 266-268 |
| 7.82 | Benzyl- | -n-Propyl | Dimethylamino- | 9 | 272-274 |
| 7.83 | Benzyl- | -n-Propyl | N-Piperidinyl- | 22 | 294-297 |

Die benötigten Formylamine werden nach literaturbekannten Verfahren durch Umsetzung der Amine mit 88%iger Ameisensäure und Acetanhydrid hergestellt.

**Stufe 8: Alkylierungsreaktionen**

Allgemeine Arbeitsvorschrift (Variante A):

4 mMol in 4- und 5-Stellung unsubstituiertes 1,2,4-Triazolo[1,5-a]purin-9-on [(9) mit $R^4$ = H; (16) mit $R^5$ = H] werden in 26 ml wasserfreiem Dimethylformamid aufgenommen und zuerst mit einer Lösung von 0,1 g (4,2 mMol) Natrium in 4 ml wasserfreiem Ethanol, dann mit 4,2 mMol des gewünschten Alkyljodids versetzt. Man rührt die Mischung über Nacht bei Raumtemperatur und engt sie dann zur Trockne ein. Der Rückstand wird in 2n NaOH gelöst, die Lösung mit Toluol gewaschen und dann auf pH 6 eingestellt. Der ausgefallene Feststoff wird abfiltriert und durch Chromatographie gereinigt. Dabei können bei Bedarf auch die Di-Alkylierungsprodukte (Ia) und (Ib) isoliert werden.
Nach diesem Verfahren wurden u.a. die folgenden Verbindungen (9) hergestellt:

Tabelle 8a

| Nr. | $R^6$ | $R^4$ | $R^5$ | $R^2$ | Ausbeute (%) | Fp (°C) |
|---|---|---|---|---|---|---|
| 8.1 | Benzyl- | -Methyl | - | -H | 9 | >300 |
| 8.2 | Cyclopentyl- | -Methyl | - | -H | 7 | >300 |
| 8.3 | -H | -Methyl | - | Cyclopentyl- | 44 | 285 |
| 8.4 | -H | -Ethyl | - | Cyclopentyl- | 57 | 249-253 |
| 8.5 | -H | -n-Propyl | - | Cyclopentyl- | 52 | 255 |
| 8.6 | -H | -n-Butyl | - | Cyclopentyl- | 45 | 243 |
| 8.7 | -H | -t-Butyl | - | Cyclopentyl- | 10 | 190-191 |
| 8.8 | Benzyl- | -Ethyl | - | -t-Butyl | 24 | 243-244 |
| 8.9 | Benzyl- | -Ethyl | - | Phenyl- | 24 | 343-345 |
| 8.10 | -H | -n-Butyl | - | Cyclopentyl- | 45 | 243 |
| 8.11 | -H | - | -t-Butyl | Cyclopentyl- | 4 | 149-150 |

Außerdem konnten nach diesem Verfahren die folgenden Verbindungen (Ia) und (Ib) erhalten werden:

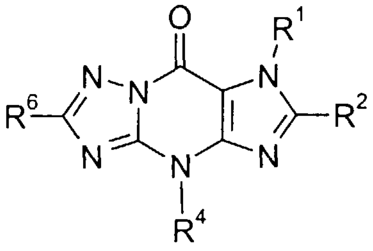

(Ia)

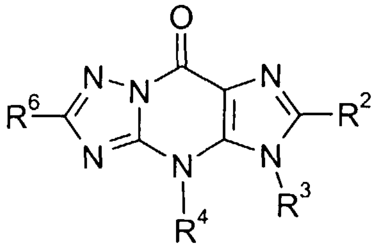

(Ib)

Tabelle 8b

| Nr. | $R^6$ | $R^4$ | $R^1$ oder $R^3$ | $R^2$ | Ausbeute (%) | Fp (°C) |
|---|---|---|---|---|---|---|
| 8.12 | Benzyl- | -Ethyl | -Ethyl | -H | 4 | >300 |
| 8.13 | -H | -Methyl | -Methyl | Cyclopentyl- | 4 | 206 |
| 8.14 | Cyclopentyl- | -n-Propyl | -n-Propyl | -H | 10 | 146 |
| 8.15 | Cyclopentyl- | -n-Propyl | -n-Propyl | -H | 5 | 151 |
| 8.16 | Benzyl- | -Ethyl | -Ethyl | 2-Furyl- | 16 | 187-189 |

Allgemeine Arbeitsvorschrift (Variante B):

20,4 mMol in 4- und 5-Stellung unsubstituiertes 1,2,4-Triazolo[1,5-a]purin-9-on [(9) mit $R^4$ = H; (16) mit $R^5$ = H] werden zusammen mit 40,8 mMol Kaliumcarbonat und 40,8 mMol tert.-Butylbromid in 100 ml wasserfreiem Dimethylformamid 7 Tage bei 70°C gerührt. Man engt zur Trockne ein, versetzt den Rückstand mit 100 ml 2n NaOH, wäscht die wäßrige Phase gründlich mit Toluol und neutralisiert sie. Der Niederschlag wird abfiltriert, mit $CH_2Cl_2/CH_3OH$ 9:1 extrahiert und der Extrakt eingedampft. Durch Chromatographie an Kieselgel mit $CH_2Cl_2/CH_3OH$ 95:5 isoliert man die isomeren Alkylierungsprodukte (9) und (16) (Tabelle 8c). Nach diesem Verfahren wurden u.a. die folgenden Verbindungen hergestellt.

Tabelle 8c

| Nr. | $R^6$ | $R^4$ | $R^5$ | $R^2$ | Ausbeute (%) | Fp (°C) |
|---|---|---|---|---|---|---|
| 8.17 | -H | -t-Butyl | - | Cyclopentyl- | 10 | 190-191 |
| 8.18 | -H | - | -t-Butyl | Cyclopentyl- | 5 | 149-150 |

Allgemeine Arbeitsvorschrift (Variante C):

0,05 Mol Ausgangsverbindung (9) werden in 500 ml abs. Dimethylformamid gelöst und mit 0,055 Mol Kaliumcarbonat und 0,055 Mol Benzylbromid über Nacht bei Raumtemperatur gerührt. Zur Aufarbeitung wird das Lösungsmittel im Vakuum abdestilliert und der Rückstand in Methylenchlorid/Wasser aufgenommen. Die organische Phase wird abgetrennt , 1x mit Wasser gewaschen, über Magnesiumsulfat getrocknet und eingedampft. Der Rückstand wird mit Ether kristallisiert. Die u. a. so erhaltenen Verbindungen (Ia/Ib) sind in Tabelle 8d aufgeführt.

Tabelle 8d

| Nr. | $R^6$ | $R^4$ | $R^1$ oder $R^3$ | $R^2$ | Ausbeute (%) | Fp (°C) |
|---|---|---|---|---|---|---|
| 8.19 | (4-MeO-Ph)-$CH_2$-O-$CH_2$- | Ethyl- | Benzyl- | Cyclopentyl- | 85 | 127-128 |

Tabelle 8d (fortgesetzt)

| Nr. | $R^6$ | $R^4$ | $R^1$ oder $R^3$ | $R^2$ | Ausbeute (%) | Fp (°C) |
|---|---|---|---|---|---|---|
| 8.20 | (4-$PhCH_2O$-Ph)-$CH_2$- | Ethyl- | Benzyl- | Cyclopentyl- | 66 | 138-140 |

**IX. Funktionalisierung der Triazolopurine der allgemeinen Formel (I):**

IX.a. Deblockierung von Triazolopurinen (Ia)/(Ib) mit geschützten Hydroxylfunktionen:

allgemeines Syntheseschema am Beispiel von Verbindungen der allgemeinen Formel (Ib):

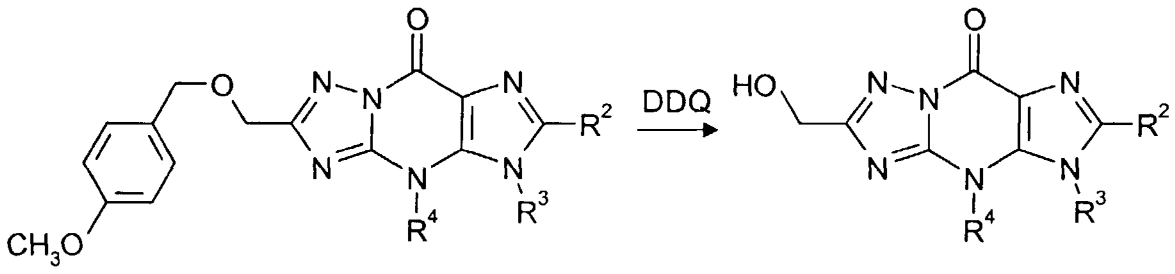


DDQ: 2,3-Dichlor-5,6-dicyano-benzochinon

Obige Reaktion wurde in Anlehnung an ein literaturbekanntes Verfahren (Tetrahedron 1986, *42,* 3021) durchgeführt. Danach wurden u.a. folgende Verbindungen erhalten:

Tabelle 9

| Nr. | $R^6$ | $R^4$ | $R^1$ oder $R^3$ | $R^2$ | Ausbeute (%) | Fp (°C) |
|---|---|---|---|---|---|---|
| 9.1 | HO-$CH_2$- | Ethyl- | Benzyl- | Cyclopentyl- | 97 | 204-207 |

IX.b. Deblockierung von Triazolopurinen (Ia)/(Ib) mit geschützten Aminfunktionen in der Seitenkette:

allgemeines Syntheseschema am Beispiel von Verbindungen der allgemeinen Formel (Ib):

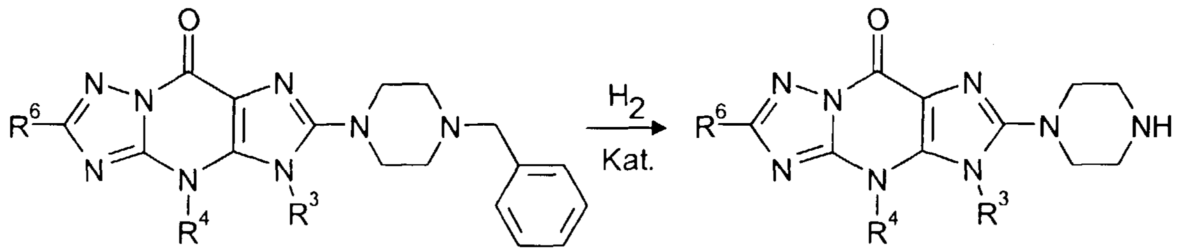


0,83 mMol eines 2-(4-N-benzyl-1-piperazinyl)-1,2,4-triazolo[1,5-a]purin-9-ons werden in 30 ml Eisessig aufgenommen und bei 60°C und 5 bar 6 h in Anwesenheit von 0,1 g 10%igem Pd auf Kohle hydriert. Der Katalysator wird abfiltriert, der Rückstand zur Trockne eingeengt und mit $CH_3OH$ verrührt, das feste Produkt abfiltriert, gewaschen und getrocknet. Nach dieser Arbeitsvorschrift wurden u.a. erhalten:

Tabelle 10

| Nr. | $R^6$ | $R^4$ | $R^1$ oder $R^3$ | $R^2$ | Ausbeute (%) | Salzform | Fp (°C) |
|---|---|---|---|---|---|---|---|
| 10.1 | Benzyl- | n-Propyl- | H- | 1-Piperazinyl- | 53 | Acetat | 260 |

IX.c. Abspaltung von Aminschutzgruppen von der Triazolopurin-Kernstruktur:

allgemeines Syntheseschema am Beispiel von Verbindungen der allgemeinen Formel (Ib):

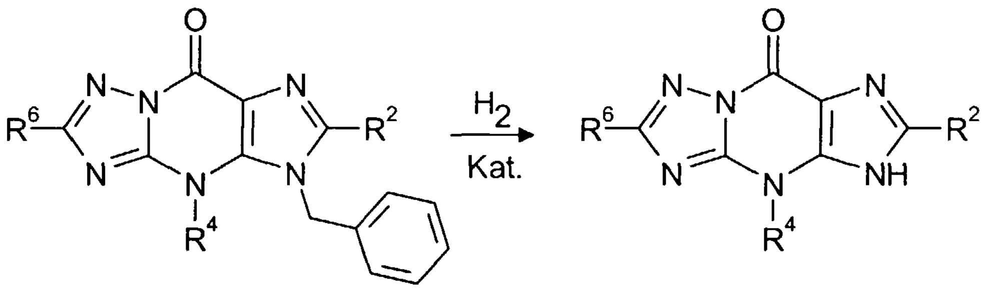


3-N-Benzylierte Triazolopurine der allgemeinen Formel (I) werden in Methanol oder absolutem Eisessig gelöst und unter einer Wasserstoffatmosphäre (1-5 bar) bei 25-80°C mit Hilfe eines Palladiumkatalysators (z.B. Pd/C) über einen Zeitraum von 2,5 - 40 h hydriert. Man saugt vom Katalysator ab, dampft ein und reinigt den Rückstand an Kieselgel. Das gereinigte Produkt wird aus dem Eluat kristallisiert, vorzugsweise mit Ether, und danach, soweit möglich, als Salz (vorzugsweise Methansulfonat) kristalliert. In dieser Weise wurden u.a. die nachstehenden Verbindungen erhalten.

Tabelle 11

| Nr. | $R_6$ | $R_4$ | $R_2$ | Salzform | Ausb. % | Fp (°C) |
|---|---|---|---|---|---|---|
| 11.1 | Methoxymethyl- | Ethyl- | Cyclopentyl- | - | 27 | 246-248 |
| 11.2 | Ethoxymethyl- | Ethyl- | Cyclopentyl- | - | 45 | 224-225 |
| 11.3 | Hydroxymethyl- | Ethyl- | Cyclopentyl- | - | 37 | 305-307 |
| 11.4 | Benzoyloxymethyl- | Ethyl- | Cyclopentyl- | - | 54 | 238-239 |
| 11.5 | Dimethylaminomethyl- | Ethyl- | Cyclopentyl- | Methansulfonat | 29 | 224-225 |
| 11.6 | N-Morpholinyl-methyl- | Ethyl- | Cyclopentyl- | Methansulfonat | 47 | 184 |
| 11.7 | Phenoxymethyl- | Ethyl- | Cyclopentyl- | - | 40 | 229 |

IX.d. Alkylierung Hydroxy-substituierter Triazolopurine:

allgemeines Syntheseschema am Beispiel von Verbindungen der allgemeinen Formel (Ib):

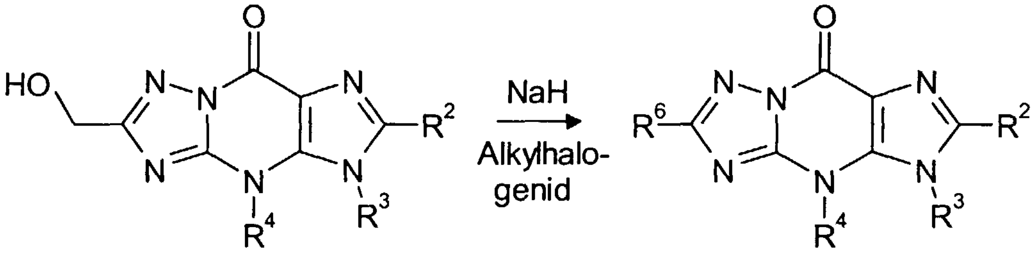


3 mMol Hydroxyalkyl- oder Hydroxyaryl-substituierter Triazolopurine der allgemeinen Formeln (Ia), (Ib), (Ic) oder (Id) werden in 30 ml wasserfreiem Dimethylformid gelöst, mit 3,6 mMol Natriumhydrid (60 %ige Suspension in Mineralöl) versetzt, 1 Stunde bei Raumtemperatur gerührt, mit 3,6 mMol Alkyljodid versetzt und 1-2 Tage bei 25-60° gerührt. Zur Vervollständigung der Umsetzung werden je 50 % Natriumhydrid und Alkylhalogenid nachgegeben. Zur Aufarbeitung wird die Umsetzung zum Rückstand eingedampft, in Methylenchlorid und Wasser aufgenommen, mit 2 n Salzsäure angesäuert, die Wasserphase noch einmal mit Methylenchlorid extrahiert, die vereinigten organischen Phasen mit Kochsalzlösung gewaschen und über Magnesiumsulfat getrocknet und eingedampft.
Der Rückstand wird an Kieselgel gereinigt und das Eluat aus Ether kristallisiert.
Nach diesem Verfahren wurden u.a. die folgenden Verbindungen hergestellt:

Tabelle 12

| Nr. | $R^6$ | $R^4$ | $R^1$ oder $R^3$ | $R^2$ | Ausbeute (%) | Fp (°C) |
|---|---|---|---|---|---|---|
| 12.1 | Methoxymethyl- | Ethyl- | Benzyl- | Cyclopentyl- | 37 | 116-118 |
| 12.2 | Ethoxymethyl- | Ethyl- | Benzyl- | Cyclopentyl- | 36 | 120-123 |

IX.e. Acylierung Hydroxy-substituierter Triazolopurine:

allgemeines Syntheseschema am Beispiel von Verbindungen der allgemeinen Formel (Ib):

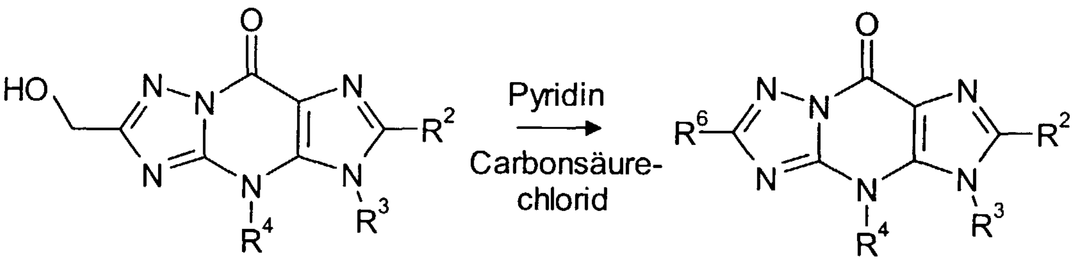


Die Hydroxylverbindung wird in Pyridin aufgenommen und unter Eiskühlung mit stöchiometrischen Mengen Säurechlorid versetzt. Nach vollständigem Umsatz wird der Ansatz auf Eiswasser gegeben und mit 2 n Salzsäure angesäuert. Die wässrige Phase wird mehrfach mit Methylenchlorid extrahiert. Die vereinigten organischen Phasen werden mit Kochsalzlösung gewaschen, über Magnesiumsulfat getrocknet und eingedampft. Der Rückstand wird an Kieselgel gereinigt und das Eluat aus Ether kristallisiert. Nach diesem Verfahren wurden u.a. die folgenden Verbindungen hergestellt:

Tabelle 13:

| Nr. | $R^6$ | $R^4$ | $R^1$ oder $R^3$ | $R^2$ | Ausbeute (%) |
|---|---|---|---|---|---|
| 12.1 | 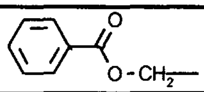 | Ethyl- | Benzyl- | Cyclopentyl- | 73 |
| 12.2 | 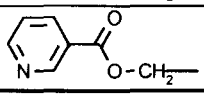 | Ethyl- | Benzyl- | Cyclopentyl- | 93 |

IX.f. Halogenierung Hydroxy-substituierter Triazolopurine:

allgemeines Syntheseschema am Beispiel von Verbindungen der allgemeinen Formel (Ib):

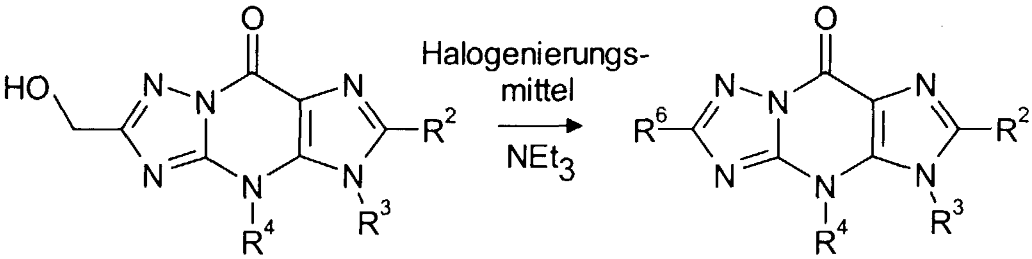


In üblicher Weise werden die hydroxylierten Triazolopurine mit Halogenierungsreagenzien wie $SOCl_2$, SOBr2, $POCl_3$ etc. in inerten Lösungsmitteln bei Anwesenheit von Basen (z.B. $NEt_3$) in die entsprechenden Halogenide überführt.

Entsprechend wurden u.a. die folgenden Verbindungen hergestellt:

Tabelle 14

| Nr. | $R^6$ | $R^4$ | $R^1$ oder $R^3$ | $R^2$ | Ausbeute (%) | Fp (°C) |
|---|---|---|---|---|---|---|
| 14.1 | $Br-CH_2-$ | Ethyl- | Benzyl- | Cyclopentyl- | 66 | 188-189 |

IX.g. Nukleophile Substitution an Halogen-substituierten Triazolopurinen:

allgemeines Syntheseschema am Beispiel von Verbindungen der allgemeinen Formel (Ib):

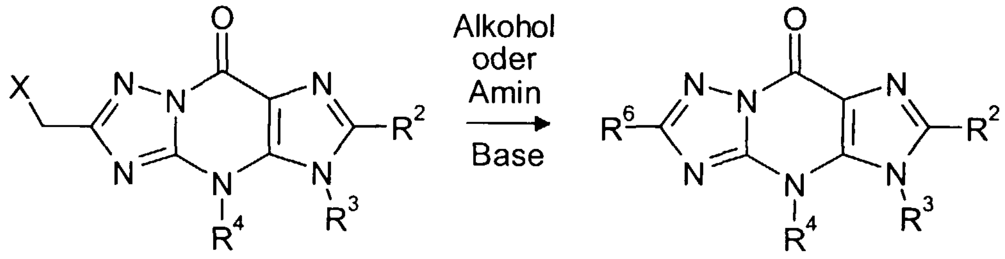


X: Chlor, Brom, Iod

Unter Anwendung allgemein bekannter Standardverfahren lassen sich Halogensubstituierte Triazolopurin-Derivate durch Umsetzung mit Nukleophilen (z.B. Alkohole, Amine) bei Anwesenheit von Base in die entsprechenden Amine und Alkyloxy-/Aryloxy-Verbindungen überführen. Zur Darstellung der Amine sei auf die zuvor für Stufe 8 beschriebenen Synthesevorschriften verwiesen. Die Alkyloxy-/Aryloxy-Verbindungen lassen sich entsprechend der allgemeinen Arbeitsvorschrift unter Punkt IX.d. synthetisieren. Entsprechend wurden u.a. die folgenden Verbindungen hergestellt:

Tabelle 15

| Nr. | $R^6$ | $R^4$ | $R^1$ oder $R^3$ | $R^2$ | Ausbeute (%) | Fp (°C) |
|---|---|---|---|---|---|---|
| 15.1 | $(CH_3)_2N-CH_2-$ | Ethyl- | Benzyl- | Cyclopentyl- | 100 | - |
| 15.2 | N-Morpholinomethyl- | Ethyl- | Benzyl- | Cyclopentyl- | 96 | - |
| 15.3 | Phenyloxymethyl- | Ethyl- | Benzyl- | Cyclopentyl- | 86 | 155 |

<u>IX.h. Oxidation Hydroxy-substituierter Triazolopurine:</u>

allgemeines Syntheseschema am Beispiel von Verbindungen der allgemeinen Formel (Ib):

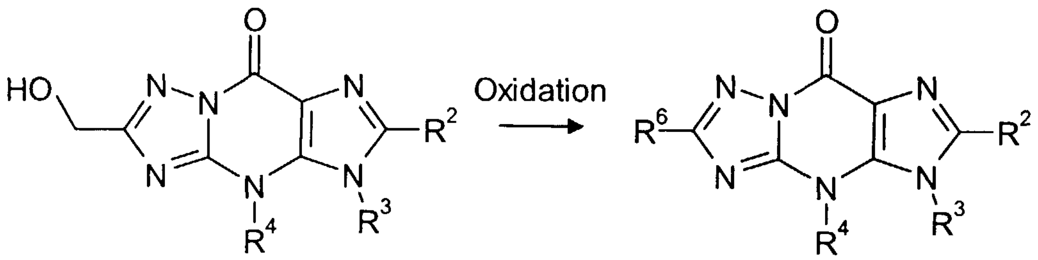


Unter Anwendung allgemein bekannter Standardverfahren (z.B. Tetrahedron Letters 1979, *5,* 399-402) lassen sich Hydroxy-substituierte Triazolopurin-Derivate in Triazolopurine mit Funktionalitäten höherer Oxidationsstufe (z.B. Aldehyde, Carbonsäuren) überführen. Entsprechend wurden u.a. die folgenden Verbindungen hergestellt:

Tabelle 16

| Nr. | $R^6$ | $R^4$ | $R^1$ oder $R^3$ | $R^2$ | Ausbeute (%) | Fp (°C) |
|---|---|---|---|---|---|---|
| 16.1 | -CHO | Ethyl- | Benzyl- | Cyclopentyl- | 48 | 153-154 |

Tabelle 16 (fortgesetzt)

| Nr. | $R^6$ | $R^4$ | $R^1$ oder $R^3$ | $R^2$ | Ausbeute (%) | Fp (°C) |
|---|---|---|---|---|---|---|
| 16.2 | -COOH | Ethyl- | Benzyl- | Cyclopentyl- | 38 | 157-158 |

IX.i. Derivatisierung von Triazolopurin-Carbonsäuren:

allgemeines Syntheseschema am Beispiel von Verbindungen der allgemeinen Formel (Ib):

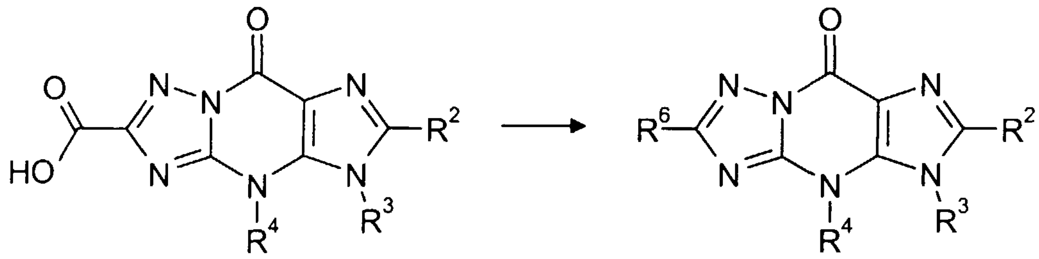

Mit Hilfe literaturbekannter Verfahren lassen sich Triazolopurin-Carbonsäuren in eine Vielzahl von Carbonsäure-Derivaten (z.B. Halogenide, Anhydride, Amide, Ester etc.) umwandeln. Zur Darstellung der entsprechenden Triazolopurin-Carbonsäuremethylester kann die folgende Allgemeine Arbeitsvorschrift dienen:

0,003 mol Triazolopurincarbonsäure werden in einer 1 molaren Lsg. aus $SOCl_2$ (1,5 eq) in MeOH suspendiert. Die entstehende klare Lsg. wird bei Raumtemperatur gerührt. Nach vollständiger Reaktion wird das Lösungsmittel im Vakuum abdestilliert und der verbleibende Rückstand durch Chromatographie an Kieselgel gereinigt. Nach diesem Verfahren wurden u.a. hergestellt:

Tabelle 17

| Nr. | $R^6$ | $R^4$ | $R^1$ oder $R^3$ | $R^2$ | Ausbeute (%) | Fp (°C) |
|---|---|---|---|---|---|---|
| 17.1 | $-COOCH_3$ | Ethyl- | Benzyl- | Cyclopentyl- | 71 | 155-157 |

IX.k. Reduktive Aminierung von Triazolopurin-Aldehyden:

allgemeines Syntheseschema am Beispiel von Verbindungen der allgemeinen Formel (Ib):

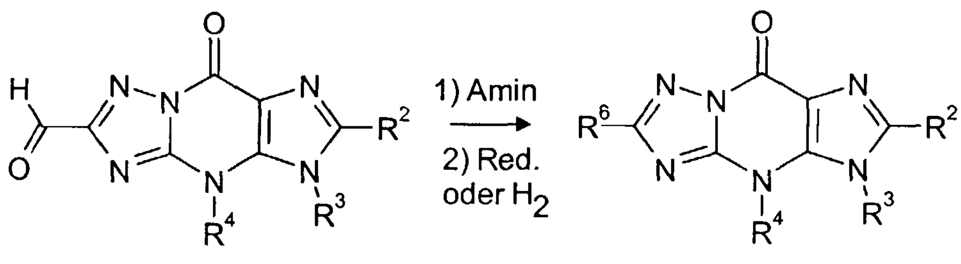


Unter Anwendung allgemeien bekannter Standardverfahren lassen sich Triazolopurin-Aldehyde durch Umsetzung mit primären oder sekundären Aminen und anschließende Reduktion oder Hydrierung der intermediär gebildeten Schiffschen Base in die entsprechenden Amine überführen. Entsprechend wurden u.a. die folgenden Verbindungen hergestellt:

Tabelle 18:

| Nr. | $R^6$ | $R^4$ | $R^1$ oder $R^3$ | $R^2$ | Ausbeute (%) | Fp (°C) |
|---|---|---|---|---|---|---|
| 18.1 | 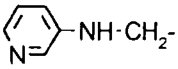 | Ethyl- | Benzyl- | Cyclopentyl- | - | - |

Tabelle 19 faßt die in Analogie zu den zuvor beschriebenen Verfahren hergestellten Verbindungen der allgemeinen Formel (I) zusammen:

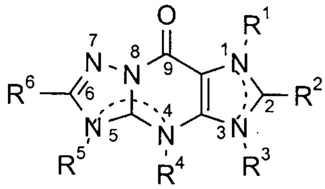

(I)

Tabelle 19:

| Beispiel Nr. | $-R^1$ oder $-R^3$ | $-R^2$ | $-R^4$ | $-R^5$ | $-R^6$ | Schmelzpunkt in °C | Chemische Bezeichnung |
|---|---|---|---|---|---|---|---|
| 1 | -H | -H | $-R^4$ oder $-R^5$ = -H | | 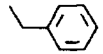 | > 300 | 6-Benzyl-1,2,4-triazolo[1,5-a]purin-9-on |
| 2 | -H | -H | -Methyl | - | 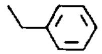 | > 300 | 6-Benzyl-4-methyl-1,2,4-triazolo[1,5-a]-purin-9-on |
| 3 | -H | -H | -n-Propyl | - | 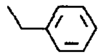 | > 300 | 6-Benzyl-4-n-propyl-1,2,4-triazolo[1,5-a]-purin-9-on |
| 4 | -H | -H | -n-Butyl | - | 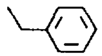 | > 300 | 6-Benzyl-4-n-butyl-1,2,4-triazolo[1,5-a]-purin-9-on |
| 5 | -H | -H | -i-Propyl | - | 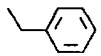 | > 300 | 6-Benzyl-4-i-propyl-1,2,4-triazolo[1,5-a]-purin-9-on |

| Beispiel Nr. | $-R^1$ oder $-R^3$ | $-R^2$ | $-R^4$ | $-R^5$ | $-R^6$ | Schmelzpunkt in °C | Chemische Bezeichnung |
|---|---|---|---|---|---|---|---|
| 6 | -H | -Ethyl | -n-Propyl | - | 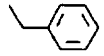 | 223 | 6-Benzyl-2-ethyl-4-n-propyl-1,2,4-triazolo-[1,5-a]purin-9-on |
| 7 | -H | -n-Propyl | -n-Propyl | - | 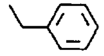 | 202 | 6-Benzyl-2,4-di-n-propyl-1,2,4-triazolo-[1,5-a]purin-9-on |
| 8 | -H | 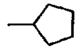 | -Methyl | - | 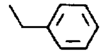 | 265-267 | 6-Benzyl-2-cyclopentyl-4-methyl-1,2,4-triazolo[1,5-a]purin-9-on |
| 9 | -H | 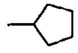 | -Ethyl | - | 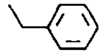 | 242 | 6-Benzyl-2-cyclopentyl-4-ethyl-1,2,4-triazolo[1,5-a]purin-9-on |
| 10 | -H | 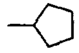 | -n-Propyl | - | 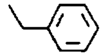 | 234-235 | 6-Benzyl-2-cyclopentyl-4-n-propyl-1,2,4-triazolo[1,5-a]purin-9-on |
| 11 | -H | 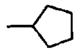 | -n-Butyl | - | 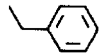 | 238-240 | 6-Benzyl-4-n-butyl-2-cyclopentyl-1,2,4-triazolo[1,5-a]purin-9-on |
| 12 | -H | 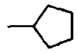 | 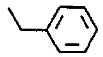 | - | 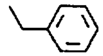 | 270 | 4,6-Dibenzyl-2-cyclopentyl-1,2,4-triazolo-[1,5-a]purin-9-on |
| 13 | -H | 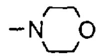 | -n-Propyl | - | 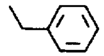 | 298 | 6-Benzyl-4-n-propyl-2-(N-morpholino)-1,2,4-triazolo[1,5-a]purin-9-on |
| 14 | -H | 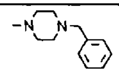 | -n-Propyl | - | 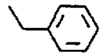 | 266-268 | 6-Benzyl-2-(4-benzylpiperazino)-4-n-propyl-1,2,4-triazolo[1,5-a]purin-9-on |
| 15 | -H | 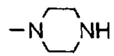 | -n-Propyl | - | 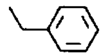 | 260 | 6-Benzyl-4-n-propyl-2-(1-piperazinyl)-1,2,4-triazolo[1,5-a]purin-9-on |

| Beispiel Nr. | $-R^1$ oder $-R^3$ | $-R^2$ | $-R^4$ | $-R^5$ | $-R^6$ | Schmelzpunkt in °C | Chemische Bezeichnung |
|---|---|---|---|---|---|---|---|
| 16 | -H | 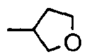 | -Ethyl | - | 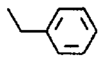 | 237 | 6-Benzyl-4-ethyl-2-(3-tetrahydrofuryl)-1,2,4-triazolo[1,5-a]purin-9-on |
| 17 | -H | 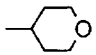 | -Ethyl | - | 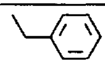 | 227 | 6-Benzyl-4-ethyl-2-(4-tetrahydropyranyl)-1,2,4-triazolo[1,5-a]purin-9-on |
| 18 | -H | 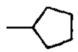 | $-R^4$ oder $-R^5$ = -H | | 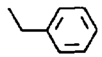 | 270 Zers. | 6-Benzyl-2-cyclopentyl-1,2,4-triazolo-[1,5-a]purin-9-on |
| 19 | -H | -H | $-R^4$ oder $-R^5$ = -H | | 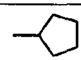 | 215 | 6-Cyclopentyl-1,2,4-triazolo[1,5-a]purin-9-on |
| 20 | -H | -H | -Methyl | - | 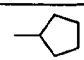 | > 300 | 6-Cyclopentyl-4-methyl-1,2,4-triazolo[1,5-a]purin-9-on |
| 21 | -H | -H | -n-Propyl | - | 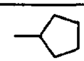 | 287 Zers. | 6-Cyclopentyl-4-n-propyl-1,2,4-triazolo-[1,5-a]purin-9-on |
| 22 | -H | $-CH_3$ | -n-Propyl | - | 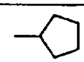 | 248-250 | 6-Cyclopentyl-4-n-propyl-2-methyl-1,2,4-triazolo[1,5-a]purin-9-on |
| 23 | -H | -Ethyl | -n-Propyl | - | 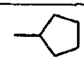 | 245-248 | 6-Cyclopentyl-2-ethyl-4-n-propyl-1,2,4-triazolo[1,5-a]purin-9-on |
| 24 | -H | 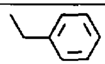 | -n-Propyl | - | 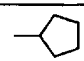 | 233 | 2-Benzyl-6-cyclopentyl-4-n-propyl-1,2,4-triazolo[1,5-a]purin-9-on |
| 25 | -H | 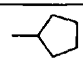 | -n-Pentyl | - | 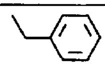 | 231-236 | 6-Benzyl-2-cyclopentyl-4-n-pentyl-1,2,4-triazolo[1,5-a]purin-9-on |

| Beispiel Nr. | $-R^1$ oder $-R^3$ | $-R^2$ | $-R^4$ | $-R^5$ | $-R^6$ | Schmelzpunkt in °C | Chemische Bezeichnung |
|---|---|---|---|---|---|---|---|
| 26 | -H | 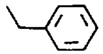 | $-CH_3$ | - | -H | 306 | 2-Benzyl-4-methyl-1,2,4-triazolo-[1,5-a]purin-9-on |
| 27 | -H | 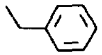 | -n-Propyl | - | -H | 222-223 | 2-Benzyl-4-n-propyl-1,2,4-triazolo-[1,5-a]purin-9-on |
| 28 | -H | 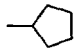 | $-R^4$ oder $-R^5$ = -H | | -H | > 360 | 2-Cyclopentyl-1,2,4-triazolo[1,5-a]purin-9-on |
| 29 | -H | 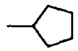 | -Methyl | - | -H | 285 | 2-Cyclopentyl-4-methyl-1,2,4-triazolo-[1,5-a]purin-9-on |
| 30 | -H | 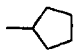 | -Ethyl | - | -H | 249-253 | 2-Cyclopentyl-4-ethyl-1,2,4-triazolo-[1,5-a]purin-9-on |
| 31 | -H | 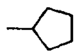 | -n-Propyl | - | -H | 250 | 2-Cyclopentyl-4-n-propyl-1,2,4-triazolo-[1,5-a]purin-9-on |
| 32 | -H | 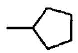 | -Ethyl | - | $-COOCH_3$ | 270-271 | 6-Carboxymethyl-2-cyclopentyl-4-ethyl-1,2,4-triazolo-[1,5-a]purin-9-on |
| 33 | -H | 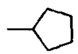 | -n-Propyl | - | $-CH_3$ | 262-264 | 2-Cyclopentyl-6-methyl-4-n-propyl-1,2,4-triazolo[1,5-a]purin-9-on |
| 34 | -H | -H | -n-Propyl | - | -Ethyl | > 300 | 6-Ethyl-4-n-propyl-1,2,4-triazolo-[1,5-a]purin-9-on |
| 35 | -H | 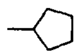 | -n-Propyl | - | -Ethyl | 253 | 2-Cyclopentyl-6-ethyl-4-n-propyl-1,2,4-triazolo[1,5-a]purin-9-on |

| Beispiel Nr. | $-R^1$ oder $-R^3$ | $-R^2$ | $-R^4$ | $-R^5$ | $-R^6$ | Schmelzpunkt in °C | Chemische Bezeichnung |
|---|---|---|---|---|---|---|---|
| 36 | -H | 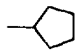 | -n-Propyl | - | -n-Propyl | 256 | 2-Cyclopentyl-4,6-di-n-propyl-1,2,4-triazolo-[1,5-a]purin-9-on |
| 37 | -H | 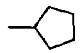 | -n-Propyl | - | -i-Propyl | 260-261 | 2-Cyclopentyl-6-i-propyl-4-n-propyl-1,2,4-triazolo[1,5-a]purin-9-on |
| 38 | -H | 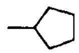 | -n-Propyl | - | -n-Butyl | 246 | 6-n-Butyl-2-cyclopentyl-4-n-propyl-1,2,4-triazolo[1,5-a]purin-9-on |
| 39 | -H | 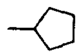 | -n-Propyl | - | 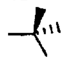 | 310 | 6-t-Butyl-2-cyclopentyl-4-n-propyl-1,2,4-triazolo[1,5-a]purin-9-on |
| 40 | -H | 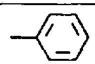 | -n-Propyl | - | -n-Propyl | 300 Zers. | 4,6-Di-n-propyl-2-phenyl-1,2,4-triazolo-[1,5-a]purin-9-on |
| 41 | -H | 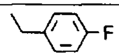 | -n-Propyl | - | -n-Propyl | 221 | 4,6-Di-n-propyl-2-(p-fluorbenzyl)-1,2,4-triazolo[1,5-a]purin-9-on |
| 42 | -H | -Ethyl | -n-Propyl | - | -Ethyl | 229 | 2,6-Diethyl-4-n-propyl-1,2,4-triazolo-[1,5-a]purin-9-on |
| 43 | -H | -H | -n-Propyl | - | 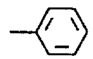 | 295-296 Zers. | 6-Phenyl-4-n-propyl-1,2,4-triazolo-[1,5-a]purin-9-on |
| 44 | -H | -Ethyl | -n-Propyl | - | 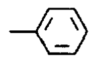 | > 300 Zers. | 2-Ethyl-6-phenyl-4-n-propyl-1,2,4-triazolo-[1,5-a]purin-9-on |
| 45 | -H | 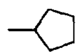 | -n-Propyl | - | 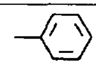 | > 300 Zers. | 2-Cyclopentyl-6-phenyl-4-n-propyl-1,2,4-triazolo[1,5-a]purin-9-on |

| Beispiel Nr. | -$R^1$ oder -$R^3$ | -$R^2$ | -$R^4$ | -$R^5$ | -$R^6$ | Schmelz-punkt in °C | Chemische Bezeichnung |
|---|---|---|---|---|---|---|---|
| 46 | -H | -H | -n-Propyl | - | 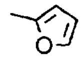 | > 300 Zers. | 6-(2-Furyl)-4-n-propyl-1,2,4-triazolo-[1,5-a]purin-9-on |
| 47 | -H | -Ethyl | -n-Propyl | - | 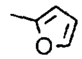 | > 300 Zers. | 2-Ethyl-6-(2-furyl)-4-n-propyl-1,2,4-triazolo-[1,5-a]purin-9-on |
| 48 | -H | 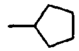 | -n-Propyl | - | 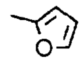 | > 300 Zers. | 2-Cyclopentyl-6-(2-furyl)-4-n-propyl-1,2,4-triazolo[1,5-a]purin-9-on |
| 49 | -n-Propyl | -H | -$R^4$ oder -$R^5$ = -H | | 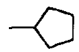 | 271-273 | 6-Cyclopentyl-1-n-propyl-1,2,4-triazolo-[1,5-a]purin-9-on |
| 50 | -H | -H | -$R^4$ oder -$R^5$ = -H | | -H | > 300 | 1,2,4-triazolo[1,5-a]purin-9-on |
| 51 | -H | 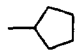 | - | -Ethyl | -H | 260 | 2-Cyclopentyl-5-ethyl-1,2,4-triazolo-[1,5-a]purin-9-on |
| 52 | -H | 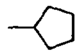 | - | -n-Propyl | -H | 217-220 | 2-Cyclopentyl-5-n-propyl-1,2,4-triazolo-[1,5-a]purin-9-on |
| 53 | -$CH_3$ | 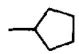 | -$CH_3$ | - | -H | 206 | 2-Cyclopentyl-3,4-dimethyl-1,2,4-triazolo-[1,5-a]purin-9-on |
| 54 | -Ethyl | -H | -Ethyl | - | 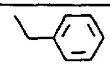 | > 300 | 6-Benzyl-3,4-diethyl-1,2,4-triazolo-[1,5-a]purin-9-on |
| 55 | -H | -H | - | -n-Propyl | 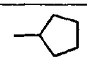 | 202-203 | 6-Cyclopentyl-5-n-propyl-1,2,4-triazolo-[1,5-a]purin-9-on |

| Beispiel Nr. | $-R^1$ oder $-R^3$ | $-R^2$ | $-R^4$ | $-R^5$ | $-R^6$ | Schmelzpunkt in °C | Chemische Bezeichnung |
|---|---|---|---|---|---|---|---|
| 56 | -n-Propyl | -H | -n-Propyl | - | 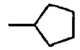 | 146 | 6-Cyclopentyl-1,4-di-n-propyl-1,2,4-triazolo-[1,5-a]purin-9-on |
| 57 | -n-Propyl | -H | -n-Propyl | - | 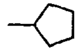 | 151 | 6-Cyclopentyl-3,4-di-n-propyl-1,2,4-triazolo-[1,5-a]purin-9-on |
| 58 | -H | 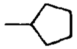 | -n-Butyl | - | -H | 243 | 2-Cyclopentyl-4-n-butyl-1,2,4-triazolo-[1,5-a]purin-9-on |
| 59 | -H | 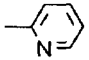 | -Ethyl | - | 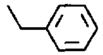 | 287 | 6-Benzyl-4-ethyl-2-(2-pyridyl)-1,2,4-triazolo-[1,5-a]purin-9-on |
| 60 | -H | 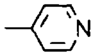 | -Ethyl | - | 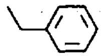 | 346 | 6-Benzyl-4-ethyl-2-(4-pyridyl)-1,2,4-triazolo-[1,5-a]purin-9-on |
| 61 | -H | 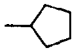 | - | 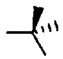 | -H | 149-150 | 2-Cyclopentyl-5-tert.-butyl-1,2,4-triazolo-[1,5-a]purin-9-on |
| 62 | -H | 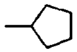 | 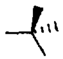 | - | -H | 190-191 | 2-Cyclopentyl-4-tert.-butyl-1,2,4-triazolo-[1,5-a]purin-9-on |
| 63 | -H | 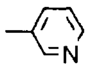 | -Ethyl | - | 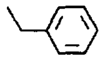 | 331-332 | 6-Benzyl-4-ethyl-2-(3-pyridyl)-1,2,4-triazolo-[1,5-a]purin-9-on |
| 64 | -H | 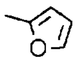 | -n-Propyl | - | 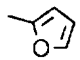 | 385-387 | 2,6-Di-(2-furyl)-4-n-propyl-1,2,4-triazolo-[1,5-a]purin-9-on |
| 65 | -H | $-N(CH_3)_2$ | -n-Propyl | - | 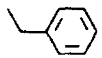 | 272-274 | 6-Benzyl-2-dimethylamino-4-n-propyl-1,2,4-triazolo-[1,5-a]purin-9-on |

| Beispiel Nr. | -$R^1$ oder -$R^3$ | -$R^2$ | -$R^4$ | -$R^5$ | -$R^6$ | Schmelz-punkt in °C | Chemische Bezeichnung |
|---|---|---|---|---|---|---|---|
| 66 | -H | 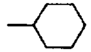 | -Ethyl | - | 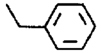 | 260-262 | 6-Benzyl-2-cyclohexyl-4-ethyl-1,2,4-triazolo-[1,5-a]purin-9-on |
| 67 | -H | 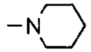 | -n-Propyl | - | 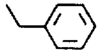 | 294-297 | 6-Benzyl-2-(N-piperidinyl)-4-n-propyl-1,2,4-triazolo-[1,5-a]purin-9-on |
| 68 | -H | 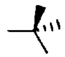 | -Ethyl | - | 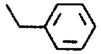 | 243-244 | 6-Benzyl-2-tert.-butyl-4-ethyl-1,2,4-triazolo-[1,5-a]purin-9-on |
| 69 | -H | 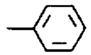 | -Ethyl | - | 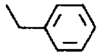 | 343-345 | 6-Benzyl-4-ethyl-2-phenyl-1,2,4-triazolo-[1,5-a]purin-9-on |
| 70 | -H | 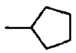 | -Ethyl | - | 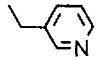 | 261-263 | 2-Cyclopentyl-4-ethyl-6-(3-pyridylmethyl)-1,2,4-triazolo-[1,5-a]purin-9-on |
| 71 | -H | -Ethyl | -Ethyl | - | 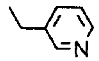 | 225-227 | 2,4-Diethyl-6-(3-pyridylmethyl)-1,2,4-triazolo-[1,5-a]purin-9-on |
| 72 | -Ethyl | 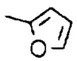 | -Ethyl | - | 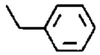 | 187-189 | 6-Benzyl-1,4-diethyl-2-(2-furyl)-1,2,4-triazolo-[1,5-a]purin-9-on |
| 73 | -H | -H | -Ethyl | - | 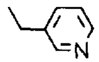 | 328-330 | 4-Ethyl-6-(3-pyridylmethyl)-1,2,4-triazolo-[1,5-a]purin-9-on |
| 74 | -H | -H | -Ethyl | - | 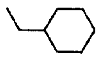 | 328-330 | 6-Cyclohexylmethyl-4-ethyl-1,2,4-triazolo-[1,5-a]purin-9-on |
| 75 | -H | -Ethyl | -Ethyl | - | 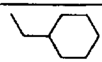 | 230-232 | 6-Cyclohexylmethyl-2,4-diethyl-1,2,4-triazolo-[1,5-a]purin-9-on |

| Beispiel Nr. | $-R^1$ oder $-R^3$ | $-R^2$ | $-R^4$ | $-R^5$ | $-R^6$ | Schmelzpunkt in °C | Chemische Bezeichnung |
|---|---|---|---|---|---|---|---|
| 76 | -H | 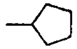 | -Ethyl | - | 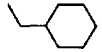 | 273-274 | 6-Cyclohexylmethyl-2-cyclopentyl-4-ethyl-1,2,4-triazolo-[1,5-a]purin-9-on |
| 77 | -H | -H | -Ethyl | - | 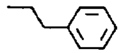 | 273 | 4-Ethyl-6-(phenylethyl)-1,2,4-triazolo-[1,5-a]purin-9-on |
| 78 | -H | -Ethyl | -Ethyl | - | 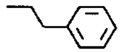 | 282-283 | 2,4-Diethyl-6-(phenylethyl)-1,2,4-triazolo-[1,5-a]purin-9-on |
| 79 | -H | 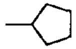 | -Ethyl | - | 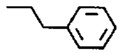 | 292-294 | 2-Cyclopentyl-4-ethyl-6-(phenylethyl)-1,2,4-triazolo-[1,5-a]purin-9-on |
| 80 | -H | 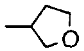 | -n-Propyl | - | 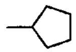 | 289-290 | 6-Cyclopentyl-4-n-propyl-2-(3-tetrahydrofuranyl)-1,2,4-triazolo-[1,5-a]purin-9-on |
| 81 | -H | 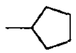 | -n-Propyl | - | 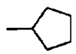 | 306-308 | 2,6-Dicyclopentyl-4-n-propyl-1,2,4-triazolo-[1,5-a]purin-9-on |
| 82 | -H | -Ethyl | -Ethyl | - | 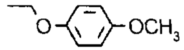 | 225 | 2,4-Diethyl-6-(4-methoxy-phenyloxy-methyl)-1,2,4-triazolo-[1,5-a]purin-9-on |
| 83 | -H | 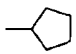 | -Ethyl | - | 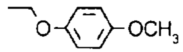 | 234-236 | 2-Cyclopentyl-4-ethyl-6-(4-methoxy-phenyloxy-methyl)-1,2,4-triazolo-[1,5-a]purin-9-on |
| 84 | 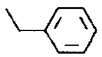 | 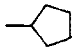 | -Ethyl | - | 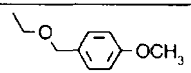 | 127-128 | 3-Benzyl-2-cyclopentyl-4-ethyl-6-(4-methoxy-benzyloxy-methyl)-1,2,4-triazolo-[1,5-a]purin-9-on |

| Beispiel Nr. | $-R^1$ oder $-R^3$ | $-R^2$ | $-R^4$ | $-R^5$ | $-R^6$ | Schmelzpunkt in °C | Chemische Bezeichnung |
|---|---|---|---|---|---|---|---|
| 85 | -H | 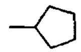 | -Ethyl | - | 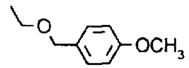 | 182 | 2-Cyclopentyl-4-ethyl-6-(4-methoxy-benzyloxy-methyl)-1,2,4-triazolo-[1,5-a]purin-9-on |
| 86 | 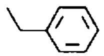 | 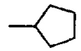 | -Ethyl | - | 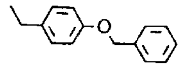 | 138-140 | 3-Benzyl-2-cyclopentyl-4-ethyl-6-(4-benzyloxy-benzyl)-1,2,4-triazolo-[1,5-a]purin-9-on |
| 87 | -H | 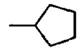 | -Ethyl | - | 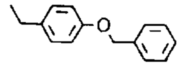 | 251 | 2-Cyclopentyl-4-ethyl-6-(4-benzyloxy-benzyl)-1,2,4-triazolo-[1,5-a]purin-9-on |
| 88 | -H | 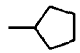 | -Ethyl | - | 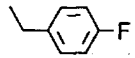 | 252-254 | 2-Cyclopentyl-4-ethyl-6-(4-fluor-benzyl)-1,2,4-triazolo-[1,5-a]purin-9-on |
| 89 | -H | 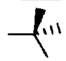 | -Ethyl | - | 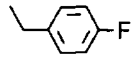 | 262-264 | 2-tert.-Butyl-4-ethyl-6-(4-fluor-benzyl)-1,2,4-triazolo-[1,5-a]purin-9-on |
| 90 | 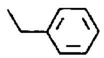 | 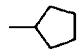 | -Ethyl | - | 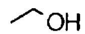 | 204-207 | 3-Benzyl-2-cyclopentyl-4-ethyl-6-hydroxymethyl-1,2,4-triazolo-[1,5-a]purin-9-on |
| 91 | -H | 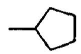 | -Ethyl | - | 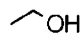 | 305-307 | 2-Cyclopentyl-4-ethyl-6-hydroxymethyl-1,2,4-triazolo-[1,5-a]purin-9-on |
| 92 | -H | 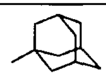 | -Ethyl | - | 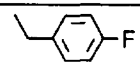 | 337-339 | 2-(Adamantan-1-yl)-4-ethyl-6-(4-fluor-benzyl)-1,2,4-triazolo-[1,5-a]purin-9-on |

| Beispiel Nr. | $-R^1$ oder $-R^3$ | $-R^2$ | $-R^4$ | $-R^5$ | $-R^6$ | Schmelzpunkt in °C | Chemische Bezeichnung |
|---|---|---|---|---|---|---|---|
| 93 | -H | 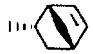 | -Ethyl | - | 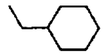 | 246-248 | 6-Cyclohexylmethyl-4-ethyl-2-(5-norbornen-2(*S*)-yl)-1,2,4-triazolo-[1,5-a]purin-9-on |
| 94 | -H | 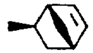 | -Ethyl | - | 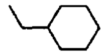 | 258-260 (Zers.) | 6-Cyclohexylmethyl-4-ethyl-2-(5-norbornen-2(*R*)-yl)-1,2,4-triazolo-[1,5-a]purin-9-on |
| 95 | 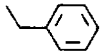 | 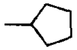 | -Ethyl | - | 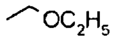 | 120-123 | 3-Benzyl-2-cyclopentyl-4-ethyl-6-ethyloxymethyl-1,2,4-triazolo-[1,5-a]purin-9-on |
| 96 | -H | 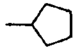 | -Ethyl | - | 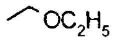 | 224-225 | 2-Cyclopentyl-4-ethyl-6-ethyloxymethyl-1,2,4-triazolo-[1,5-a]purin-9-on |
| 97 | 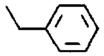 | 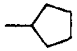 | -Ethyl | - | 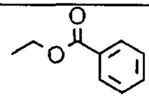 | - | 2-Cyclopentyl-4-ethyl-6-(benzoyloxymethyl)-1,2,4-triazolo-[1,5-a]purin-9-on |
| 98 | -H | 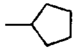 | -Ethyl | - | 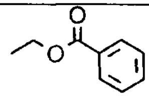 | 238-239 | 2-Cyclopentyl-4-ethyl-6-(benzoyloxymethyl)-1,2,4-triazolo-[1,5-a]purin-9-on |
| 99 | 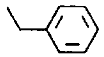 | 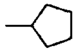 | -Ethyl | - | 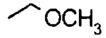 | 116-118 | 3-Benzyl-2-cyclopentyl-4-ethyl-6-methyloxymethyl-1,2,4-triazolo-[1,5-a]purin-9-on |
| 100 | -H | 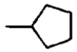 | -Ethyl | - | 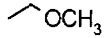 | 246-248 | 2-Cyclopentyl-4-ethyl-6-methyloxymethyl-1,2,4-triazolo-[1,5-a]purin-9-on |

| Beispiel Nr. | -$R^1$ oder -$R^3$ | -$R^2$ | -$R^4$ | -$R^5$ | -$R^6$ | Schmelzpunkt in °C | Chemische Bezeichnung |
|---|---|---|---|---|---|---|---|
| 101 | -H | 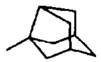 | -Ethyl | - | 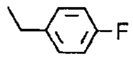 | 325-327 | 4-Ethyl-6-(4-fluor-benzyl)-2-(noradamantan-3-yl)-1,2,4-triazolo-[1,5-a]purin-9-on |
| 102 | 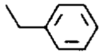 | 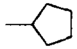 | -Ethyl | - | 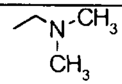 | - | 3-Benzyl-2-cyclopentyl-4-ethyl-6-(dimethylamino-methyl)-1,2,4-triazolo-[1,5-a]purin-9-on |
| 103 | -H | 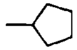 | -Ethyl | - | 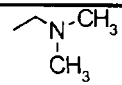 | 224-225[a] | 2-Cyclopentyl-4-ethyl-6-(dimethylamino-methyl)-1,2,4-triazolo-[1,5-a]purin-9-on |
| 104 | -H | 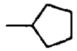 | -Ethyl | - | 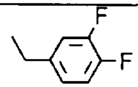 | 264-266 | 2-Cyclopentyl-4-ethyl-6-(3,4-difluor-benzyl)-1,2,4-triazolo-[1,5-a]purin-9-on |
| 105 | -H | 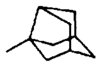 | -Ethyl | - | 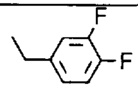 | 315-317 | 4-Ethyl-6-(3,4-difluor-benzyl)-2-(noradamantan-3-yl)-1,2,4-triazolo-[1,5-a]purin-9-on |
| 106 | 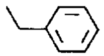 | 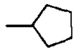 | -Ethyl | - | 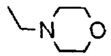 | - | 3-Benzyl-2-cyclopentyl-4-ethyl-6-(N-morpholino-methyl)-1,2,4-triazolo-[1,5-a]purin-9-on |
| 107 | -H | 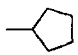 | -Ethyl | - | 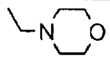 | 184[a] | 2-Cyclopentyl-4-ethyl-6-(N-morpholino-methyl)-1,2,4-triazolo-[1,5-a]purin-9-on |
| 108 | -H | 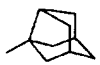 | -Ethyl | - | 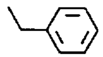 | 320-322 | 6-Benzyl-4-ethyl-2-(noradamantan-3-yl)-1,2,4-triazolo-[1,5-a]purin-9-on |

| Beispiel Nr. | $-R^1$ oder $-R^3$ | $-R^2$ | $-R^4$ | $-R^5$ | $-R^6$ | Schmelzpunkt in °C | Chemische Bezeichnung |
|---|---|---|---|---|---|---|---|
| 109 | -H | 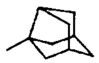 | -Ethyl | - | 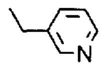 | 330-332 | 4-Ethyl-2-(noradamantan-3-yl)-6-(3-pyridylmethyl)-1,2,4-triazolo-[1,5-a]purin-9-on |
| 110 | 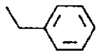 | 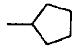 | -Ethyl | - | 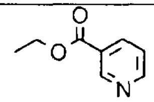 | - | 3-Benzyl-2-cyclopentyl-4-ethyl-6-(3-pyridylcarbonyloxy-methyl)-1,2,4-triazolo-[1,5-a]purin-9-on |
| 111 | 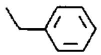 | 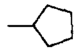 | -Ethyl | - | -COOH | 157-158 | 3-Benzyl-6-carboxy-2-cyclopentyl-4-ethyl-1,2,4-triazolo-[1,5-a]purin-9-on |
| 112 | 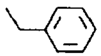 | 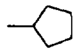 | -Ethyl | - | $-COOCH_3$ | 155-157 | 3-Benzyl-6-carboxymethyl-2-cyclopentyl-4-ethyl-1,2,4-triazolo-[1,5-a]purin-9-on |
| 113 | 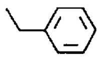 | 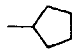 | -Ethyl | - | -CHO | 153-154 | 3-Benzyl-2-cyclopentyl-4-ethyl-6-formyl-1,2,4-triazolo-[1,5-a]purin-9-on |
| 114 | 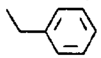 | 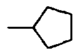 | -Ethyl | - | $-CH_2-Br$ | 153-154 | 3-Benzyl-6-brommethyl-2-cyclopentyl-4-ethyl-1,2,4-triazolo-[1,5-a]purin-9-on |
| 115 | 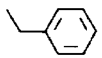 | 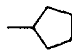 | -Ethyl | - | 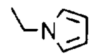 | 137-138 | 3-Benzyl-2-cyclopentyl-4-ethyl-6-(N-pyrolylmethyl)-1,2,4-triazolo-[1,5-a]purin-9-on |
| 116 | 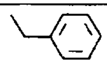 | 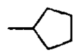 | -Ethyl | - | 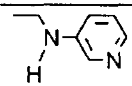 | - | 3-Benzyl-2-cyclopentyl-4-ethyl-6-(3-pyridylaminomethyl)-1,2,4-triazolo-[1,5-a]purin-9-on |

a): Methansulfonat

Die Strukturen der zuvor synthetisierten Beispiele der Verbindungen (Ia) bis (Id) sind durch NMR-Spektroskopie bestätigt worden.

NMR-spektroskopische Daten ausgewählter Verbindungen:

**Beispiel (3)**

$^1$H-NMR (DMSO-d6): $\delta$ = 14.10 (1H, s, broad, NH); 8.24 (1H, s, H2); 7.35-7.14 (5H, m, Aryl-H); 4.12 (2H, t, J = 7.5 Hz; $N\text{-}\underline{CH_2}CH_2CH_3$); 4.09 (2H, s, $-\underline{CH_2}$-Phenyl); 1.73 (2H, m, $N\text{-}CH_2\underline{CH_2}CH_3$); 0.89 (3H, t, J = 7.5 Hz, $N\text{-}CH_2CH_2\underline{CH_3}$).

**Beispiel (4)**

$^1$H-NMR (DMSO-d6): $\delta$ = 13.28 (1H, s, broad, NH); 8.24 (1H, s, H2); 7.35-7.14 (5H, m, Aryl-H); 4.15 (2H, t, J = 7.5 Hz, $N\text{-}\underline{CH_2}CH_2CH_2CH_3$); 1.68 (2H, m, $N\text{-}CH_2\underline{CH_2}CH_2CH_3$); 1.32 (2H, m, $N\text{-}CH_2CH_2\underline{CH_2}CH_3$); 0.90 (3H, t, J = 7.5 Hz, $N\text{-}CH_2CH_2CH_2\underline{CH_3}$).

**Beispiel (5)**

$^1$H-NMR (DMSO-d6): $\delta$ = 13.22 (1H, s, broad, NH); 8.25 (1H, s, H2); 7.35-7.14 (5H, m, Aryl-H);5.33 (1H, m, CH-Isopropyl); 4.09 (2H, s, $-\underline{CH_2}$-Phenyl); 1.55 (6H, d, J = 7.0 Hz, $(CH_3)_2$-Isopropyl).

**Beispiel (6)**

$^1$H-NMR (DMSO-d6): $\delta$ = 13.66 (1H, s, broad, NH); 7.34-7.14 (5H, m, Aryl-H); 4.11 (2H, t, J = 7.5 Hz, $N\text{-}\underline{CH_2}CH_2CH_3$); 4.07 (2H, s, $-\underline{CH_2}$-Phenyl); 2.79 (2H, qu, J = 7.5 Hz, $-\underline{CH_2}CH_3$); 1.71 (2H, m, $N\text{-}CH_2\underline{CH_2}CH_3$); 1.29 (3H, t, J = 7.5 Hz, $-CH_2\text{-}\underline{CH_3}$); 0.90 (3H, t, J = 7.5 Hz, $N\text{-}CH_2CH_2\underline{CH_3}$).

**Beispiel (7)**

$^1$H-NMR (DMSO-d6): $\delta$ = 13.70 (1H, s, broad, NH); 7.43-7.14 (5H, m, Aryl-H); 4.09 (2H, t, J = 7.5 Hz, $N\text{-}\underline{CH_2}CH_2CH_3$); 4.07 (2H, s, $-\underline{CH_2}$-Phenyl); 2.75 (2H, t, J = 7.5 Hz, $-\underline{CH_2}CH_2CH_3$); 1.74 (4H, m, $N\text{-}CH_2\underline{CH_2}CH_3$; $-CH_2\underline{CH_2}CH_3$); 0.92 (6H, m, $N\text{-}CH_2CH_2\underline{CH_3}$; $-CH_2CH_2\underline{CH_3}$).

**Beispiel (8)**

$^1$H-NMR (DMSO-d6): $\delta$ = 7.34-7.12 (5H, m, Aryl-H); 4.07 (2H, s, $CH_2$-Phenyl); 3.55 (3H, s, $N\text{-}CH_3$); 3.23 (1H, m, CH-Cyclopentyl); 2.14-1.52 (8H, m, $CH_2$-Cyclopentyl).

**Beispiel (9)**

$^1$H-NMR (DMSO-d6): $\delta$ = 13.68 (1H, s, broad, NH); 7.40-7.14 (5H, m, Aryl-H); 4.19 (2H, qu, J = 7.5 Hz, $N\text{-}\underline{CH_2}CH_3$); 4.09 (2H, s, $CH_2$-Aryl); 3.24 (1H, m, CH-Cyclopentyl); 2.26-1.50 (8H, m, $CH_2$-Cyclopentyl); 1.26 (3H, t, J = 7.5 Hz, $N\text{-}CH_2\underline{CH_3}$).

**Beispiel (10)**

$^1$H-NMR (DMSO-d6): δ = 13.65 (1H, s, broad, NH); 7.38-7.14 (5H, m, Aryl-H): 4.11 (2H, t, J = 7.5 Hz, N-$\underline{CH_2}CH_2CH_3$); 4.08 (2H, s, Phenyl-$\underline{CH_2}$-); 3.23 (1H, m, CH-Cyclopentyl); 2.14-1.49 (10 H, m, $CH_2$-Cyclopentyl; N-$CH_2\underline{CH_2}CH_3$); 0.89 (3H, t, J = 7.5 Hz, N-$CH_2CH_2\underline{CH_3}$).

**Beispiel (11)**

$^1$H-NMR (DMSO-d6): δ = 13.85 (1H, s, broad, NH); 7.62-7.33 (5H, m, Aryl-H); 4.34 (2H, t, J = 7.5 Hz, N-$\underline{CH_2}$); 4.28 (2H, s, $CH_2$-Phenyl); 3.43 (1H, m, CH-Cyclopentyl); 2.33-1.38 (12H, m, $CH_2$-Cyclopentyl; N-$CH_2$-$\underline{CH_2CH_2}$-$CH_3$); 1.10 (3H, t, J = 7.5 Hz, N-$(CH_2)_3$-$\underline{CH_3}$).

**Beispiel (12)**

$^1$H-NMR (DMSO-d6): δ = 12.58 (1H, s, broad, NH); 7.52-7.14 (10 H, m, Aryl-H); 5.52 (2H, s, N-$CH_2$-Aryl); 4.18 (2H, s, -$CH_2$-Aryl); 3.31 (1H, m, CH-Cyclopentyl); 2.21-1.50 (8H, m, $CH_2$-Cyclopentyl).

**Beispiel (13)**

$^1$H-NMR (DMSO-d6): δ = 12.38 (1H, s, broad, NH); 7.34-7.14 (5H, m, Aryl-H); 4.08 (2H, t, J = 7.5 Hz, N-$\underline{CH_2}CH_2CH_3$); 4.6 (2H s -$\underline{CH_2}$-Phenyl); 3.69; 3.59 (8H, m, morpholin-H); 1.70 (2H, m, N-$CH_2\underline{CH_2}CH_3$); 0.88 (3H, t, J = 7.5 Hz, N-$CH_2CH_2\underline{CH_3}$).

**Beispiel (14)**

$^1$H-NMR (DMSO-d6): δ = 12.26 (1H, s, broad, NH); 7.38-7.14 (10 H, m, Aryl-H); 4.08 (2H, t, J = 7.5 Hz, N-$\underline{CH_2}CH_2CH_3$); 4.06 (2H, s, $CH_2$-Phenyl); 3.57; 2.46 (8H, m, -$CH_2$-Piperaz.); 3.52 (2H, s, N-$CH_2$-Phenyl); 1.70 (2H, m, N-$CH_2\underline{CH_2}CH_3$); 0.88 (3H, t, J = 7.5 Hz, N-$CH_2CH_2\underline{CH_3}$).

**Beispiel (15)**

$^1$H-NMR (DMSO-d6): δ = 7.40-7.14 (5H, m, Aryl-H); 4.76 (3H, s, broad, NH, $NH_2$); 4.08 (2H, t, J = 7.5 Hz; N-$\underline{CH_2}CH_2$-$CH_3$); 4.06 (2H s -$\underline{CH_2}$-Phenyl); 3.48; 2.79 (8H, 2m, $CH_2$-Piper.); 1.90 (3H, s, $\underline{CH_3}COOH$); 1.70 (2H, m, N-$CH_2\underline{CH_2}$-$CH_3$); 0.88 (3H, t, J = 7.5 Hz, N-$CH_2CH_2$-$\underline{CH_3}$).

**Beispiel (16)**

$^1$H-NMR (DMSO-d6): δ = 13.82 (1H, s, broad, NH); 7.34-7.14 (5H, m, Aryl-H); 4.19 (2H, qu, J = 7.0 Hz, N-$\underline{CH_2}CH_3$); 4.08 (2H, s, -$\underline{CH_2}$-Phenyl); 3.93; 3.43 (4H, 2m, 2 $CH_2$-0); 3.09 (1H, m, -CH-THP); 2.00-1.68 (4H, m, 2-$CH_2$-THP); 1.25 (3H, t, J = 7.0 Hz, N-$CH_2\underline{CH_3}$).

**Beispiel (17)**

$^1$H-NMR (DMSO-d6): δ = 13.84 (1H, s, broad, NH); 7.34-7.14 (5H, m, Aryl-H); 4.18 (2H, qu, J = 7.0 Hz N-$\underline{CH_2}CH_3$); 4.08 (2H, s, -$\underline{CH_2}$-Phenyl); 4.08-3.74 (4H, m, 2 $CH_2$-O-3THF); 3.62 (1H, m, CH- 3 THF); 2.29 (2H, m, $CH_2$- 3 THF); 1.25 (3H, t, J = 7.0 Hz, N $CH_2\underline{CH_3}$).

**Beispiel (18)**

$^1$H-NMR (DMSO-d6): δ = 13.64; 12.76 (2H, 2s, broad, 2NH); 7.35-7.14 (5H, m, Aryl-H); 4.02 (2H, s, -$\underline{CH_2}$-Phenyl); 3.23 (1H, m, CH-Cyclopentyl); 2.14-1.50 (8H, m, $CH_2$-Cyclopentyl).

**Beispiel (19)**

$^1$H-NMR (DMSO-d6): δ = 13.42; 13.20 (2H, 2s, broad, 2 NH); 8.24 (1H, s, H2); 3.20 (1H, m, CH-Cyclopentyl); 2.17-1.50 (1H, m, $CH_2$-Cyclopentyl).

**Beispiel (20)**

$^1$H-NMR (DMSO-d6): δ = 13.86 (1H, s, broad, NH); 8.22 (1H, s, H2); 3.58 (3H, s, N-$CH_3$); 3.23 (1H, m, CH-Cyclopentyl); 2.14-1.52 (8H, m, $CH_2$-cylcopentyl).

**Beispiel (21)**

$^1$H-NMR (DMSO-d6): δ = 14.04 (1H, s, broad, NH); 8.24 (1H, s, H2); 4.13 (2H, t, J = 7.5 Hz, N-$\underline{CH_2}CH_2CH_3$); 3.21 (1H, m, CH-Cyclopentyl); 2.14-1.52 (10 H, m, $CH_2$-Cyclopentyl; N-$CH_2\underline{CH_2}CH_3$); 0.90 (3H, t, J = 7.5 Hz, N-$CH_2CH_2\underline{CH_3}$).

**Beispiel (22)**

$^1$H-NMR (DMSO-d6): δ = 13.68 (1H, s, broad, NH); 4.12 (2H, t, J = 7.5 Hz, N-$\underline{CH_2}$-$CH_2CH_3$); 3.20 (1H, m, CH-Cyclopropyl); 2.45 (3H, s, -$CH_3$); 2.12-1.52 (10 H, m, $CH_2$-Cyclopentyl); 0.89 (3H, t, J = 7.5 Hz, N-$CH_2$-$CH_2\underline{CH_3}$).

**Beispiel (23)**

$^1$H-NMR (DMSO-d6): δ = 13.64 (1H, s, broad, NH); 4.12 (2H, t, J = 7.5 Hz, N-$\underline{CH_2}$-$CH_2CH_3$); 3.20 (1H, m, CH-Cyclopentyl); 2.80 (2H, qu, J = 7.5 Hz, -$\underline{CH_2}$-$CH_3$); 2.13-1.52 (10 H, m, $CH_2$-Cyclopentyl; N-$CH_2$-$\underline{CH_2}CH_3$); 1.29 (3H, t, J = 7.5 Hz, - $CH_2$-$\underline{CH_3}$); 0.90 (3H, t, J = 7.5 Hz, -N-$CH_2$-$CH_2\underline{CH_3}$).

**Beispiel (24)**

$^1$H-NMR (DMSO-d6): δ = 13.88 (1H, s, broad, NH); 7.35-7.14 (5H, m, Aryl-H); 4.13 (2H, s, -$\underline{CH_2}$-Phenyl); 4.11 (2H, t, J = 7.5 Hz N-$\underline{CH_2}CH_2CH_3$); 3.19 (1H, m, CH-Cyclopentyl); 2.09-1.52 (1=H, m, $CH_2$-Cyclopentyl; N-$CH_2\underline{CH_2}CH_3$);

0.89 (3H, t, J = 7.5 Hz, N-$CH_2CH_2\underline{CH_3}$).

**Beispiel (25)**

$^1$H-NMR (DMSO-d6): δ = 13.60 (1H, s, broad, NH); 7.43-7.14 (5H, m, Aryl-H); 4.36 (2H, t, J = 7.5 Hz, N-$\underline{CH_2}(CH_2)_3$-$CH_3$); 4.19 (2H, s, -$\underline{CH_2}$-Phenyl); 3.40 (1H, m, CH-Cyclopentyl); 2.31-1.24 (14H, m, $CH_2$-Cyclopentyl; N-$CH_2(\underline{CH_2})_3$-$CH_3$); 0.89 (3H, m, N-$(CH_2)_4$-$\underline{CH_3}$).

**Beispiel (26)**

$^1$H-NMR (DMSO-d6): δ = 13.85 (1H, s, broad, NH); 8.16 (1H, s, H6); 7.35-7.14 (5H, m, Aryl-H); 4.15 (2H, s, -$\underline{CH_2}$-Phenyl); 3.59 (3H, s, N-$CH_3$).

**Beispiel (27)**

$^1$H-NMR (DMSO-d6): δ = 14.02 (1H, s, broad, NH); 8.19 (1H, s, H6); 7.35-7.14 (5H, m, Aryl-H): 4.18 (2H, s, -$\underline{CH_2}$-Phenyl); 4.15 (2H, t, J = 7.5 Hz, N-$\underline{CH_2}CH_2CH_3$); 1.75 (2H, m, N-$CH_2\underline{CH_2}CH_3$); 0.93 (3H, t, J = 7.5 Hz, N-$CH_2CH_2\underline{CH_3}$).

**Beispiel (28)**

$^1$H-NMR (DMSO-d6): δ = 13.50, 12.92 (2H, 2s, broad, 2NH); 8.10 (1H, s, H6); 3.24 (1H, m, CH-Cyclopentyl); 2.17-1.52 (8H, m, $CH_2$-Cyclopentyl).

**Beispiel (29)**

$^1$H-NMR (DMSO-d6): δ = 13.70 (1H, s, broad, NH); 8.18 (1H, s, H6); 3.59 (3H, s, C-$CH_3$); 3.25 (1H, m, CH-Cyclopentyl); 2.15-1.52 (8H, m, $CH_2$-Cyclopentyl).

**Beispiel (30)**

$^1$H-NMR (DMSO-d6): δ = 13.70 (1H, s, broad, NH); 8.20 (1H, s, H6); 4.25 (2H, qu, J = 7.0 Hz, N-$\underline{CH_2}$-$CH_3$); 3.27 (1H, m, CH-Cyclopentyl); 2.21-1.52 (8H, m, $CH_2$-Cyclopentyl); 1.29 (3H, t, J = 7.0 Hz, N-$CH_2$-$\underline{CH_3}$).

**Beispiel (31)**

$^1$H-NMR (DMSO-d6): δ = 13.74 (1H, s, broad, NH); 8.18 (1H, s, H6); 4.14 (2H, t, J = 7.5 Hz, N-$\underline{CH_2}$-$CH_2CH_3$); 3.25 (1H, m, CH-Cyclopentyl); 2.15-1.54 (10 H, m, $CH_2$-Cyclopentyl; N-$CH_2$-$\underline{CH_2}CH_3$); 0.90 (3H, t, J = 7.5 Hz, N-$CH_2$-$CH_2\underline{CH_3}$).

**Beispiel (33)**

$^1$H-NMR (DMSO-d6): δ = 13.62 (1H, s, broad, NH); 4.11 (2H, t, J = 7.5 Hz, N-$\underline{CH_2}CH_2CH_3$); 3.23 (1H, m, CH-Cyclopentyl); 2.37 (3H, s, -$CH_3$); 2.14-1.50 (10 H, m, $CH_2$-Cyclopentyl, N-$CH_2\underline{CH_2}CH_3$); 0.90 (3H, t, J = 7.5 Hz, N-$CH_2CH_2\underline{CH_3}$).

**Beispiel (34)**

$^1$H-NMR (DMSO-d6): δ = 8.22 (1H, s, Hz); 4.13 (2H, t, J = 7.5 Hz, N-$\underline{CH_2}CH_2CH_3$); 2.75 (2H, qu, J = 7.5 Hz, -$\underline{CH_2}CH_3$); 1.74 (2H, m, N-$CH_2\underline{CH_2}CH_3$); 1.29 (3H, t, J = 7.5 Hz, $CH_2$-$\underline{CH_3}$); 0.92 (3H, t, J = 7.5 Hz, N-$CH_2CH_2\underline{CH_3}$).

**Beispiel (35)**

$^1$H-NMR (DMSO-d6): δ = 13.62 (1H, s, broad, NH); 4.12 (2H, t, J = 7.5 Hz, N-$\underline{CH_2}CH_2CH_3$); 3.25 (1H, m, CH-Cyclopentyl); 2.74 (2H, qu, J = 7.5 Hz -$\underline{CH_2}CH_3$); 2.17-1.51 (10 H, m, $CH_2$-Cyclopentyl; N-$CH_2\underline{CH_2}CH_3$); 1.29 (3H, t, J = 7.5 Hz, - $CH_2\underline{CH_3}$); 0.90 (3H, t, J = 7.5 Hz, N-$CH_2CH_2\underline{CH_3}$).

**Beispiel (36)**

$^1$H-NMR (DMSO-d6): δ = 13.58 (1H, s, broad, NH); 4.12 (2H, t, J = 7.5 Hz, N-$\underline{CH_2}CH_2CH_3$); 3.24 (1H, m, CH-Cyclopentyl);2.69 (2H, t, J = 7.5 Hz, -$\underline{CH_2}CH_2CH_3$); 2.15-1.50 (12H, m, $CH_2$-Cyclopentyl; $(CH_2\underline{CH_2}CH_3)_2$); 0.95; 0.90 (6H, 2t, J = 7.5 Hz, $(CH_2CH_2\underline{CH_3})_2$).

**Beispiel (37)**

$^1$H-NMR (DMSO-d6): δ = 13.62 (1H, s, broad, NH); 4.13 (2H, t, J = 7.5 Hz, N-$\underline{CH_2}CH_2CH_3$); 3.24 (1H, m, CH-Cyclopentyl); 3.05 (1H, m, CH-Isoprop.); 2.14-1.50 (10 H, m, $CH_2$-Cyclopentyl; N-$CH_2\underline{CH_2}CH_3$); 1.32 (6H, d, J = 7.5 Hz, $CH_3$-Isoprop.); 0.90 (3H, t, J = 7.5 Hz, $CH_2CH_2\underline{CH_3}$).

**Beispiel (38)**

$^1$H-NMR (DMSO-d6): δ = 13.62 (1H, s, broad, NH); 4.12 (2H, t, J = 7.5 Hz, N-$\underline{CH_2}CH_2CH_3$); 3.24 (1H, m, Cyclopentyl-H); 2.70 (2H, t, J = 7.5 Hz, $\underline{CH_2}$-$CH_2CH_2CH_3$); 2.15-1.26 (14H, m, $CH_2$-Cyclopentyl; N-$CH_2\underline{CH_2}$-$CH_3$, $CH_2$-$\underline{CH_2CH_2}CH_3$); 0.90 (6H, m, N-$CH_2CH_2CH_3$); (-$CH_2)_3$-$\underline{CH_3}$).

**Beispiel (39)**

$^1$H-NMR ($CDCl_3$): δ = 4.36 (2H, t, J = 7.5 Hz, N-$\underline{CH_2}CH_2CH_3$); 3.40 (1H, m, CH -Cyclopentyl); 2.32-1.63 (10 H, m, $CH_2$-Cyclopentyl; N-$CH_2\underline{CH_2}CH_3$); 1.46 (9H, s, $C(CH_3)_3$; 1.02 (3H, t, J = 7.5 Hz, N-$CH_2CH_2CH_3$).

**Beispiel (40)**

$^{1}$H-NMR (DMSO-d6): δ = 14.28 (1H, s, broad, NH); 8.26-7.45 (5H, m, Aryl-H); 4.14 (2H, t, J = 7.5 Hz, N-$\underline{CH_2}CH_2CH_3$); 2.71 (2H, t, J = 7.5 Hz, -$\underline{CH_2}CH_2CH_3$); 1.76 (4H, m, (-$CH_2\underline{CH_2}CH_3)_2$); 0.98; 0.93 (6H, 2t, (-$CH_2CH_2\underline{CH_3})_2$).

**Beispiel (41)**

$^{1}$H-NMR (DMSO-d6): δ = 7.44-7.05 (4H, m, Aryl-H); 4.13 (2H, s, $CH_2$-Phenyl); 4.11 (2H, t, J = 7.5 Hz, N-$\underline{CH_2}CH_2$-$CH_3$); 2.68 (2H, t, J = 7.5 Hz, -$\underline{CH_2}CH_2$-$CH_3$); 1.74 (4H, m, (-$CH_2\underline{CH_2}$-$CH_3)_2$); 0.95; 0.89 (6H, 2t, J = 7.5 Hz, ($CH_2CH_2$-$\underline{CH_3})_2$).

**Beispiel (42)**

$^{1}$H-NMR (DMSO-d6): δ = 13.64 (1H, s, broad, NH); 4.12 (2H, t, J = 7.5 Hz; N-$\underline{CH_2}CH_2CH_3$); 2.76 (4H, m, ($\underline{CH_2}CH_3)_2$); 1.73 (2H, m, N-$CH_2\underline{CH_2}CH_3$)); 2.29 (6H, m, ($CH_2$-$\underline{CH_3})_2$); 0.90 (3H, t, J = 7.5 Hz, N-$CH_2CH_2\underline{CH_3}$).

**Beispiel (43)**

$^{1}$H-NMR (DMSO-d6): δ = 14.19 (1H, s, broad, NH); 8.31 (1H, s, H2); 8.16-7.38 (5H, m, Aryl-H); 4.21 (2H, t, J = 7.5 Hz, N-$\underline{CH_2}CH_2CH_3$); 1.83 (2H, m, N-$CH_2\underline{CH_2}CH_3$); 0.98 (3H, t, J = 7.5 Hz, N-$CH_2CH_2\underline{CH_3}$).

**Beispiel (44)**

$^{1}$H-NMR (DMSO-d6): δ = 13.78 (1H, s, broad, NH); 8.19-7.43 (5H, m, Aryl-H); 4.21 (2H, t, J = 7.5 Hz, N-$\underline{CH_2}CH_2CH_3$); 2.83 (2H, qu, J = 7.5 Hz, -$\underline{CH_2}CH_3$); 1.81 (2H, m, N-$CH_2\underline{CH_2}CH_3$); 1.33 (3H, t, j = 7.5 Hz, -$CH_2\underline{CH_3}$); 0.95 (3H, t, J = 7.5 Hz, -N-$CH_2CH_2\underline{CH_3}$).

**Beispiel (45)**

$^{1}$H-NMR (DMSO-d6): δ = 13.70 (1H, s, broad, NH); 8.19-7.38 (5H, m, Aryl-H); 4.21 (2H, t, J = 7.5 Hz, N-$\underline{CH_2}CH_2CH_3$); 3.28 (1H, m, CH-Cyclopentyl); 2.19-1.50 (10 H, m, $CH_2$-Cyclopentyl; N-$CH_2\underline{CH_2}CH_3$); 0.94 (3H, t, J = 7.5 Hz, N-$CH_2CH_2\underline{CH_3}$).

**Beispiel (46)**

$^{1}$H-NMR (DMSO-d6): δ = 8.26 (1H, s, Hz); 7.84; 7.10; 6.69 (3H, 3m, Furyl-H); 4.10 (2H, t, J = 7.5 Hz N-$\underline{CH_2}CH_2CH_3$); 1.81 (2H, m, N-$CH_2\underline{CH_2}CH_3$); 0.98 (3H, t, J = 7.5 Hz, N-$CH_2CH_2\underline{CH_3}$).

**Beispiel (47)**

$^{1}$H-NMR (DMSO-d6): δ = 13.56 (1H, s, broad, NH); 7.70; 6.91; 6.50 (3H, 3m, Furyl-H); 4.00 (2H, t, J = 7.5 Hz, N-

$\underline{CH_2}CH_2CH_3$); 2.66 (2H, qu, J = 7.5 Hz, -$\underline{CH_2}CH_3$); 1.61 (2H, m, N-$CH_2$-$\underline{CH_2}$-$CH_3$); 1.14 (3H, t, J = 7.5 Hz, N-$CH_2CH_2\underline{CH_3}$); 0.77 (3H, t, J = 7.5 Hz, -$CH_2\underline{CH_3}$).

**Beispiel (48)**

$^1$H-NMR (DMSO-d6): δ = 13.84 (1H, s, broad, NH); 8.00; 7.18; 6.78 (3H, 3m, Furan-H); 4.26 (2H, t, J = 7.5 Hz, N-$\underline{CH_2}CH_2CH_3$); 3.37 (1H, m, CH-Cyclopentyl); 2.28-1.62 (10 H, m, $CH_2$-Cyclopentyl; N-$CH_2\underline{CH_2}CH_3$); 1.04 (3H, t, J = 7.5 Hz; N-$CH_2CH_2\underline{CH_3}$).

**Beispiel (49)**

$^1$H-NMR (DMSO-d6): δ = 12.84 (1H, s, broad, NH); 8.26 (1H, s, H2); 4.32 (2H, t, J = 7.5 Hz, N-$\underline{CH_2}CH_2CH_3$); 3.18 (1H, m, CH-Cyclopentyl); 2.09-1.52 (10 H,m, $CH_2$-Cyclopentyl; N-$CH_2\underline{CH_2}CH_3$); 0.83 (3H, t, J = 7.5 Hz, N-$CH_2CH_2\underline{CH_3}$).

**Beispiel (50)**

$^1$H-NMR (DMSO-d6): δ = 8.28 (1H, s, H6); 8.15 (1H, s, H2); 13.35 (2H, s, broad).

**Beispiel (51)**

$^1$H-NMR (DMSO-d6): δ = 13.74 (1H, s, broad, NH); 8.34 (1H, s, H6); 4.63 (2H, qu, J = 7.5 Hz, N-$\underline{CH_2}CH_3$); 3.33 (1H, m, CH-Cyclopentyl); 2.24-1.57 (8H, m, $CH_2$-Cyclopentyl); 1.47 (3H, t, J = 7.5 Hz, N-$CH_2\underline{CH_3}$).

**Beispiel (52)**

$^1$H-NMR (DMSO-d6): δ = 13.72 (1H, s, broad, NH); 8.34 (1H, s, H6); 4.53 (2H, t, J = 7.5 Hz, N-$\underline{CH_2}CH_2CH_3$); 3.34 (1H, m, CH-Cyclopentyl); 2.20-1.57 (8H, m, $CH_2$-Cyclopentyl); 1.87 (2H, m, N-$CH_2\underline{CH_2}CH_3$); 1.06 (3H, t, J = 7.5 Hz, N-$CH_2CH_2\underline{CH_3}$).

**Beispiel (53)**

$^1$H-NMR (DMSO-d6): δ = 8.19 (1H, s, H6); 4.00 (3H, s, N-$CH_3$); 3.58 (3H, s, N-$CH_3$); 3.42 (1H, m, CH-Cyclopentyl); 2.18-1.57 (8H, m, $CH_2$-Cyclopentyl).

**Beispiel (55)**

$^1$H-NMR (DMSO-d6): δ = 14.12 (1H, s, broad, NH); 8.44 (1H, s, Hz); 4.53 (2H, t, J = 7.5 Hz, N-$\underline{CH_2}CH_2CH_3$); 3.28 (1H, m, CH-Cyclopentyl); 2.17-1.55 (8H, m, $CH_2$-Cyclopentyl); 1.87 (2H, m, N-$CH_2\underline{CH_2}CH_3$); 1.05 (3H, t, J = 7.5 Hz, N-$CH_2CH_2\underline{CH_3}$).

**Beispiel (56)**

$^1$H-NMR (DMSO-d6): δ = 8.28 (1H, s, H2); 4.36 (2H, t, J = 7.5 Hz, N-$\underline{CH_2}CH_2CH_3$); 4.12 (2H, t, J = 7.5 Hz, N-$\underline{CH_2}CH_2CH_3$); 3.21 (1H, m, CH-Cyclopentyl). 2.12-1.57 (12H, m, $CH_2$-Cyclopentyl; $(N-CH_2\underline{CH_2}CH_3)_2$); 0.92; 0.87 (6H, 2t, J = 7.5 Hz, $(N-CH_2CH_2\underline{CH_3})_2$).

**Beispiel (57)**

$^1$H-NMR (DMSO-d6): δ = 8.02 (1 H, s, H2); 4.45 (2H, t, J = 7.5 Hz, N-$\underline{CH_2}CH_2CH_3$); 4.14 (2H, t, J = 7.5 Hz, N-$\underline{CH_2}CH_2CH_3$); 3.24 (1H, m, CH-Cyclopentyl); 2.14-1.57 (12H, m, $CH_2$-Cyclopentyl; $(N-CH_2\underline{CH_2}CH_3)_2$; 0.93; 0.89 (6H, 2t, J = 7.5 Hz, $(N-CH_2CH_2\underline{CH_3})_2$;

Die nachfolgende Tabelle enthält $KiA_1$ (human) und $KiA_2$ (Ratte) Rezeptorbindungswerte.

Tabelle 20

| Beispiel-Nr.: | $K_iA_1$ [nM] | $K_iA_2$ [nM] |
|---|---|---|
| 7 | 8,1 | 158 |
| 8 | 1,9 | 363 |
| 9 | 1,4 | 422 |
| 10 | 1,7 | 730 |
| 11 | 8,3 | 345 |
| 13 | 5,2 | 82 |
| 25 | 10,3 | 1231 |
| 30 | 81,5 | 3292 |
| 36 | 5,8 | 731 |
| 37 | 6,4 | 307 |
| 38 | 6,4 | 532 |
| 39 | 6,0 | 539 |
| 48 | 11,4 | 4455 |

Die nachfolgende Tabelle enthält $KiA_3$ (human) Rezeptorbindungswerte.

Tabelle 21

| Beispiel Nr. | $K_iA_3$ [nM] |
|---|---|
| 3 | 4,7 |
| 4 | 2,3 |
| 6 | 2 |
| 12 | 20 |
| 19 | 3,8 |
| 23 | 2 |
| 25 | 26 |

Tabelle 21 (fortgesetzt)

| Beispiel Nr. | $K_iA_3$ [nM] |
|---|---|
| 38 | 22 |
| 65 | 3 |
| 68 | 30 |
| 78 | 5,3 |
| 82 | 25 |

Die Verbindungen der allgemeinen Formel (I) können allein oder in Kombination mit anderen erfindungsgemäßen Wirkstoffen, gegebenenfalls auch in Kombination mit weiteren pharmakologisch aktiven Wirkstoffen, zur Anwendung gelangen. Geeignete Anwendungsformen sind beispielsweise Tabletten, Kapseln, Zäpfchen, Lösungen, Säfte, Emulsionen oder dispersible Pulver. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Alkalisalzen der Ethylendiamintetraessigsäure hergestellt und in Injektionsflaschen oder Ampullen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Eine therapeutisch wirksame Tagesdosis beträgt zwischen 1 und 800 mg, bevorzugt 10 - 300 mg pro Erwachsener.

Die nachfolgenden Beispiele illustrieren die vorliegende Erfindung ohne sie jedoch in ihrem Umfang zu beschränken:

Pharmazeutische Formulierungsbeispiele

A)

| Tabletten | pro Tablette |
|---|---|
| Wirkstoff | 100 mg |
| Milchzucker | 140 mg |
| Maisstärke | 240 mg |

(fortgesetzt)

| Tabletten | pro Tablette |
|---|---|
| Polyvinylpyrrolidon | 15 mg |
| Magnesiumstearat | 5 mg |
| | 500 mg |

Der feingemahlene Wirkstoff, Milchzucker und ein Teil der Maisstärke werden miteinander vermischt. Die Mischung wird gesiebt, worauf man sie mit einer Lösung von Polyvinylpyrrolidon in Wasser befeuchtet, knetet, feuchtgranuliert und trocknet. Das Granulat, der Rest der Maisstärke und das Magnesiumstearat werden gesiebt und miteinander vermischt. Das Gemisch wird zu Tabletten geeigneter Form und Größe verpreßt.

B)

| Tabletten | pro Tablette |
|---|---|
| Wirkstoff | 80 mg |
| Maisstärke | 190 mg |
| Milchzucker | 55 mg |
| Mikrokristalline Cellulose | 35 mg |
| Polyvinylpyrrolidon | 15 mg |
| Natrium-carboxymethylstärke | 23 mg |
| Magnesiumstearat | 2 mg |
| | 400 mg |

Der feingemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natriumcarboxymethylstärke und das Magnesiumstearat, vermischt und verpreßt das Gemisch zu Tabletten geeigneter Größe.

C)

| Dragées | pro Dragée |
|---|---|
| Wirkstoff | 5 mg |
| Maisstärke | 41,5 mg |
| Milchzucker | 30 mg |
| Polyvinylpyrrolidon | 3 mg |
| Magnesiumstearat | 0,5 mg |
| | 80 mg |

Der Wirkstoff, Maisstärke, Milchzucker und Polyvinylpyrrolidon werden gut gemischt und mit Wasser befeuchtet. Die feuchte Masse drückt man durch ein Sieb mit 1 mm-Maschenweite, trocknet bei ca. 45°C und schlägt das Granulat anschließend durch dasselbe Sieb. Nach dem Zumischen von Magnesiumstearat werden auf einer

Tablettiermaschine gewölbte Dragéekerne mit einem Durchmesser von 6 mm gepreßt. Die so hergestellten Dragéekerne werden auf bekannte Weise mit einer Schicht überzogen, die im wesentlichten aus Zucker und Talkum besteht. Die fertigen Dragées werden mit Wachs poliert.

D)

| Kapseln | pro Kapsel |
|---|---|
| Wirkstoff | 50 mg |
| Maisstärke | 268,5 mg |
| Magnesiumstearat | 1,5 mg |
| | 320 mg |

Substanz und Maisstärke werden gemischt und mit Wasser befeuchtet. Die feuchte Masse wird gesiebt und getrocknet. Das trockene Granulat wird gesiebt und mit Magensiumstearat gemischt. Die Endmischung wird in Hartgelatinekapseln Größe 1 abgefüllt.

E)

| Ampullenlösung | |
|---|---|
| Wirkstoff | 50 mg |
| Natriumchlorid | 50 mg |
| Aqua pro inj. | 5 ml |

Der Wirkstoff wird bei Eigen-pH oder gegebenenfalls bei pH 5,5 bis 6,5 in Wasser gelöst und mit Natriumchlorid als Isotonans versetzt, die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff.

F)

| Suppositorien | |
|---|---|
| Wirkstoff | 50 mg |
| Adeps solidus | 1650 mg |
| | 1700 mg |

Das Hartfett wird geschmolzen. Bei 40°C wird die gemahlene Wirksubstanz homogen dispergiert. Es wird auf 38°C abgekühlt und in schwach vorgekühlte Suppositorienformen ausgegossen.

G)

| orale Suspension | |
|---|---|
| Wirkstoff | 50 mg |

(fortgesetzt)

| orale Suspension | |
|---|---|
| Hydroxyethylcellulose | 50 mg |
| Sorbinsäure | 5 mg |
| Sorbit (70%ig) | 600 mg |
| Glycerin | 200 mg |
| Aroma | 15 mg |
| Wasser ad | 5 ml |

Destilliertes Wasser wird auf 70°C erhitzt. Hierin wird unter Rühren Hydroxyethylcellulose gelöst. Nach Zugabe von Sorbitlösung und Glycerin wird auf Raumtemperatur abgekühlt. Bei Raumtemperatur werden Sorbinsäure, Aroma und Substanz zugegeben. Zur Entlüftung der Suspension wird unter Rühren evakuiert.

**Patentansprüche**

1. Verbindungen erhältlich durch folgende Reaktionsschritte:

a) Umsetzung von 0,1 Mol eines Triazols der allgemeinen Formel (3)

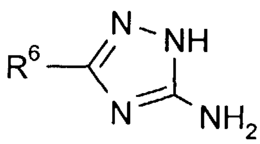

(3)

worin

$R^6$ Wasserstoff, Methyl, Ethyl, n-Propyl, i-Propyl, n-Butyl, tert.-Butyl, Phenyl, Benzyl, Cyclopentyl, 2-Furyl, 3-Pyridylmethyl, Cyclohexylmethyl, 2-Phenylethyl, (4-MeO-Ph)-O-$CH_2$-, (4-MeO-Ph)-$CH_2$-O-$CH_2$-, (4-$PhCH_2O$-Ph)-$CH_2$-, 4-F-Ph-$CH_2$- oder 3,4-F-Ph-$CH_2$- bedeuten können, mit 0,13 Mol Cyanessigsäureethylester in Gegenwart von 0,1 Mol Natriumethylat in absolutem Ethanol unter Rückfluß über einen Zeitraum von 4-6 h zu dem entsprechenden Triazolopyrimidin, welches nach Abkühlen des Reaktionsgemisches sowie anschließendem Versetzen mit Wasser und Ansäuern isoliert wird

b) anschließende Umsetzung von 66mMol des so erhaltenen Triazolopyrimidins bei Raumtemperatur mit 76mMol eines Alkylhalogenids des Typs R'-X,
wobei R' für den Rest $R^4$ steht, wobei
$R^4$ Methyl, Ethyl, n-Propyl, n-Butyl, n-Pentyl oder Benzyl bedeutet, in wassrefreiem Dimethylformamid in Gegenwart von 76mMol Kaliumcarbonat über einen Zeitraum von 1-2 Tagen zu dem entsprechenden alkylierten Triazolopyrimidin, das nach dessen Isolierung gegebenenfalls einer chromatographischen Reinigung unterworfen wird,

c) Umsetzung von 10mMol des aus Schritt b) resultierenden Alkyltriazolopyrimidins mit 20 mMol Isoamylnitrit bei einer Temperatur von <0°C in wasserfreiem Dimethylformamid über einen Zeitraum von 4-24 h zu der entsprechenden Nitrosoverbindung, die isoliert wird,

d) Zugabe von 4.8 mMol der gemäß Schritt c) erhaltenen Nitrosoverbindung zu einer Lösung von 6,8 mMol eines N-Formylamins ($R^2$-CHO) in 6 ml Diglyme zu der bei 0°C 0,66 ml (7,2 mMol) Phosphoroxychlorid gegeben wurden und die vor Zugabe der nach Schritt c) erhaltenen Nitrosoverbindung 30 Minuten im Eisbad gehalten wurde, Rühren der so erhaltenen Mischung für 30 - 60 Min. bei 80°C die anschließend auf Eiswasser

gegeben wird, wonach das feste Produkt abfiltriert, gewaschen und getrocknet, zur Reinigung kristallisiert oder chromatographiert wird,
wobei R2 für einen Rest ausgewählt aus der Gruppe N-Piperidinyl, N-Morpholinyl, N-Piperazinyl, 4-Benzylpiperazinyl und Dimethylamino steht,

e) gegebenfalls Deblockierung von gemäß Schritt d erhältlichen und in der Seitenkette benzylierten Verbindungen mit $R^2$ gleich 4-Benzylpiperazinyl, durch Umsetzung von 0,83 mMol der nach Schritt d erhaltenen Verbindung in 30 ml Eisessig bei 60°C und bei 5 bar Wasserstoffdruck über 6 h in Anwesenheit von 0,1 g 10%igem Pd auf Kohle, Abfiltrieren des Katalysators, Einengen des Rückstands zur Trockne, Verrühren des Rückstands mit $CH_3OH$, Abfiltrieren, Waschen und Trocknen des festen Produkts.

**2.** Verbindung erhältlich gemäß Anspruch 1, in denen $R^6$ Benzyl und $R^4$ n-Propyl bedeutet, ausgewählt aus der Gruppe bestehend aus:

- Verbindung vom Schmp. 298°C, worin $R^2$ N-Morpholinyl bedeutet;
- Verbindung vom Schmp. 266-268°C, worin $R^2$ 4-Benzyl-1-piperazinyl bedeutet;
- Verbindung vom Schmp. 272-274°C, worin $R^2$ Dimethylamino bedeutet;
- Verbindung vom Schmp. 294-297°C, worin $R^2$ N-Piperidinyl bedeutet;
- Verbindung vom Schmp. 260°C (Acetat), worin $R^2$ 1-Piperazinyl bedeutet;

gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

**3.** Verbindungen erhältlich durch folgende Reaktionsschritte:

a) Umsetzung von 0,1 Mol eines Triazols der allgemeinen Formel (3)

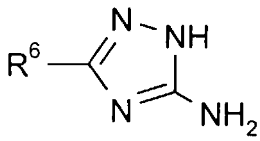

(3)

worin

$R^6$ (4-MeO-Ph)-$CH_2$-O-$CH_2$- bedeuten kann,

mit 0,13 Mol Cyanessigsäureethylester in Gegenwart von 0,1 Mol Natriumethylat in absolutem Ethanol unter Rückfluß über einen Zeitraum von 4-6 h zu dem entsprechenden Triazolopyrimidin, welches nach Abkühlen des Reaktionsgemisches sowie anschließendem Versetzen mit Wasser und Ansäuern isoliert wird

b) anschließende Umsetzung von 66mMol des so erhaltenen Triazolopyrimidins bei Raumtemperatur mit 76mMol eines Alkylhalogenids des Typs R'-X, wobei R' für den Rest $R^4$ steht, wobei $R^4$ Methyl, Ethyl, n-Propyl, n-Butyl, n-Pentyl oder Benzyl bedeutet, in wassrefreiem Dimethylformamid in Gegenwart von 76mMol Kaliumcarbonat über einen Zeitraum von 1-2 Tagen zu dem entsprechenden alkylierten Triazolopyrimidin, das nach dessen Isolierung gegebenenfalls einer chromatographischen Reinigung unterworfen wird,

c) Umsetzung von 10mMol des aus Schritt b) resultierenden Alkyltriazolopyrimidins mit 20 mMol Isoamylnitrit bei einer Temperatur von <0°C in wasserfreiem Dimethylformamid über einen Zeitraum von 4-24 h zu der entsprechenden Nitrosoverbindung, die isoliert wird,

d1) Suspendieren von 12,7 mMol eines gemäß Schrill c) erhaltenen Nitrosoderivats in Wasser, Zusetzen von 4,9 g $Na_2S_2O_4$, Erhitzen der Suspension auf 80°C, Zugabe von 15 ml 50%iger H2SO4 und Kochen der Mischung unter Rückfluß bis zum vollständigen Umsatz, wodurch nach Abkühlen das Produkt entweder dirket als ausfallendes Semisulfat oder, nach Neutralisieren mit 30%-iger Natronlauge, als ausgefallene freie Base isoliert werden kann, oder

d2) Lösen von 11,5 mMol eines gemäß Schritt c) erhaltenen Nitrosoderivats in einer Mischung aus 175 ml 25%-igem wässrigen Ammoniak und 36 ml Ethanol und Umsetzung mit einer Lösung von 6,1 g $Na_2S_2O_4$ in 57 ml Wasser bei Raumtemperatur über einen Zeitraum von 1,5-3 h, Abfiltrieren des resultierenden Diamins,

e) Versetzen von 30 mMol eines gemäß Schritt d1) oder gemäß Schritt d2) erhaltenen, in wasserfreiem Diemthylformamid suspendierten, Diamins mit 45 mMol Dimethylaminopyridin (im Falle von Semisulfaten mit 78 mMol) und Rühren der Mischung über einen Zeitraum von 30 Minuten bei Raumtemperatur, Abkühlen des Reaktionsgemisches auf eine Temperatur von 5-10°C und Zugabe von in 16 ml wasserfreiem Dimethylformamid gelösten 39 mMol eines Carbonsäurechlorids des Typs $R^2$-COCl,
wobei

$R^2$ Methyl, Ethyl, n-Propyl, tert-Butyl, Phenyl, Benzyl, 2-Furyl, 2-Pyridyl, 3-Pyridyl, 4-Pyridyl, 3-Tetrahydrofuranyl, 4-Tetrahydropyranyl, Cyclopentyl, Cyclohexyl, 4-F-Ph-$CH_2$-, Adamantan-1-yl oder Noradamantan-3-yl bedeuten,

sowie 2-stündiges Rühren bei 10°C und anschließendes Rühren über Nacht bei Raumtemperatur, woraus die entsprechenden Amide resultieren, die nach Aufarbeitung und Reinigung durch Kristallisation oder Chromatographie isoliert werden,

f) Cyclisieren von 14,4 mMol der in Schrift e) erhaltenen Carbonsäureamiderivate durch Erhitzen der Amide in einem Lösemittelgemisch aus 75,5 ml Wasser und 37,8 ml Ethanol nach Zugabe von 17,4 ml 50%iger NaOH und 4,82 g $Ca(OH)_2$ über einen Zeitraum von 24 h bei Rückflußtemperatur und Isolieren der entsprechenden Cyclisierungsprodukte nach Abkühlen und Ansäuern des Reaktionsgemisches sowie gegebenenfalls Reinigung durch Umkristallisation oder Chromatographie,

g) Lösen von 0,05 Mol der gemäß Schritt f) erhaltenen Verbindungen in 500 ml abs. Dimethylformamid, Versetzen mit 0,055 Mol Kaliumcarbonat und 0,055 Mol Benzylbromid und Rühren über Nacht bei Raumtemperatur, anschließendes Abdestillieren des Lösungsmittels im Vakuum und Aufnehmen des Rückstands in Methylenchlorid/Wasser, Abtrennen der organischen Phase, Waschen mit Wasser, Trocknen über Magnesiumsulfat Abdestillieren des Lösungsmittels und Kristallisieren des Produkts (mit $R^1$ oder $R^3$ gleich Benzyl) aus Ether,

h) Überführen der gemäß Schrift g) erhaltenen Verbindungen in Verbindungen, in denen $R^6$ -$CH_2$-OH bedeutet in an sich bekannter Art und Weise unter Verwendung von 2,3-Dichlor-5,6-dicyano-benzochinon (DDQ),

i1)Überführen der gemäß Schrift h) erhaltenen Verbindungen in Verbindungen, in denen $R^6$ -$CH_2$-OMe oder -$CH_2$-OMe bedeutet gemäß folgender Vogehensweise: 3 mMol der gemäß Schritt h erhaltenen Verbindungen werden in 30 ml wasserfreiem Dimethylformid gelöst, mit 3,6 mMol Natriumhydrid (60 %ige Suspension in Mineralöl) versetzt, 1 Stunde bei Raumtemperatur gerührt, mit 3,6 mMol Methyl- oder Ethyljodid versetzt und 1-2 Tage bei 25-60° gerührt, zur Vervollständigung der Umsetzung werden je 50 % Natriumhydrid und Alkylhalogenid nachgegeben, zur Aufarbeitung wird die Umsetzung zum Rückstand eingedampft, in Methylenchlorid und Wasser aufgenommen, mit 2 n Salzsäure angesäuert, die Wasserphase noch einmal mit Methylenchlorid extrahiert, die vereinigten organischen Phasen mit Kochsalzlösung gewaschen und über Magnesiumsulfat getrocknet und eingedampft, oder

i2)Überführen der gemäß Schrift h) erhaltenen Verbindungen in Verbindungen, in denen $R^6$ -$CH_2$-$NMe_2$, N-Morpholinomethyl oder Phenyloxymethyl bedeutet gemäß folgender Vogehensweise: Überführen der gemäß Schrift h) erhaltenen Verbindungen in die entsprechenden Halogenide in an sich bekannter Art und Weise unter Verwendung von Halogenierungsreagenzien wie $SOCl_2$, $SOBr_2$ und $POCl_3$ in inerten Lösungsmitteln bei Anwesenheit von Basen (z.B. $NEt_3$), anschließend
Überführen der so erhaltenen Halogenide in an sich bekannter Art und Weise in die Verbindungen in denen $R^6$ -$CH_2$-$NMe_2$, N-Morpholinomethyl oder Phenyloxymethyl bedeutet, oder

i3)Überführen der gemäß Schrift h) erhaltenen Verbindungen in Verbindungen, in denen $R^6$ -CHO bedeutet durch Oxidation in an sich bekannter Art und Weise, oder

i4)Überführen der gemäß Schrift h) erhaltenen Verbindungen in Verbindungen, in denen $R^6$ -COOH bedeutet durch Oxidation in an sich bekannter Art und Weise, oder

i5)Überführen der gemäß Schrift h) erhaltenen Verbindungen in Verbindungen, in denen $R^6$ -CHO bedeutet durch Oxidation in an sich bekannter Art und Weise und anschließende reduktive Aminierung durch Umsetzung mit 3-Aminopyridin in an sich bekannter Art und Weise zu Verbindungen in denen $R^6$ 3-Pyridylaminomethyl bedeutet, oder

i6)Überführen der gemäß Schrift h) erhaltenen Verbindungen in Verbindungen, in denen $R^6$ -COOH bedeutet durch Oxidation in an sich bekannter Art und Weise und anschließende Veresterung zu Verbindungen in denen $R^6$ -COOMe bedeutet nach Standardverfahren,

i7)Überführen der gemäß Schrift h) erhaltenen Verbindungen in Verbindungen, in denen $R^6$ Benzoyloxymethyl oder 3-Pyridylcarbonyloxymethyl bedeutet gemäß nachstehend beschriebener Vorgehensweise: die gemäß Schrift h) erhaltene Hydroxylverbindung wird in Pyridin aufgenommen, unter Eiskühlung mit stöchiometrischen Mengen Säurechlorid versetzt, nach vollständigem Umsatz wird der Ansatz auf Eiswasser gegeben und mit 2 n Salzsäure angesäuert, die wässrige Phase wird mehrfach mit Methylenchlorid extrahiert, die vereinigten organischen Phasen werden mit Kochsalzlösung gewaschen, über Magnesiumsulfat getrocknet und eingedampft, der Rückstand wird an Kieselgel gereinigt und das Eluat aus Ether kristallisiert.

j) Abspaltung der Benzylschutzgruppen aus den Verbindungen erhältlich gemäß einem der Schrille i1) bis i7) gemäß nachstehend beschriebener Vorgehensweise: Lösen der Verbindungen erhältlich gemäß Schrift i1) bis i7) in Methanol oder absolutem Eisessig, Hydrieren unter einer Wasserstoffatmosphäre (1-5 bar) bei 25-80°C mit Hilfe eines Palladiumkatalysators (z.B. Pd/C) über einen Zeitraum von 2,5 - 40 h, Absaugen vom Katalysator, Eindampfen, Reinigen des Rückstands an Kieselgel und Kristallisieren des gereinigten Produkts aus dem Eluat mit Ether.

**4.** Verbindung erhältlich gemäß Anspruch 3, Schrille a) bis h), in der $R^1$ oder $R^3$ Benzyl, $R^2$ Cyclopentyl, $R^4$ n-Propyl und $R^6$ $-CH_2-OH$ bedeutet;

**5.** Verbindung erhältlich gemäß Anspruch 3, Schrille a) bis i1), in der $R^1$ oder $R^3$ Benzyl, $R^2$ Cyclopentyl, $R^4$ Ethyl und $R^6$ $-CH_2-OMe$ oder $-CH_2-OEt$ bedeutet;

**6.** Verbindung erhältlich gemäß Anspruch 3, Schritte a) bis i2), in der $R^1$ oder $R^3$ Benzyl, $R^2$ Cyclopentyl, $R^4$ Ethyl und $R^6$ $-CH_2-NMe_2$, N-Morpholinomethyl oder Phenyloxymethyl bedeutet;

**7.** Verbindung erhältlich gemäß Anspruch 3, Schrille a) bis i3), in der $R^1$ oder $R^3$ Benzyl, $R^2$ Cyclopentyl, $R^4$ Ethyl und $R^6$ -CHO bedeutet;

**8.** Verbindung erhältlich gemäß Anspruch 3, Schrille a) bis i4), in der $R^1$ oder $R^3$ Benzyl, $R^2$ Cyclopentyl, $R^4$ Ethyl und $R^6$ -COOH bedeutet;

**9.** Verbindung erhältlich gemäß Anspruch 3, Schrille a) bis i5), in der $R^1$ oder $R^3$ Benzyl, $R^2$ Cyclopentyl, $R^4$ Ethyl und $R^6$ 3-Pyridylaminomethyl bedeutet

**10.** Verbindung erhältlich gemäß Anspruch 3, Schrille a) bis i6), in der $R^1$ oder $R^3$ Benzyl, $R^2$ Cyclopentyl, $R^4$ Ethyl und $R^6$ -COOMe bedeutet;

**11.** Verbindung erhältlich gemäß Anspruch 3, Schrille a) bis i7), in der $R^1$ oder $R^3$ Benzyl, $R^2$ Cyclopentyl, $R^4$ Ethyl und $R^6$ Benzoyloxymethyl oder 3-Pyridylcarbonyloxymethyl bedeutet;

**12.** Verbindung erhältlich gemäß Anspruch 3, in der $R^2$ Cyclopentyl, $R^4$ Ethyl und $R^6$ Methoxymethyl, Ethoxymethyl, Hydoxymethyl, Benzoyloxymethyl, Dimethylaminomethyl, N-Morpholinylmethyl oder Phenoxymethyl bedeutet;

**13.** Verwendung einer Verbindung gemäß einem der Ansprüche 1 bis 12 zur Herstellung eines Arzneimittels.

**14.** Pharmazeutische Zubereitungen, enthaltend als Wirkstoff eine oder mehrere Verbindungen gemäß einem der Ansprüche 1 bis 12 oder deren physiologisch verträgliche Säureadditionssalze in Kombination mit üblichen Hilfs- und/oder Trägerstoffen.